(19) Europäisches Patentamt
European Patent Office
Office européen des brevets

(11) **EP 4 772 497 A1**

(12) **EUROPEAN PATENT APPLICATION**
published in accordance with Art. 153(4) EPC


(43) Date of publication:
**08.07.2026 Bulletin 2026/28**

(21) Application number: **24873075.6**

(22) Date of filing: **27.09.2024**

(51) International Patent Classification (IPC):
***C07D 211/60*** (2006.01) ***A61K 31/445*** (2006.01)
***A61K 45/06*** (2006.01) ***A61P 25/00*** (2006.01)
***A61P 25/28*** (2006.01) ***A61K 39/00*** (2006.01)

(52) Cooperative Patent Classification (CPC):
**A61K 31/445; A61K 39/00; A61K 45/06; A61P 25/00; A61P 25/28; C07D 211/60**

(86) International application number:
**PCT/KR2024/014808**

(87) International publication number:
**WO 2025/071368 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 KR 20230131187**



(71) Applicant: **BNH Research Co., Ltd.**
**Goyang-si, Gyeonggi-do 10594 (KR)**

(72) Inventors:
- **KIM, Young Hwan**
  **Goyang-si, Gyeonggi-do 10218 (KR)**
- **JEONG, Ji Hyun**
  **Seoul 03974 (KR)**

(74) Representative: **Ullrich & Naumann PartG mbB**
**Schneidmühlstrasse 21**
**69115 Heidelberg (DE)**


(54) **POLYMORPHS OF NITROGEN-CONTAINING HETEROCYCLIC COMPOUNDS, AND USES THEREOF**


(57) The present invention relates to polymorphs of nitrogen-containing heterocyclic compounds. The polymorphs of the present invention effectively improve or reactivate synaptic plasticity without side effects, and inhibit or remove the accumulation of amyloid beta proteins, thereby exhibiting a therapeutic effect on various neurological disorders or neurodegenerative disorders. In addition, the polymorphs of the present invention exhibit excellent physicochemical properties such as stability, solubility, hygroscopicity, and mechanical strength, and exhibits excellent permeability to the blood-brain barrier (BBB).


FIG. 3a

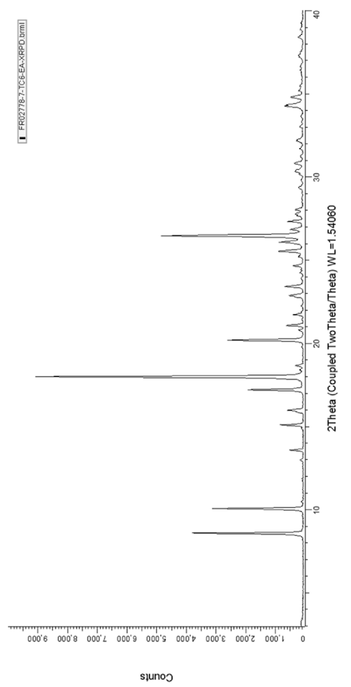


EP 4 772 497 A1

Processed by Luminess, 75001 PARIS (FR)

## Description

### FIELD OF THE INVENTION

The present application claims priority to Korean Patent Application No. 10-2023-0131187 filed on September 27, 2023, the disclosure of which is incorporated herein by reference in its entirety.

The present disclosure relates to polymorphs of nitrogen-containing heterocyclic compounds. Specifically, the present disclosure relates to polymorphs of compounds for the prevention or treatment of a neurological disorder or a neurodegenerative disorder, methods for preparing the same, pharmaceutical compositions, and uses thereof.

### BACKGROUND

#### Synaptic plasticity and critical period

A neural network is a network made of neurons, and a synapse is a node in the neural network through which neurons exchange information with each other. Within the network, synapses are "plastic," because their numbers and strengths change continuously throughout life, as synaptic inputs are added, removed, or refined in response to ongoing neural activities. Synaptic plasticity refers to these neural activity-dependent modifications of the synapses, which is manifested by changes in the strength and/or efficiency of synaptic transmission.

Higher-order functions, such as learning, cognition, synthesis, judgment, art, or creativity, depend on the ability to form neural networks in the cerebral cortex (called cortical neural networks). Synaptic plasticity of the cortical neural network peaks during the early days after birth, which is known as the critical period of synaptic plasticity. After the critical period, the cortical synaptic plasticity decreases gradually with aging, eventually leading to a state in which experiences no longer result in active changes in the cerebral cortex. Interestingly, however, recent studies have demonstrated that under certain conditions, synaptic plasticity could be restored to heightened levels even after the critical period had been closed: In an animal model, peripheral nerve injury reactivated synaptic plasticity in the somatosensory thalamocortical circuit that transmits peripheral sensory signals to the cerebral cortex (Yu et al., 2012, Neuron. 74:731-42; Petrus et al., 2014, Neuron. 81:664-73; Gao et al., 2014, Cell 159.4: 775-788; and Gao et al., 2014, J Neurosci. 34(32): 10770-10779).

Reduced synaptic plasticity is also related to degeneration of the brain function in adults. In particular, the synaptic plasticity of the thalamic cortical circuit is significantly limited with aging. If reopening the critical period is possible, as mentioned above, reactivating or increasing cortical synaptic plasticity via therapeutical means may be useful for treating diseases caused by reduced synaptic plasticity.

#### Amyloid-beta protein

Alzheimer's disease (AD) accounts for the largest proportion of dementia cases and remains a degenerative brain disease for which no effective treatment has yet been established. Alzheimer's disease is characterized by the accumulation of amyloid-beta (Aβ) and tau proteins in the brain, chronic neuroinflammation, and mitochondrial dysfunction. Previous research on Alzheimer's disease has focused on the accumulation of amyloid-beta and tau proteins; however, most attempts to develop new drugs targeting these two causative agents have failed. Aducanumab, an amyloid-beta-targeting drug approved for clinical use in 2021, also demonstrated lower-than-expected efficacy with adverse effects, which led to the withdrawal of its approval in Europe. Thus, there is a need for therapeutic agents targeting the pathological phenotypes of Alzheimer's disease other than the two causative agents.

#### Crystalline forms of compounds

A crystalline form affects the physical properties of a compound to some extent. Due to different crystal lattice structures, pharmacological compounds having many types of crystalline forms not only have distinct appearances (for example, color; forms such as needle-like crystals, crystalline plates, and crystalline particles) but also have different physical properties (for example, melting point, solubility, density, stability, and hygroscopicity), thereby displaying different solubility and absorption profiles in vivo, which may affect the clinical efficacy and stability of the pharmacological compounds.

A certain crystalline form has distinct thermodynamic behaviors compared to an amorphous state or other crystalline forms. To characterize thermal properties for distinguishing a certain crystalline form, an amorphous state, and other crystalline forms, techniques such as melting point apparatus, thermogravimetric analysis (TGA), or differential scanning calorimetry (DSC) may be used. Certain crystalline forms may have unique spectroscopic properties. For example, an X-ray powder diffraction (XRPD) pattern may characterize a specific crystalline form.

[Prior Art Documents]

[Non-Patent Documents]

(Non-Patent Document 1) Yu, Xin, et al. "Thalamocortical inputs show post-critical-period plasticity." Neuron 74.4 (2012): 731-742.
(Non-Patent Document 2) Petrus, Emily, et al. "Crossmodal induction of thalamocortical potentiation leads to enhanced information processing in the auditory cortex." Neuron 81.3 (2014): 664-673.
(Non-Patent Document 3) Gao, Peng, et al. "Deterministic progenitor behavior and unitary production of neurons in the neocortex." Cell 159.4 (2014): 775-788.
(Non-Patent Document 4) Gao, Ming, et al. "Rebound potentiation of inhibition in juvenile visual cortex requires vision-induced BDNF expression." Journal of Neuroscience 34.32 (2014): 10770-10779.

DISCLOSURE OF INVENTION

TECHNICAL PROBLEM

The object of the present disclosure is to solve all of the above-mentioned problems.

An object of the present disclosure is to provide a novel crystalline form of a nitrogen-containing heterocyclic compound for enhancing synaptic plasticity and/or inhibiting or removing amyloid-beta (Aβ) protein accumulation.

Another object of the present disclosure is to provide a pharmaceutical composition comprising, as an active ingredient, the crystalline form of the nitrogen-containing heterocyclic compound for enhancing synaptic plasticity and/or inhibiting or removing amyloid-beta protein accumulation.

Yet another object of the present disclosure is to provide a method for treating or preventing a neurological disorder or a neurodegenerative disorder, comprising administering to a subject the crystalline form of the nitrogen-containing heterocyclic compound for enhancing synaptic plasticity and/or inhibiting or removing amyloid-beta protein accumulation.

Still yet another object of the present disclosure is to provide a pharmaceutical composition, kit, or method for co-administration of the crystalline form of the nitrogen-containing heterocyclic compound for enhancing synaptic plasticity and/or inhibiting or removing amyloid-beta protein accumulation with a second therapeutic agent.

The object of the present disclosure is not limited to the above-mentioned objects. The objects of the present disclosure will become clearer from the following description and will be realized by means and combinations thereof as set forth in the claims.

SOLUTION TO PROBLEM

The present disclosure provides a compound of Formula (1), that is, a new polymorph of (3S,6R)-1-(2-(2-chloro-4-fluorophenyl)acetyl)-6-methylpiperidine-3-carboxylic acid, a pharmaceutical composition thereof, a method for preparing the same, and a use thereof for the prevention and/or treatment of a neurological disorder or a neurodegenerative disorder.

In an aspect of the present disclosure, there is provided a crystalline form of a compound represented by Formula (1).

Formula (1)

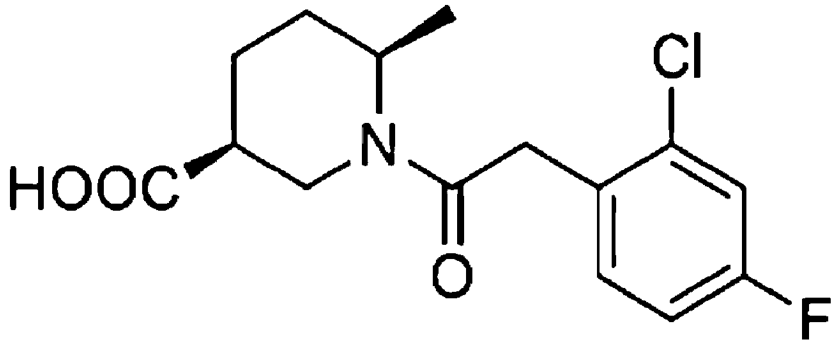

In an aspect of the present disclosure, there is provided crystalline form A of the compound represented by Formula (1), characterized by an X-ray powder diffraction pattern having diffraction peaks at diffraction angles 2θ(°) of 9.9 ±0.10, 14.9±0.10, 18.4±0.10, 24.5±0.10, 24.8±0.10, 25.2±0.10, and 28.7±0.10.

In an embodiment, the X-ray powder diffraction pattern of crystalline form A may further comprise diffraction peaks at diffraction angles 2θ(°) of 5.0±0.10, 13.4±0.10, 19.7±0.10, 19.8±0.10, 20.5±0.10, 22.5±0.10, 24.0±0.10, and 35.0±0.10.

In an embodiment, the X-ray powder diffraction pattern of crystalline form A may further comprise diffraction peaks at diffraction angles 2θ(°) of 12.1±0.10, 16.2±0.10, 20.1±0.10, 21.5±0.10, 27.4±0.10, 29.6±0.10, 30.0±0.10, 31.2±0.10, 31.5±0.10, and 36.8±0.10, in addition to the above-described diffraction peaks.

In another aspect of the present disclosure, there is provided crystalline form B of the compound represented by Formula (1), characterized by an X-ray powder diffraction pattern having diffraction peaks at diffraction angles 2θ(°) of 8.6 ±0.10, 9.1±0.10, 9.2±0.10, 15.8±0.10, 20.0±0.10, 20.3±0.10, 20.7±0.10, 23.9±0.10, and 32.6±0.10.

In an embodiment, the X-ray powder diffraction pattern of the crystalline form B may further comprise diffraction peaks at diffraction angles 2θ(°) of 8.2±0.10, 14.2±0.10, 14.7±0.10, 18.0±0.10, 18.3±0.10, 22.9±0.10, 26.4±0.10, 26.7±0.10, 27.6±0.10, 28.1±0.10, and 29.1±0.10.

In an embodiment, the X-ray powder diffraction pattern of the crystalline form B may further comprise diffraction peaks at diffraction angles 2θ(°) of 11.9±0.10, 15.1±0.10, 16.5±0.10, 19.3±0.10, 22.1±0.10, 22.6±0.10, 24.5±0.10, 24.8±0.10, 25.9±0.10, 27.0±0.10, 27.8±0.10, 28.9±0.10, and 36.5±0.10, in addition to the above-described diffraction peaks.

In yet another aspect of the present disclosure, there is provided crystalline form C of the compound represented by Formula (1), characterized by an X-ray powder diffraction pattern having diffraction peaks at diffraction angles 2θ(°) of 8.6±0.10, 10.0±0.10, 18.0±0.10, and 26.4±0.10.

In an embodiment, the X-ray powder diffraction pattern of the crystalline form C may further comprise diffraction peaks at diffraction angles 2θ(°) of 17.2±0.10, 20.2±0.10, and 25.5±0.10.

In an embodiment, the X-ray powder diffraction pattern of the crystalline form C may further comprise diffraction peaks at diffraction angles 2θ(°) of 15.1±0.10, 16.0±0.10, 21.1±0.10, 23.4±0.10, 26.1±0.10, and 34.2±0.10, in addition to the above-described diffraction peaks.

In still yet another aspect of the present disclosure, there is provided crystalline form D of the compound represented by Formula (1), characterized by an X-ray powder diffraction pattern having diffraction peaks at diffraction angles 2θ(°) of 8.8±0.10, 10.7±0.10, 17.1±0.10, 17.6±0.10, 19.1±0.10, 21.5±0.10, 22.2±0.10, and 23.4±0.10.

In an embodiment, the X-ray powder diffraction pattern of the crystalline form D may further comprise diffraction peaks at diffraction angles 2θ(°) of 12.5±0.10, 15.4±0.10, 16.1±0.10, 16.7±0.10, 18.3±0.10, 22.5±0.10, 22.9±0.10, 27.4±0.10, 27.8±0.10, and 29.8±0.10.

In an embodiment, the X-ray powder diffraction pattern of the crystalline form D may further comprise diffraction peaks at diffraction angles 2θ(°) of 9.5±0.10, 15.7±0.10, 21.0±0.10, 25.1±0.10, 25.3±0.10, 27.0±0.10, 28.6±0.10, 30.9±0.10, 32.1±0.10, 32.6±0.10, and 34.1±0.10, in addition to the above-described diffraction peaks.

In still yet another aspect of the present disclosure, there is provided crystalline form E of the compound represented by Formula (1), characterized by an X-ray powder diffraction pattern having diffraction peaks at diffraction angles 2θ(°) of 10.2±0.10, 11.3±0.10, 18.3±0.10, 20.5±0.10, 22.8±0.10, 23.5±0.10, 25.2±0.10, 26.1±0.10, 28.6 ±0.10, 28.9±0.10, and 31.2±0.10.

In an embodiment, the X-ray powder diffraction pattern of the crystalline form E may further comprise diffraction peaks at diffraction angles 2θ(°) of 9.9±0.10, 13.4±0.10, 17.9±0.10, 18.6±0.10, 19.6±0.10, 20.8±0.10, 21.0±0.10, 24.4±0.10, 24.8±0.10, 29.5±0.10, 29.8±0.10, and 32.5±0.10.

In an embodiment, the X-ray powder diffraction pattern of the crystalline form E may further comprise diffraction peaks at diffraction angles 2θ(°) of 4.9±0.10, 7.2±0.10, 14.8±0.10, 16.1±0.10, 16.4±0.10, 19.8±0.10, 20.0±0.10, 21.5 ±0.10, 22.5±0.10, 24.0±0.10, 25.4±0.10, 27.4±0.10, 32.8±0.10, and 39.5±0.10, in addition to the above-described diffraction peaks.

In an embodiment, the crystalline forms of the present disclosure may have high BBB permeability.

In still yet another aspect of the present disclosure, there is provided a pharmaceutical composition comprising, as an active ingredient, any one of the crystalline forms of the present disclosure.

In an embodiment, the composition may be for the prevention or treatment of a neurological disorder or a neurodegenerative disorder.

In still yet another aspect of the present disclosure, there is a method for preventing or treating a neurological disorder or a neurodegenerative disorder, comprising administering to a subject in need thereof any one of the crystalline forms of the present disclosure.

In an embodiment, the disorder may be Alzheimer's disease.

In an embodiment, the disorder may be post-traumatic stress disorder (PTSD).

In an embodiment, any one of the crystalline forms of the present disclosure may be used in combination with a second therapeutic agent.

In an embodiment, the second therapeutic agent may be a drug for the prevention or treatment of a neurological disorder or a neurodegenerative disorder.

In an embodiment, the second therapeutic agent may be selected from the group consisting of donepezil, rivastigmine, galantamine, memantine, verubecestat, solanezumab, bapineuzumab, aducanumab, lecanemab, tideglusib, epothilone D, and ABBV-8E12.

In an embodiment, the second therapeutic agent may be an antibody that specifically binds to amyloid-beta (for example, aducanumab).

## EFFECTS OF INVENTION

The novel crystalline forms of the present disclosure are capable of effectively enhancing or reactivating synaptic plasticity without adverse effects and inhibiting or removing amyloid-beta protein accumulation, thereby exerting therapeutic effects on various neurological disorders or neurodegenerative disorders. In addition, the crystalline forms of the present disclosure exhibit excellent physicochemical properties such as stability, solubility, hygroscopicity, and mechanical strength. Furthermore, the crystalline forms of the present disclosure exhibit excellent permeability across the blood-brain barrier (BBB). Therefore, the novel crystalline forms of the present disclosure may be developed as excellent therapeutic agents for neurological or neurodegenerative disorders such as Alzheimer's disease or PTSD.

## BRIEF DESCRIPTION OF THE DRAWINGS

Fig. 1a is a graph showing the results of X-ray powder diffraction (XRPD) analysis of crystalline form A of the compound of Formula (1).

Fig. 1b is a graph showing the results of $^1$H nuclear magnetic resonance (NMR) analysis of crystalline form A of the compound of Formula (1).

Fig. 1c is a graph showing the results of differential scanning calorimetry (DSC) analysis of crystalline form A of the compound of Formula (1).

Fig. 1d is a graph showing the results of thermogravimetric analysis (TGA) of crystalline form A of the compound of Formula (1).

Fig. 2a is a graph showing the results of X-ray powder diffraction (XRPD) analysis of crystalline form B of the compound of Formula (1).

Fig. 2b is a graph showing the results of differential scanning calorimetry (DSC) analysis of crystalline form B of the compound of Formula (1).

Fig. 2c is a graph showing the results of thermogravimetric analysis (TGA) of crystalline form B of the compound of Formula (1).

Fig. 3a is a graph showing the results of X-ray powder diffraction (XRPD) analysis of crystalline form C of the compound of Formula (1).

Fig. 3b is a graph showing the results of $^1$H nuclear magnetic resonance (NMR) analysis of crystalline form C of the compound of Formula (1).

Fig. 3c is a graph showing the results of differential scanning calorimetry (DSC) analysis of crystalline form C of the compound of Formula (1).

Fig. 3d is a graph showing the results of thermogravimetric analysis (TGA) of crystalline form C of the compound of Formula (1).

Fig. 4a is a graph showing the results of X-ray powder diffraction (XRPD) analysis of crystalline form D of the compound of Formula (1).

Fig. 4b is a graph showing the results of $^1H$ nuclear magnetic resonance (NMR) analysis of crystalline form D of the compound of Formula (1).

Fig. 4c is a graph showing the results of differential scanning calorimetry (DSC) analysis of crystalline form D of the compound of Formula (1).

Fig. 4d is a graph showing the results of thermogravimetric analysis (TGA) of crystalline form D of the compound of Formula (1).

Fig. 5a is a graph showing the results of X-ray powder diffraction (XRPD) analysis of crystalline form E of the compound of Formula (1).

Fig. 5b is a graph showing the results of $^1H$ nuclear magnetic resonance (NMR) analysis of crystalline form E of the compound of Formula (1).

Fig. 5c is a graph showing the results of differential scanning calorimetry (DSC) analysis of crystalline form E of the compound of Formula (1).

Fig. 5d is a graph showing the results of thermogravimetric analysis (TGA) of crystalline form E of the compound of Formula (1).

Fig. 6 is a diagram showing the interconversion relationships among crystalline forms A, B, C, D, and E of the compound of Formula (1).

Fig. 7a is a graph showing the primary X-ray powder diffraction (XRPD) overlay of samples obtained from a competitive equilibrium experiment at 5°C.

Fig. 7b is a graph showing the secondary X-ray powder diffraction (XRPD) overlay of samples obtained from a competitive equilibrium experiment at 5°C.

Fig. 7c is a graph showing the primary X-ray powder diffraction (XRPD) overlay of samples obtained from a competitive equilibrium experiment at 25°C.

Fig. 7d is a graph showing the secondary X-ray powder diffraction (XRPD) overlay of samples obtained from the competitive equilibrium experiment at 25°C.

Fig. 7e is a graph showing the primary X-ray powder diffraction (XRPD) overlay of samples obtained from a competitive equilibrium experiment at 50°C.

Fig. 7f is a graph showing the secondary X-ray powder diffraction (XRPD) overlay of samples obtained from a competitive equilibrium experiment at 50°C.

Fig. 8 is a graph showing the X-ray powder diffraction (XRPD) overlay of samples obtained by applying stress to crystalline form C of the compound of Formula (1).

Figs. 9a and 9b are diagrams showing the results of dynamic vapor sorption (DVS) analysis of crystalline form C of the compound of Formula (1).

Fig. 9c is a graph showing the X-ray powder diffraction (XRPD) overlay of crystalline form C of the compound of Formula (1) before and after the DVS test.

Fig. 10 is a graph showing the X-ray powder diffraction (XRPD) overlay of samples obtained by compressing crystalline form C of the compound of Formula (1).

Fig. 11 is a graph showing the X-ray powder diffraction (XRPD) overlay of samples obtained by grinding crystalline form C of the compound of Formula (1).

Fig. 12 is a graph showing the X-ray powder diffraction (XRPD) overlay of samples obtained by granulating crystalline form C of the compound of Formula (1).

Fig. 13a illustrates a dosing schedule for analyzing the efficacy of single administration of crystalline form C of the compound of Formula (1) (designated as BnH-015B) or aducanumab, and co-administration thereof, in enhancing ex vivo synaptic plasticity in an animal model of Alzheimer's disease.

Fig. 13b illustrates the results of LTP measurement of field excitatory postsynaptic potentials in the hippocampus of a mouse model of Alzheimer's disease following single administration of crystalline form C of the compound of Formula (1) (designated as BnH-015B) or aducanumab, and co-administration thereof.

Fig. 13c illustrates the results obtained by measuring the balance of excitation/inhibition (right) and the changes in TC EPSC potency (left), in the sensory cerebral cortex of a mouse model of Alzheimer's disease, following single administration of crystalline form C of the compound of Formula (1) (designated as BnH-015B) or aducanumab, and co-administration thereof.

Fig. 14a illustrates the changes in TC EPSC potency in the mouse barrel cortex (layer 4) following oral administration (1 mg/kg) of crystalline form C of the compound of Formula (1) (designated as BnH-015B enantiomer (3S, 6R, 98%<)).

Fig. 14b illustrates the results obtained by measuring the balance of excitation/inhibition in the mouse barrel cortex (layer 4) following oral administration (1 mg/kg) of crystalline form C of the compound of Formula (1) (designated as

BnH-015B enantiomer (3S, 6R, 98%<)).

Fig. 15 illustrates the results of a one-tailed unpaired t-test on the expression level data of IL-33 and OPN following administration of crystalline form C (designated as 015B) in mouse proteome profiling array analysis.

Fig. 16a illustrates the results of Bielschowsky silver staining in the cerebral cortex of APP/PS1 mice following administration of a vehicle control and crystalline form C (designated as 015B).

Fig. 16b illustrates the results of a one-tailed unpaired t-test on the percent area of Aβ plaques following administration of a vehicle control and crystalline form C (designated BnH-015B).

## MODES FOR CARRYING OUT INVENTION

The detailed description of the present disclosure described below will be described with reference to specific drawings (if any) regarding specific embodiments in which the present disclosure may be practiced, but the present disclosure is not limited thereto, but is limited only by the appended claims with respect to any scope identical to or equivalent to that described in the claims. It should be understood that the various embodiments/examples of the present disclosure are different from each other but do not need to be mutually exclusive. For example, certain shapes, structures, and features described herein may be altered from one embodiment/example to another, or may be realized in a combination of a plurality of embodiments/examples, without departing from the technical ideas and scope of the present disclosure. Technical and academic terms used in this specification, unless otherwise defined, have the same meanings as those commonly used in the field to which the present disclosure belongs. The definitions of terms set forth in this specification shall apply for the purpose of interpreting this specification, and terms expressed in the singular form shall be construed to refer also to the plural form (i.e., at least one) unless the context makes it inappropriate, and vice versa.

### Definitions

The term "and/or" is used herein to mean either "and" or "or" unless otherwise indicated.

The term "at least one" includes individually each of the recited objects after the expression and the various combinations of two or more of the recited objects unless otherwise understood from the context and usage.

The terms "comprise," "comprises," "comprising," "have," "has," "having," "contain," "contains," or "containing" (including grammatical equivalents thereof) should generally be understood as open-ended and non-limiting, for example, not excluding additional non-recited elements or steps, unless otherwise specifically stated or understood from the context.

The term "about" means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

The terms "individual," "patient," and "subject" are used interchangeably and may include mammals, for example, primates (such as humans), companion animals (such as dogs and cats), livestock animals (such as cattle, pigs, horses, sheep, and goats), and laboratory animals (such as rats, mice, and guinea pigs). In an embodiment, the subject is a human.

The term "treatment" generally means obtaining a desired pharmacological effect and/or physiological effect. Such effects are therapeutic in that they partially or completely cure a disease and/or adverse effects caused thereby. Desirable therapeutic effects include, but are not limited to, preventing the onset or recurrence of a disease, improving symptoms, reducing any direct or indirect pathological consequences of a disease, preventing metastasis, slowing progression of a disease, ameliorating or alleviating a disease state, and achieving remission or improved prognosis. Preferably, "treatment" may refer to medical intervention for a disease or disorder that has already manifested.

The term "pharmaceutically acceptable" includes molecular entities and formulations that do not produce an adverse, allergic or other untoward reaction when administered to an animal or a human, as appropriate.

The term "pharmaceutically acceptable salt" refers to a salt which retains the biological effects and properties of the corresponding free base or free acid. For example, a pharmaceutically acceptable salt may be prepared by adding an inorganic base or an organic base to a free acid. Salts derived from inorganic bases include, but are not limited to, sodium, potassium, lithium, ammonium, calcium, magnesium salts, and the like. Salts derived from organic bases include, but are not limited to, primary, secondary, and tertiary amines, substituted amines, including naturally occurring substituted amines, cyclic amines and basic ion exchange resins, such as isopropyl amine, trimethylamine, diethyl amine, triethylamine, tripropylamine, ethanolamine, diethyl amine, lysine, arginine, N-ethyl piperidine, piperidine, piperazine, and the like.

All solvents used herein are commercially available. The present disclosure uses the following abbreviations: EtOH means ethanol; MeOH means methanol; MEK means methyl ethyl ketone; ACN means acetonitrile; EA means ethyl acetate; IPAc means isopropyl acetate; MTBE means methyl tert-butyl ether; THF means tetrahydrofuran; 2-MeTHF

means 2-methyltetrahydrofuran; DCM means dichloromethane; DMSO means dimethyl sulfoxide; DMF means N,N-dimethylformamide; FeSSIF-v1 means fed state (a period during which nutrients are digested and absorbed) simulated intestinal fluid version 1; FaSSIF-v1 means fasted state simulated intestinal fluid version 1; and PBS means phosphate buffered saline.

**New polymorphs of nitrogen-containing heterocyclic compounds**

The present disclosure provides a new polymorph of a compound represented by Formula (1), that is, (3S,6R)-1-(2-(2-chloro-4-fluorophenyl)acetyl)-6-methylpiperidine-3-carboxylic acid, a pharmaceutical composition thereof, a method for preparing the same, and a use thereof for the prevention or treatment of a neurological disorder or a neurodegenerative disorder, and a method for preventing or treating a neurological disorder or a neurodegenerative disorder:

Formula (1)

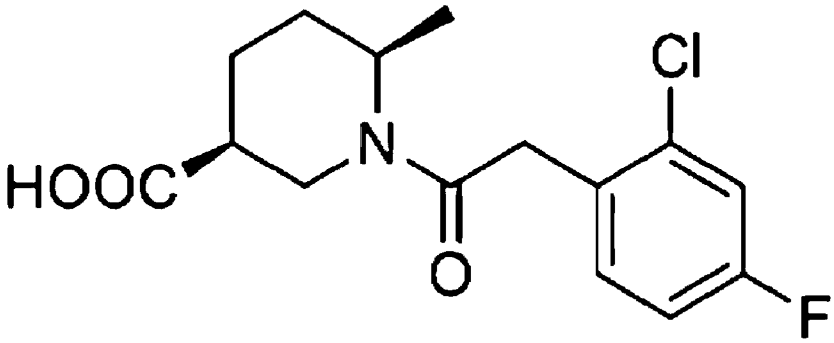

The compound represented by Formula (1) is described in detail in Korean Patent Application No. 10-2023-0040835 and International Patent Application No. PCT/KR2023/004138, the entire contents of which are incorporated herein by reference.

The term "compound" refers to a chemical having a structure represented by Formula (1) described herein, a stereoisomer thereof (including mixtures of stereoisomers), a polymorph thereof, or a pharmaceutically acceptable salt, hydrate, solvate or prodrug thereof.

The term "active agent" is used to indicate a substance, a stereoisomer thereof (including mixtures of stereoisomers), a polymorph thereof, or a pharmaceutically acceptable salt, hydrate, solvate or prodrug thereof, which has biological activity. In an embodiment, the "active agent" is a compound of the present disclosure, in particular, a polymorph of the present disclosure, which has pharmaceutical utility. For example, the active agent may be a therapeutic agent for a neurological disorder or a neurodegenerative disorder.

A novel crystalline form of the compound represented by Formula (1) ((3S,6R)-1-(2-(2-chloro-4-fluorophenyl)acetyl)-6-methylpiperidine-3-carboxylic acid) has been found to have excellent chemical and physical properties that facilitate the manufacture and formulation of the compound, while also exhibiting excellent pharmacological effects, such as enhancement of synaptic plasticity and inhibition or removal of amyloid-beta (A$\beta$) protein accumulation, as well as excellent BBB permeability.

In an aspect of the present disclosure, there is provided a crystalline form of the compound of Formula (1), and there are also provided five polymorphs of the compound of Formula (1), that is, polymorphs designated as crystalline form A, crystalline form B, crystalline form C, crystalline form D, and crystalline form E.

In an aspect of the present disclosure, there is provided a crystalline form with an X-ray powder diffraction pattern having diffraction peaks at diffraction angles 2$\theta$(°) of 9.9$\pm$0.10, 14.9$\pm$0.10, 18.4$\pm$0.10, 24.5$\pm$0.10, 24.8$\pm$0.10, 25.2 $\pm$0.10, and 28.7$\pm$0.10, which is defined herein as crystalline form A.

Preferably, the X-ray powder diffraction pattern of crystalline form A further comprises diffraction peaks at diffraction angles 2$\theta$(°) of 5.0$\pm$0.10, 13.4$\pm$0.10, 19.7$\pm$0.10, 19.8$\pm$0.10, 20.5$\pm$0.10, 22.5$\pm$0.10, 24.0$\pm$0.10, and 35.0 $\pm$0.10.

More preferably, the X-ray powder diffraction pattern of crystalline form A further comprises diffraction peaks at diffraction angles 2$\theta$(°) of 12.1$\pm$0.10, 16.2$\pm$0.10, 20.1$\pm$0.10, 21.5$\pm$0.10, 27.4$\pm$0.10, 29.6$\pm$0.10, 30.0$\pm$0.10, 31.2$\pm$0.10, 31.5$\pm$0.10, and 36.8$\pm$0.10.

The XRPD pattern of crystalline form A is shown in Fig. 1a, and analytical data for the XRPD pattern of crystalline form A are set forth in Table 1.

[Table 1]

| Analytical data for XRPD pattern of crystalline form A | | | | | |
|---|---|---|---|---|---|
| **No.** | **Diffraction angle 2θ(°)** | **Relative intensity (%)** | **No.** | **Diffraction angle 2θ(°)** | **Relative intensity (%)** |
| 1 | 4.96 | 17.8 | 14 | 22.51 | 19.27 |
| 2 | 9.88 | 55.43 | 15 | 24.04 | 20.53 |
| 3 | 12.09 | 7.32 | 16 | 24.45 | 41.93 |
| 4 | 13.4 | 35.68 | 17 | 24.84 | 48.57 |
| 5 | 14.85 | 41.01 | 18 | 25.23 | 83.57 |
| 6 | 16.2 | 7.87 | 19 | 27.39 | 12.63 |
| 7 | 18.4 | 100 | 20 | 28.68 | 46.64 |
| 8 | 19.65 | 18.17 | 21 | 29.64 | 8.66 |
| 9 | 19.83 | 18.88 | 22 | 30.01 | 8.75 |
| 10 | 20.06 | 7.76 | 23 | 31.15 | 10.45 |
| 11 | 20.49 | 35.84 | 24 | 31.5 | 15.38 |
| 12 | 21.47 | 11.49 | 25 | 35.03 | 24.84 |
| 13 | 22.49 | 20.18 | 26 | 36.77 | 10.7 |

Crystalline form A exhibits an $^{1}$H-NMR pattern substantially as shown in Fig. 1b. The $^{1}$H-NMR pattern of crystalline form A indicates that no detectable residual solvent is present. Crystalline form A exhibits a DSC pattern substantially as shown in Fig. 1c. The DSC pattern of crystalline form A shows a single melting peak at 128.3°C with an enthalpy of about 61 J/g. Crystalline form A exhibits a TGA pattern substantially as shown in Fig. 1d. The TGA pattern of crystalline form A shows a weight loss of about 0.7% upon heating from ambient temperature to 120°C.

In another aspect of the present disclosure, there is provided a crystalline form with an X-ray powder diffraction pattern having diffraction peaks at diffraction angles 2θ(°) of 8.6±0.10, 9.1±0.10 and/or 9.2±0.10, 15.8±0.10, 20.0 ±0.10, 20.3±0.10, 20.7±0.10, 23.9±0.10, and 32.6±0.10, which is defined herein as crystalline form B.

Preferably, the X-ray powder diffraction pattern of crystalline form B further comprises diffraction peaks at diffraction angles 2θ(°) of 8.2±0.10, 14.2±0.10, 14.7±0.10, 18.0±0.10, 18.3±0.10, 22.9±0.10, 26.4±0.10, 26.7±0.10, 27.6±0.10, 28.1±0.10, and 29.1±0.10.

More preferably, the X-ray powder diffraction pattern of crystalline form B further comprises diffraction peaks at diffraction angles 2θ(°) of 11.9±0.10, 15.1±0.10, 16.5±0.10, 19.3±0.10, 22.1±0.10, 22.6±0.10, 24.5±0.10, 24.8±0.10, 25.9±0.10, 27.0±0.10, 27.8±0.10, 28.9±0.10, and 36.5±0.10.

The XRPD pattern of crystalline form B is shown in Fig. 2a, and analytical data for the XRPD pattern of crystalline form B are set forth in Table 2.

[Table 2]

| Analytical data for XRPD pattern of crystalline form B | | | | | |
|---|---|---|---|---|---|
| No. | Diffraction angle 2θ(°) | Relative intensity (%) | No. | Diffraction angle 2θ(°) | Relative intensity (%) |
| 1 | 8.22 | 12 | 18 | 22.14 | 8.87 |
| 2 | 8.62 | 84.28 | 19 | 22.61 | 9.39 |
| 3 | 8.64 | 29.84 | 20 | 22.9 | 16.31 |
| 4 | 9.09 | 100 | 21 | 23.88 | 23.87 |
| 5 | 9.12 | 35.74 | 22 | 24.49 | 7.09 |
| 6 | 11.88 | 5.74 | 23 | 24.78 | 6.14 |
| 7 | 14.21 | 13.42 | 24 | 25.86 | 7.18 |
| 8 | 14.7 | 14.47 | 25 | 26.4 | 13.05 |
| 9 | 15.09 | 5.51 | 26 | 26.7 | 10 |

(continued)

| Analytical data for XRPD pattern of crystalline form B | | | | | |
|---|---|---|---|---|---|
| No. | Diffraction angle 2θ(°) | Relative intensity (%) | No. | Diffraction angle 2θ(°) | Relative intensity (%) |
| 10 | 15.78 | 56.21 | 27 | 26.95 | 5.46 |
| 11 | 16.48 | 9.38 | 28 | 27.58 | 10.93 |
| 12 | 18.01 | 13.98 | 29 | 27.77 | 7.57 |
| 13 | 18.28 | 12.66 | 30 | 28.11 | 11 |
| 14 | 19.26 | 8.69 | 31 | 28.89 | 6.01 |
| 15 | 19.99 | 50.61 | 32 | 29.05 | 18.63 |
| 16 | 20.34 | 59.19 | 33 | 32.59 | 20.21 |
| 17 | 20.67 | 23.94 | 34 | 36.46 | 5.12 |

Crystalline form B exhibits a DSC pattern substantially as shown in Fig. 2b. The DSC pattern of crystalline form B shows a single melting peak at 111.4°C with an enthalpy of about 64 J/g and a single endothermic peak at 126.8°C with an enthalpy of about 2 J/g. Crystalline form B exhibits a TGA pattern substantially as shown in Fig. 2c. The TGA pattern of crystalline form B shows a weight loss of about 0.4% upon heating from ambient temperature to 110°C.

In yet another aspect of the present disclosure, there is provided a crystalline form with an X-ray powder diffraction pattern having diffraction peaks at diffraction angles 2θ(°) of 8.6±0.10, 10.0±0.10, 18.0±0.10, and 26.4±0.10, which is defined herein as crystalline form C.

Preferably, the X-ray powder diffraction pattern of crystalline form C further comprises diffraction peaks at diffraction angles 2θ(°) of 17.2±0.10, 20.2±0.10, and 25.5±0.10.

More preferably, the X-ray powder diffraction pattern of crystalline form C further comprises diffraction peaks at diffraction angles 2θ(°) of 15.1±0.10, 16.0±0.10, 21.1±0.10, 23.4±0.10, 26.1±0.10, and 34.2±0.10.

The XRPD pattern of crystalline form C is shown in Fig. 3a, and analytical data for the XRPD pattern of crystalline form C are set forth in Table 3.

[Table 3]

| Analytical data for XRPD pattern of crystalline form C | | | | | |
|---|---|---|---|---|---|
| No. | Diffraction angle 2θ(°) | Relative intensity (%) | No. | Diffraction angle 2θ(°) | Relative intensity (%) |
| 1 | 8.55 | 36.71 | 9 | 20.17 | 25.69 |
| 2 | 8.57 | 8.2 | 10 | 21.05 | 5.31 |
| 3 | 10.03 | 27.84 | 11 | 23.38 | 5.68 |
| 4 | 15.06 | 6.99 | 12 | 25.51 | 8.15 |
| 5 | 15.96 | 5.4 | 13 | 26.06 | 7.81 |
| 6 | 17.18 | 19.3 | 14 | 26.43 | 50.02 |
| 7 | 17.96 | 100 | 15 | 34.24 | 6.17 |
| 8 | 17.98 | 15.37 | | | |

Crystalline form C exhibits an $^1$H-NMR pattern substantially as shown in Fig. 3b. Crystalline form C exhibits a DSC pattern substantially as shown in Fig. 3c. The DSC pattern of crystalline form C shows a single melting peak at 116.1°C with an enthalpy of about 76 J/g. Crystalline form C exhibits a TGA pattern substantially as shown in Fig. 3d. The TGA pattern of crystalline form C shows a weight loss of about 0.3% upon heating from ambient temperature to about 110°C.

In still yet another aspect of the present disclosure, there is provided a crystalline form with an X-ray powder diffraction pattern having diffraction peaks at diffraction angles 28(°) of 8.8±0.10, 10.7±0.10, 17.1±0.10, 17.6±0.10, 19.1 ±0.10, 21.5±0.10, 22.2±0.10, and 23.4±0.10, which is defined herein as crystalline form D.

Preferably, the X-ray powder diffraction pattern of crystalline form D further comprises diffraction peaks at diffraction angles 2θ(◦) of 12.5±0.10, 15.4±0.10, 16.1±0.10, 16.7±0.10, 18.3±0.10, 22.5±0.10, 22.9±0.10, 27.4±0.10, 27.8±0.10, and 29.8±0.10.

More preferably, the X-ray powder diffraction pattern of crystalline form D further comprises diffraction peaks at diffraction angles 2θ(°) of 9.5±0.10, 15.7±0.10, 21.0±0.10, 25.1±0.10, 25.3±0.10, 27.0±0.10, 28.6±0.10, 30.9±0.10, 32.1±0.10, 32.6±0.10, and 34.1±0.10.

The XRPD pattern of crystalline form D is shown in Fig. 4a, and analytical data for the XRPD pattern of crystalline form D are set forth in Table 4.

[Table 4]

| **Analytical data for XRPD pattern of crystalline form D** | | | | | |
|---|---|---|---|---|---|
| No. | Diffraction angle 2θ(°) | Relative intensity (%) | No. | Diffraction angle 2θ(°) | Relative intensity (%) |
| 1 | 8.76 | 37.22 | 16 | 22.51 | 11.21 |
| 2 | 9.52 | 8 | 17 | 22.88 | 15.4 |
| 3 | 10.72 | 47.93 | 18 | 23.41 | 100 |
| 4 | 12.5 | 22.45 | 19 | 25.07 | 5.1 |
| 5 | 15.36 | 17.34 | 20 | 25.25 | 8.65 |
| 6 | 15.65 | 9.23 | 21 | 26.99 | 7.88 |
| 7 | 16.1 | 33.26 | 22 | 27.37 | 26.83 |
| 8 | 16.69 | 10.86 | 23 | 27.78 | 31.17 |
| 9 | 17.14 | 52.92 | 24 | 28.64 | 8.57 |
| 10 | 17.57 | 52.43 | 25 | 29.81 | 21.7 |
| 11 | 18.32 | 11.28 | 26 | 30.87 | 7.39 |
| 12 | 19.1 | 38.15 | 27 | 32.09 | 6.66 |
| 13 | 21 | 6 | 28 | 32.56 | 10.43 |
| 14 | 21.53 | 63.08 | 29 | 34.07 | 6.37 |
| 15 | 22.16 | 95.56 | | | |

Crystalline form D exhibits an $^1$H-NMR pattern substantially as shown in Fig. 4b. The $^1$H-NMR pattern of crystalline form D shows a toluene residue of 5.5% by weight (0.2 equivalents of toluene on a molar basis). Crystalline form D exhibits a DSC pattern substantially as shown in Fig. 4c. The DSC pattern of crystalline form D shows a desolvation peak at 62°C, a single endothermic peak at 114.4°C with an enthalpy of about 39 J/g, and a single endothermic peak at 128.1°C with an enthalpy of about 22 J/g. Crystalline form D exhibits a TGA pattern substantially as shown in Fig. 4d. The TGA pattern of crystalline form D shows a weight loss of about 4.8% upon heating from ambient temperature to about 80°C and a weight loss of about 2.5% from about 80°C to 170°C.

In still yet another aspect of the present disclosure, there is provided a crystalline form with an X-ray powder diffraction pattern having diffraction peaks at diffraction angles 2θ(°) of 10.2±0.10, 11.3±0.10, 18.3±0.10, 20.5±0.10, 22.8±0.10, 23.5±0.10, 25.2±0.10, 26.1±0.10, 28.6±0.10, 28.9±0.10, and 31.2±0.10, which is defined herein as crystalline form E.

Preferably, the X-ray powder diffraction pattern of crystalline form E further comprises diffraction peaks at diffraction angles 2θ(°) of 9.9±0.10, 13.4±0.10, 17.9±0.10, 18.6±0.10, 19.6±0.10, 20.8±0.10, 21.0±0.10, 24.4±0.10, 24.8±0.10, 29.5±0.10, 29.8±0.10, and 32.5±0.10.

More preferably, the X-ray powder diffraction pattern of crystalline form E further comprises diffraction peaks at diffraction angles 2θ(°) of 4.9±0.10, 7.2±0.10, 14.8±0.10, 16.1±0.10, 16.4±0.10, 19.8±0. 10, 20.0±0.10, 21.5±0.10, 22.5±0.10, 24.0±0.10, 25.4±0.10, 27.4±0.10, 32.8±0.10, and 39.5±0.10.

The XRPD pattern of crystalline form E is shown in Fig. 5a, and analytical data for the XRPD pattern of crystalline form E are set forth in Table 5.

[Table 5]

| **Analytical data for XRPD pattern of crystalline form E** | | | | | |
|---|---|---|---|---|---|
| No. | Diffraction angle 2θ(°) | Relative intensity (%) | No. | Diffraction angle 2θ(°) | Relative intensity (%) |
| 1 | 4.93 | 7.39 | 20 | 22.46 | 6.11 |

(continued)

| Analytical data for XRPD pattern of crystalline form E | | | | | |
|---|---|---|---|---|---|
| No. | Diffraction angle 2θ(°) | Relative intensity (%) | No. | Diffraction angle 2θ(°) | Relative intensity (%) |
| 2 | 7.16 | 6.68 | 21 | 22.81 | 100 |
| 3 | 9.86 | 11.06 | 22 | 23.54 | 21.98 |
| 4 | 10.17 | 21.53 | 23 | 24.01 | 7.6 |
| 5 | 11.29 | 31.33 | 24 | 24.42 | 10.83 |
| 6 | 13.38 | 10 | 25 | 24.81 | 10.79 |
| 7 | 14.81 | 8.51 | 26 | 25.2 | 37.58 |
| 8 | 16.1 | 7.68 | 27 | 25.42 | 8.12 |
| 9 | 16.36 | 6.22 | 28 | 26.1 | 35.97 |
| 10 | 17.88 | 9.53 | 29 | 27.35 | 5.32 |
| 11 | 18.33 | 32.49 | 30 | 28.63 | 18.02 |
| 12 | 18.59 | 13.28 | 31 | 28.86 | 16.22 |
| 13 | 19.62 | 10.58 | 32 | 29.53 | 12.14 |
| 14 | 19.82 | 6.81 | 33 | 29.84 | 9.52 |
| 15 | 20.03 | 5.41 | 34 | 31.23 | 14.67 |
| 16 | 20.45 | 24.17 | 35 | 32.52 | 10.41 |
| 17 | 20.82 | 12.11 | 36 | 32.81 | 5.88 |
| 18 | 21.03 | 11.77 | 37 | 39.47 | 5.28 |
| 19 | 21.46 | 8.46 | | | |

Crystalline form E exhibits an $^1$H-NMR pattern substantially as shown in Fig. 5b. The $^1$H-NMR pattern of crystalline form E shows an ethanol residue of 9.3% by weight (0.7 equivalents of ethanol on a molar basis). Crystalline form E exhibits a DSC pattern substantially as shown in Fig. 5c. The DSC pattern of crystalline form E shows a desolvation peak at about 40°C. Crystalline form E exhibits a TGA pattern substantially as shown in Fig. 5d. The TGA pattern of crystalline form E shows a weight loss of about 10.2% upon heating from ambient temperature to about 130°C.

The crystalline forms of Formula (1) disclosed herein, in particular, crystalline form C, exhibit excellent physicochemical properties such as stability, solubility, hygroscopicity, and mechanical strength, and exhibit excellent permeability across the blood-brain barrier (BBB) (see Examples 7 to 12).

Furthermore, the crystalline forms of Formula (1) disclosed herein, in particular, crystalline form C, are capable of effectively enhancing or reactivating synaptic plasticity without adverse effects and effectively inhibiting or removing amyloid-beta protein accumulation, thereby exerting excellent prophylactic or therapeutic effects on various neurological disorders or neurodegenerative disorders (see Examples 13 to 15).

XRPD patterns, $^1$H-NMR patterns, DSC patterns, and TGA patterns may be measured by methods known in the art, including the methods described in the Examples described below. Due to instrument errors or differences among operators, those skilled in the art will understand that there may be some differences in the parameters that determine the physical properties of the crystalline forms. Therefore, while the aforementioned parameters are helpful in characterizing the polymorphs provided by the present disclosure, they should not be understood as limiting the polymorphs disclosed herein.

## Preparation method

The compound of Formula (1) of the present disclosure may be prepared by methods known in the art. In addition, the compound of the present disclosure may be prepared, for example, by the method presented in Example 1 and Scheme 1.

Crystalline form A of the present disclosure may be prepared from the compound of Formula (1), for example, according to the method of Example 2. Crystalline forms B, C, D, and E of the compound of Formula (1) may be prepared from crystalline form A. A schematic diagram of the interconversion relationships among crystalline forms A, B, C, D, and E

is shown in Fig. 6. For representative preparation examples of the crystalline forms of the compound of Formula (1), see the Examples.

**Pharmaceutical composition**

According to still yet another aspect of the present disclosure, there is provided a pharmaceutical composition comprising, as an active ingredient, a compound represented by Formula (1) or a novel crystalline form thereof. The novel polymorphs of the present disclosure may include crystalline forms A to E characterized by comprising the analytical data of the XRPD patterns in Tables 1 to 5.

In an embodiment, the composition comprises one or more selected from crystalline forms A, B, C, D, and E of the present disclosure.

In an embodiment, the composition comprises crystalline form A of the present disclosure in an effective amount.

In an embodiment, the composition comprises crystalline form B of the present disclosure in an effective amount.

In an embodiment, the composition comprises crystalline form C of the present disclosure in an effective amount.

In an embodiment, the composition comprises crystalline form D of the present disclosure in an effective amount.

In an embodiment, the composition comprises crystalline form E of the present disclosure in an effective amount.

In an embodiment, the composition may be for the prevention or treatment of a neurological disorder or a neurodegenerative disorder.

In an embodiment, the disorder may be Alzheimer’s disease or PTSD.

In an embodiment, the pharmaceutical composition of the present disclosure may further comprise one or more pharmaceutically acceptable excipients and/or carriers. Specifically, the one or more pharmaceutically acceptable excipients are selected from the group consisting of diluents, buffers, preservatives, stabilizers, solubilizers, or any combination thereof.

The terms "pharmaceutically acceptable excipient" and "pharmaceutically acceptable carrier" refer to a substance that aids the administration of an active agent to and absorption by a subject and can be included in the composition of the present disclosure without causing a significant adverse toxicological effect on a patient.

Non-limiting examples of pharmaceutically acceptable excipients or carriers include, but are not limited to, buffers (for example, phosphate, citrate, or other organic acids); antioxidants (for example, ascorbic acid or methionine); preservatives (for example, octadecyl dimethyl benzyl ammonium chloride; hexamethonium chloride; benzalkonium chloride, benzethonium chloride; phenol, butyl or benzyl alcohol; alkyl parabens, such as methyl or propyl paraben; catechol; resorcinol; cyclohexanol; 3-pentanol; and m-cresol); low molecular weight (about 10 or fewer residues) polypeptides; proteins (for example, serum albumin, gelatin, or immunoglobulins); hydrophilic polymers (for example, polyvinylpyrrolidone); amino acids (for example, glycine, glutamine, asparagine, histidine, arginine, or lysine); monosaccharides, disaccharides, and other carbohydrates, such as glucose, mannose, or dextrin; chelating agents (for example, EDTA); sugars (for example, sucrose, mannitol, trehalose, or sorbitol); salt-forming counter-ions (for example, sodium); metal complexes (for example, Zn-protein complexes); and/or nonionic surfactants (for example, TWEEN®, PLURONICS®, or polyethylene glycol (PEG)).

The pharmaceutically acceptable excipients or carriers may be sterilized and, if desired, may be mixed with auxiliary agents that do not deleteriously react with the compounds of the present disclosure, such as lubricants, preservatives, stabilizers, wetting agents, emulsifiers, salts that affect osmotic pressure, buffers, colorants, and/or aromatic substances.

In an embodiment, the pharmaceutical composition may be formulated for administration as a liquid dosage form suitable for oral, intracavitary, intradermal, intramuscular, intrathecal, intravenous, subcutaneous, or intraventricular administration. For example, see Remington’s Pharmaceutical Sciences and U.S. Pharmacopeia: National Formulary, Mack Publishing Company, Easton, PA (1984).

In an embodiment, the liquid dosage form of the pharmaceutical composition disclosed herein may further comprise a diluent. In some embodiments, the inert diluent is saline.

In an embodiment, the liquid dosage form of the pharmaceutical composition as described herein may further comprise a buffer. For example, buffers suitable for use in the present disclosure include, but are limited to, both organic acids and inorganic acids and salts thereof, such as citrate buffers (for example, monosodium citrate-disodium citrate mixture, citric acid-trisodium citrate mixture, citric acid-monosodium citrate mixture), succinate buffers (for example, succinic acid-monosodium succinate mixture, succinic acid-sodium hydroxide mixture, succinic acid-disodium succinate mixture), tartrate buffers (for example, tartaric acid-sodium tartrate mixture, tartaric acid-potassium tartrate mixture, tartaric acid-sodium hydroxide mixture), fumarate buffers (for example, fumaric acid-monosodium fumarate mixture, fumaric acid-disodium fumarate mixture, monosodium fumarate-disodium fumarate mixture), gluconate buffers (for example, gluconic acid-sodium gluconate mixture, gluconic acid-sodium hydroxide mixture, gluconic acid-potassium gluconate mixture), oxalate buffers (for example, oxalic acid-sodium oxalate mixture, oxalic acid-sodium hydroxide mixture, oxalic acid-potassium oxalate mixture, and the like), lactate buffers (for example, lactic acid-sodium lactate

mixture, lactic acid-sodium hydroxide mixture, lactic acid-potassium lactate mixture, and the like), and acetate buffers (for example, acetic acid-sodium acetate mixture, acetic acid-sodium hydroxide mixture, and the like). In addition, phosphate buffers, histidine buffers, and trimethylamine salts, such as Tris, may be used.

In an embodiment, the liquid dosage form of the pharmaceutical composition disclosed herein may further comprise a preservative. For example, preservatives suitable for use in the present disclosure include, but are not limited to, phenol, benzyl alcohol, meta-cresol, methyl paraben, propyl paraben, octadecyl dimethyl benzyl ammonium chloride, benzalkonium halides (for example, chloride, bromide, and iodide), hexamethonium chloride, and alkyl parabens (for example, methyl or propyl paraben, catechol, resorcinol, cyclohexanol, and 3-pentanol).

In an embodiment, the liquid dosage form of the pharmaceutical composition disclosed herein may further comprise a stabilizer. For example, suitable stabilizers include, but are not limited to, polyhydric sugar alcohols, sugar alcohols with three or more hydroxyl groups, amino acids, organic sugars or sugar alcohols, polyvinylpyrrolidone monosaccharides, trisaccharides, polysaccharides, proteins, sulfur-containing reducing agents, amino acid polymers, and polyethylene glycol.

In an embodiment, the liquid dosage form of the pharmaceutical composition disclosed herein may further comprise a solubilizer. Specifically, the solubilizer is an ionic surfactant. Examples of nonionic surfactants include, but are not limited to, polysorbates, polyoxamers, pluronic polyols, and polyoxyethylene sorbitan monoethers.

In an embodiment, the pharmaceutical composition disclosed herein may be prepared for storage as a lyophilized formulation or an aqueous solution by mixing the composition with any pharmaceutically acceptable carriers, excipients, or stabilizers typically used in the art (for example, buffering agents, stabilizers, preservatives, isotonic agents, non-ionic detergents, antioxidants, and various other additives).

The term "administration" means providing a substance (for example, a pharmaceutical composition comprising, as an active ingredient, a compound represented by Formula (1) of the present disclosure or a novel crystalline form thereof) to a subject to achieve a prophylactic or therapeutic purpose (for example, a neurological disorder or a neurodegenerative disorder).

Administration may be carried out by any route, including oral, parenteral, and transmucosal (for example, buccal, sublingual, palatal, gingival, nasal, vaginal, rectal, or transdermal). Parenteral administration refers to administration by injection (for example, bolus injection) or infusion. Parenteral administration includes subcutaneous injection, intravenous injection, intramuscular injection, intraarterial injection, intraperitoneal injection, intracerebral injection, intrathecal injection, or intracerebroventricular injection. Other modes of delivery include, but are not limited to, the use of liposomal formulations, transdermal patches, and the like.

In a case where the compound is administered orally, it may be formulated into pills, capsules, sachets, tablets, or the like, together with pharmaceutically acceptable excipients.

Compressed tablets may be prepared by compressing, in a suitable machine, the active ingredient in a free-flowing form such as a powder or granules, optionally mixed with a binder, a lubricant, an inert diluent, a preservative, a surfactant, or a dispersing agent. Molded tablets may be prepared by molding, in a suitable machine, a mixture of the powdered active ingredient moistened with an inert liquid diluent. Tablets may optionally be coated or scored and may optionally be formulated to provide slow or controlled release of the active ingredient. Tablets, troches, lozenges, aqueous or oily suspensions, dispersible powders or granules, emulsions, hard or soft capsules, such as gelatin capsules, syrups, or elixirs may be prepared for oral use. Formulations of the compound of the present disclosure intended for oral use may be prepared according to any method known in the art for the manufacture of pharmaceutical compositions, and such compositions may contain one or more agents including sweetening agents, flavoring agents, coloring agents, and preservatives so as to provide palatable preparations. Tablets containing the active ingredient are suitably mixed with non-toxic, pharmaceutically acceptable excipients suitable for the manufacture of tablets. Such excipients may include, for example, inert diluents such as calcium carbonate or sodium carbonate, lactose, calcium phosphate or sodium phosphate; granulating and disintegrating agents such as corn starch or alginic acid; binders, such as starch, gelatin, or acacia; and lubricants, such as magnesium stearate, stearic acid, or talc. Tablets may be uncoated or coated by known techniques, including microencapsulation, to delay disintegration and absorption in the gastrointestinal tract and provide a sustained action over a longer period. For example, a timedelay substance such as glyceryl monostearate or glyceryl distearate may be used alone or in combination with a wax.

In an embodiment, the pharmaceutical composition of the present disclosure may be administered to a subject, for example, by oral, intracavitary, intradermal, intramuscular, intrathecal, intravenous, subcutaneous, or intracerebroventricular administration.

The pharmaceutical formulation of the present disclosure may be administered to mammals, such as humans, but may also be administered to other mammals, such as animals in need of veterinary treatment, for example, domestic animals (for example, dogs, cats, and the like), farm animals (for example, cattle, sheep, pigs, horses, and the like), and laboratory animals (for example, rats, mice, guinea pigs, and the like).

The active ingredient and its effective dosage in the pharmaceutical composition may vary depending on the rate of absorption, inactivation, and excretion of the drug, as well as other factors known to those skilled in the art. It should also

be noted that the therapeutically effective amount values will vary depending on the severity of the disease to be alleviated. It should further be understood that for any particular subject, specific dosage regimens should be adjusted over time according to the subject's needs and the professional judgment of the person administering or supervising the administration of the composition, and the concentration ranges set forth herein are merely exemplary and are not intended to limit the scope or practice of the claimed compositions. The active ingredient may be administered at once or may be divided into a plurality of smaller doses to be administered at various time intervals.

The term "effective amount" or "therapeutically effective amount" refers to an amount of a compound, composition, or drug sufficient to treat a specified disease, condition, or disorder, such as by enhancing a specified function, for example, enhancing or increasing a desired and/or beneficial function, or reducing or diminishing an undesired function; or ameliorating, temporarily alleviating, attenuating, and/or delaying one or more symptoms.

In some embodiments, the pharmaceutical composition may comprise about 25 mg, about 50 mg, about 75 mg, about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, about 1000 mg, about 1250 mg, about 1500 mg, about 1750 mg, about 2000 mg, about 2250 mg, about 2500 mg, about 2750 mg, or about 3000 mg of the compound.

In some embodiments, the pharmaceutical composition may comprise about 0.01 mg to about 125 mg, preferably about 1 mg to about 50 mg of the compound per kg of the subject's body weight, which is administered one or more times per day to obtain a desired therapeutic effect.

In an embodiment, the composition may comprise the compound in an amount of, for example, 0.0001 to 99.9% by weight, preferably 0.001 to 50% by weight, based on the total weight of the composition. The content ratio is a value based on the dry weight from which the solvent has been removed.

In another embodiment, the composition may comprise a 5% (w/v), 10% (w/v), 15% (w/v), 20% (w/v), 25% (w/v), 30% (w/v), 35% (w/v), or 40% (w/v) aqueous solution of one or more compounds. In some embodiments, the effective amount of the composition may comprise a 25% (w/v) solution of one or more compounds.

**Biological assay**

Methods for selecting crystalline forms useful in the present disclosure are described herein.

Blood-brain barrier permeability is an important property in making decisions to evaluate compounds as potential drug candidates for brain diseases. Drugs often need to cross the blood-brain barrier (BBB) to reach their target of action, and this serves as an important property for evaluating the ability of compounds to passively cross such a membrane. The BBB is formed of brain endothelial cells with tight junctions. Rapid early screening of compounds for BBB penetration is highly desirable in drug discovery.

In an embodiment, the crystalline forms of the present disclosure are selected for their BBB membrane permeability.

Detailed examples of methods useful for selecting the crystalline forms of the present disclosure are described herein.

**Therapeutic uses of compounds of present disclosure**

In still yet another aspect of the present disclosure, there is provided a method for preventing or treating a neurological disorder or a neurodegenerative disorder, comprising administering to a subject in need thereof a crystalline form of the compound represented by Formula (1) disclosed herein.

The term "neurological disorder" includes neurodegenerative diseases, neuropsychiatric disorders, amnesia, cognitive dysfunction/impairment, and extinction learning disorders.

The term "neurodegenerative disorder" refers to any disorder that can be reversed, inhibited, managed, treated, ameliorated, or eliminated by an agent that stimulates the production of new neurons.

The compounds of the present disclosure may be useful for enhancing synaptic plasticity. In an embodiment, the compounds described herein may be therapeutically useful, as reduced synaptic plasticity is also associated with deterioration of brain function in adults. In particular, synaptic plasticity of thalamocortical circuits is significantly limited with aging, and thus reactivating or increasing cortical synaptic plasticity through therapeutic means may be useful for treating diseases caused by reduced synaptic plasticity. Therefore, the crystalline forms described herein may be useful in a case where brain pathology is associated with or mediated by reduced synaptic plasticity.

The compounds of the present disclosure may be useful for inhibiting or removing amyloid-beta protein accumulation. In an embodiment, the compounds described herein may be useful for treating Alzheimer's disease caused by amyloid-beta protein accumulation by inhibiting or eliminating such accumulation. That is, the crystalline forms described herein may be useful in a case where brain pathology is associated with or mediated by amyloid-beta protein accumulation.

Neurogenesis, or the birth of new neurons, was thought to occur only in developing organisms. However, recent

studies have demonstrated that neurogenesis continues into adulthood and throughout adult life. Ongoing neurogenesis is considered an important mechanism underlying neural plasticity that enables organisms to adapt to environmental changes and to influence learning and memory throughout their lives.

In an embodiment, the present disclosure comprises a method for increasing synaptic density in a subject, comprising administering to the subject in need thereof a compound of the present disclosure.

In another embodiment, the present disclosure comprises a method for increasing synaptic plasticity in a subject, comprising administering to the subject in need thereof a compound of the present disclosure.

In yet another embodiment, the present disclosure comprises a method for increasing dendritic density of neurons in a subject, comprising administering to the subject in need thereof a compound of the present disclosure.

The present disclosure provides a method for improving memory in a subject with a memory disorder. Examples of types of memory disorders include, but are not limited to, Alzheimer's disease, forgetfulness, bromism, fugue state, Korsakov's syndrome, prosopamnesia, Ribot's Law, repressed memory, false memory syndrome, memory distrust syndrome, hyperthymesia, amnesia (for example, childhood amnesia, partial amnesia, post-traumatic amnesia, psychogenic amnesia, anterograde amnesia, retrograde amnesia, selective amnesia, source amnesia, transient epileptic amnesia, transient global amnesia, blackout (alcohol-related amnesia)), sourcemonitoring errors, the seven sins of memory, the tip of the tongue phenomenon, and twilight sleep.

In an embodiment, the memory disorder may be Alzheimer's disease. Such methods comprise optionally administering an inhibitor to a subject suffering from Alzheimer's disease and monitoring the subject to determine whether previously lost memories are recovered. The subject may be monitored by routine tests known in the art.

In an embodiment, the Alzheimer's subject may be a subject suffering from late-stage Alzheimer's disease. Many compounds proposed for treating Alzheimer's disease are designed to treat the early stages of the disease by preventing plaque accumulation. The compounds of the present disclosure are useful for treating both early and late-stage dementia because they improve memory and cognition while also preventing plaque accumulation.

In an embodiment, the disease or disorder mediated by reduced synaptic plasticity and/or amyloid-beta protein accumulation may be cognitive dysfunction/impairment. Accordingly, there is also provided a method for enhancing cognitive function in a subject suffering from cognitive dysfunction/impairment, comprising administering to the subject in need thereof an effective amount of at least one compound described herein. The present disclosure also provides a method for enhancing cognitive function in a normal subject.

The "cognitive function" of a subject may be defined as intellectual activities or processes. Examples of intellectual activities or processes include, but are not limited to, attention, processing speed, learning and memory, executive function, verbal fluency, and working memory. For example, the composition of the present disclosure may improve cognitive function when it improves one or more intellectual activities or processes in a subject with impaired cognitive function.

The term "memory" refers to the ability to retrieve information about past events or knowledge.

The term "age-related memory loss" refers to any continuum of conditions characterized by deterioration of neurological function that does not rise to the level of dementia, as further defined herein, and reference is made to the definition set forth in Diagnostic and Statistical Manual of Mental Disorders: 4th Edition of the American Psychiatric Association (DSM-IV, 1994).

The term "injury-related memory loss" refers to memory loss in which there is brain injury and there may also be neurological impairment.

The term "impaired cognitive function" refers to cognitive function that is not as robust as that observed in age-matched normal subjects, and includes a state of reduced cognitive function.

In some cases, cognitive function may be reduced by about 5%, about 10%, about 30%, or more, compared to cognitive function measured in age-matched normal subjects. Cognitive function may be improved to any detectable extent. In humans, it may be preferably improved sufficiently such that a subject with impaired cognitive function can perform normal daily activities.

Cognitive function may be evaluated through one or more tests or assays for cognitive function and may be optionally defined accordingly. Non-limiting examples of tests or assays for cognitive function may include, but are not limited to, CANTAB (for example, see Fray et al. "CANTAB battery: proposed utility in neurotoxicology." Neurotoxicol Teratol 1996; 18(4):499-504), the Stroop Test, Trail Making, the Wechsler Digit Span, or the CogState computerized cognitive test (see Dehaene et al. "Reward-dependent learning in neuronal networks for planning and decision making." Brain Res. 2000; 126:21729; Iverson et al. "Interpreting change on the WAIS-III/ WMS-I11 in clinical samples." Arch Clin Neuropsychol. 2001;16(2):183-91; and Weaver et al. "Mild memory impairment in healthy older adults is distinct from normal aging." Cogn. 2006;60(2):146-55). The method of the present disclosure may be used to enhance cognitive function in a normal subject, to treat and/or alleviate cognitive dysfunction in a subject with cognitive dysfunction, and/or to prevent cognitive dysfunction in a subject. As used herein, a normal subject is a subject not diagnosed with a disorder associated with cognitive impairment.

In an embodiment, cognitive dysfuction or impairment may be associated with, but is not limited to, Alzheimer's

disease, Huntington's disease, seizure-induced memory loss, schizophrenia, Rubinstein-Taybi syndrome, Rett syndrome, fragile X syndrome, Lewy body dementia, vascular dementia, social, cognitive, and learning disorders associated with bipolar disorder and autism spectrum disorder (ASD), attention deficit hyperactivity disorder (ADHD), dyslexia, learning disabilities, traumatic head injury, stroke-induced cognitive and motor impairment, traumatic brain injury, neurodegeneration and neuronal loss-mediated cognitive disorder, and attention deficit disorder.

In an embodiment, the cognitive dysfunction or impairment may be associated with, but is not limited to, anxiety disorders, conditioned fear responses, panic disorder, obsessive-compulsive disorder, post-traumatic stress disorder, phobias, social anxiety disorder, substance dependence recovery, or age-associated memory impairment (AAMI), and age-related cognitive decline (ARCD).

In an embodiment, the disease or disorder mediated by reduced synaptic plasticity and/or amyloid-beta protein accumulation may be extinction learning disorder, for example, fear extinction deficit. Accordingly, there is also provided a method for treating or preventing extinction learning disorder in a subject, comprising administering to the subject suffering from the extinction learning disorder an effective amount of at least one compound described herein.

The compounds of the present disclosure may be used to promote the psychological process of extinction learning and are thus useful for treating, alleviating, and/or preventing neuropsychiatric disorders and other related disorders. Unlike traditional anxiolytic drugs that are administered over a long period and address physiological symptoms of anxiety, the compounds of the present disclosure may be used for a long or short period in conjunction with a second therapy, for example, psychotherapy.

In some embodiments, the disease or disorder mediated by reduced synaptic plasticity and/or amyloid-beta protein accumulation may be an immune disorder that can be cotreated with TNF$\alpha$ or another immunomodulator upon administration of a therapeutically effective amount of at least one compound as described herein to a subject. Accordingly, there is also provided a method for treating an immune disorder mediated by reduced synaptic plasticity and/or amyloid-beta protein accumulation in a subject, comprising administering to the subject in need thereof a therapeutically effective amount of at least one compound described herein before, during, or after treatment with TNF$\alpha$ or another immunomodulator.

In an embodiment, the disease or disorder mediated by reduced synaptic plasticity and/or amyloid-beta protein accumulation includes disorders that can also be treated by precursor/stem cell-based therapies, such as: diabetesrelated disorders (for example, organ failure, cirrhosis, and hepatitis); central nervous system (CNS) disorders associated with dysregulation of progenitor cells in the brain (for example, PTSD); tumors (for example, retinoblastoma); disorders affecting oligodendrocyte progenitor cells (for example, astrocytoma and ependymoma); multiple sclerosis; demyelinating disorders such as leukodystrophy; neuropathies associated with white matter loss; cerebellar disorders such as ataxia; and olfactory progenitor disorders (for example, anosmia). Accordingly, there is also provided a method for treating a disorder mediated by reduced synaptic plasticity and/or amyloid-beta protein accumulation in a subject, comprising administering to the subject in need thereof a therapeutically effective amount of at least one compound described herein before, during, or after treatment with a progenitor/stem cell-based therapy.

In an embodiment, the disease or disorder mediated by reduced synaptic plasticity and/or amyloid-beta protein accumulation may include blood pressure disorders associated with nitric oxide (NO) regulation (for example, hypertension, erectile dysfunction, asthma; and ocular disorders such as glaucoma). Accordingly, there is also provided a method for treating a blood pressure disorder associated with nitric oxide (NO) regulation mediated by reduced synaptic plasticity and/or amyloid-beta protein accumulation in a subject, comprising administering to the subject in need thereof a therapeutically effective amount of at least one compound described herein.

In an embodiment, the disease or disorder may be a cardiac hypertrophy disorder. Accordingly, there is also provided a method for treating a cardiac hypertrophy disorder mediated by reduced synaptic plasticity and/or amyloid-beta protein accumulation in a subject, comprising administering to the subject in need thereof a therapeutically effective amount of at least one compound described herein.

In an embodiment, the disease or disorder mediated by reduced synaptic plasticity and/or amyloid-beta protein accumulation may include hematological disorders, such as thalassemia, anemia, and sickle cell anemia. Accordingly, there is also provided a method for treating a hematological disorder mediated by reduced synaptic plasticity and/or amyloid-beta protein accumulation in a subject, comprising administering to the subject in need thereof a therapeutically effective amount of at least one compound described herein.

In an embodiment, the disease or disorder mediated by reduced synaptic plasticity and/or amyloid-beta protein accumulation may include metabolic diseases such as prediabetes or diabetes (type I or II). Accordingly, there is also provided a method for treating a metabolic disease, such as prediabetes or diabetes (type I or II), mediated by reduced synaptic plasticity and/or amyloid-beta protein accumulation in a subject, comprising administering to the subject in need thereof a therapeutically effective amount of at least one compound described herein.

In an aspect of the present disclosure, there is provided a pharmaceutical composition or kit further comprising a second therapeutic agent for the treatment of a disease or disorder mediated by reduced synaptic plasticity and/or amyloid-beta protein accumulation in a subject suffering from such a disease or disorder. In an embodiment, the second

therapeutic agent may be co-administered to treat a disease or disorder mediated by reduced synaptic plasticity and/or amyloid-beta protein accumulation.

The term "co-administration" may be intended to mean that the composition described herein is administered simultaneously with, immediately before, or immediately after the application of one or more additional therapies (for example, an anticancer agent, a chemotherapeutic agent, or a therapeutic agent for neurodegenerative diseases). In an embodiment, the compounds of the present disclosure may be administered alone or co-administered to a subject. Co-administration is intended to include simultaneous or sequential administration of the compounds individually or in combination (one or more compounds or preparations). Accordingly, the preparation may also be combined with other active agents if desired (for example, to reduce metabolic degradation).

In addition, there is provided a method for treating a disease or disorder mediated by reduced synaptic plasticity and/or amyloid-beta protein accumulation in a subject suffering from such a disease or disorder, wherein at least one compound described herein or a pharmaceutically acceptable salt thereof may be administered to the subject in combination with one or more additional active agents (second therapeutic agents).

In an embodiment, the second therapeutic agent may be a drug for the prevention or treatment of a neurological disorder or a neurodegenerative disorder. Preferably, it may be a drug that exhibits a synergistic effect when used together with the compounds of the present disclosure, or a drug that can improve prophylactic or therapeutic effects by complementing each other.

In an embodiment, the second therapeutic agent may be selected from the group consisting of, but not limited to, cholinesterase inhibitors, NMDA receptor antagonists, humanized antibodies targeting tau protein, humanized antibodies targeting amyloid-beta protein, and BACE inhibitors. The humanized antibody targeting tau protein or the humanized antibody targeting amyloid-beta protein may be an antibody that inhibits or reduces the accumulation of tau protein or amyloid-beta protein, respectively.

In an embodiment, the second therapeutic agent may be selected from the group consisting of, but not limited to, donepezil, rivastigmine, galantamine, memantine, verubecestat, solanezumab, bapineuzumab, aducanumab, lecanemab, tideglusib, epothilone D, and ABBV-8E12.

Typical therapies included in the present disclosure comprise combination therapy. For example, the combination therapy may be pharmacotherapy (i.e., compounds of the present disclosure) and behavioral therapy. Behavioral therapy includes, but is not limited to, electroconvulsive therapy, exercise, group therapy, talk therapy, or conditioning. In another embodiment, the behavioral therapy may be cognitive-behavioral therapy. Examples of the behavioral therapy that may be used in the ongoing methods can be found, for example, in Cognitive-Behavioral Therapies by K. Dobson, ed., Guilford Publications, Inc., 2002, and The New Handbook of Cognitive Therapy: Basics and Beyond by Judith S.S. Beck, Guilford Publications, Inc., 1995, which are incorporated herein by reference in their entirety.

In addition, any pharmaceutically active ingredient may be used in the treatment method of the present disclosure as a pharmacological agent for enhancing learning or conditioning, and such a pharmacological agent may be appropriately selected by those skilled in the art. For example, one class of pharmacologically active ingredients contemplated herein includes compounds that increase the level of norepinephrine in the brain. Such compounds may include compounds that act as norepinephrine reuptake inhibitors (for example, tomoxetine, reboxetine, duloxetine, venlafaxine, and milnacipran) and compounds that induce the release of norepinephrine (for example, amphetamine, dextroamphetamine, pemoline, and methylphenidate). Another class of such pharmacologically active ingredients may be compounds that increase the level of acetylcholine in the brain, and may include, for example, compounds that block the degradation of acetylcholine. Examples of such compounds may include, but are not limited to, Aricept (generic name: donepezil hydrochloride) and Cognex (generic name: tacrine), which are inhibitors of cholinesterase activity.

Methods for combination therapy may comprise subjecting a subject to one or more sessions of a combination therapy protocol, wherein the combination therapy protocol may comprise acute administration of a therapeutically effective amount of a compound of the present disclosure, which enhances learning or conditioning, in conjunction with a psychotherapy session. In an embodiment, exposure to the compound may occur within about 24 hours prior to the start of the psychotherapy session, preferably within about 12 hours prior to the start of the psychotherapy session, and more preferably within about 6 hours prior to the start of the psychotherapy session. The entire course of treatment for a neuropsychiatric disorder may involve at least one session of such a combination therapy protocol.

Hereinafter, the present disclosure will be described in more detail by way of the following Examples. The following Examples are presented to facilitate understanding of the present disclosure and are not intended to be, nor should they be construed as, limiting its scope in any way.

Methods for obtaining the compounds described herein or pharmaceutically acceptable salts thereof will be apparent to those skilled in the art, and suitable procedures are described, for example, in the following preparative examples and in the references cited herein.

## Examples

Representative examples of polymorphs included within the scope of the present disclosure are described in the following examples. The polymorphs described in the following Preparation Examples are provided to enable those skilled in the art to more clearly understand and practice the present disclosure.

In general, the nomenclature used in the present disclosure is based on AUTONOM (trademark) v.4.0, a computerized system of Beilstein Institute for generating IUPAC systematic nomenclature. In the event of a discrepancy between a depicted structure and a name given to the structure, greater weight shall be given to the depicted structure. Furthermore, if the stereochemistry of a structure or a portion thereof is not indicated, for example, by bold or dashed lines, the structure or portion thereof shall be construed as encompassing all stereoisomers thereof.

## Experimental Methods

### Experimental Method 1. Nuclear magnetic resonance (NMR)

Instrument model: Bruker Avance-AV 400M (for $^1$H-NMR)
Detailed $^1$H-NMR parameters are as follows.
Frequency: 400 MHz
Probe: 5 mm PABBO BB/19F-1H/D Z-GRD Z108618/0406
Number of scans: 8
Temperature: about 24°C
Relaxation time: 1 second

### Experimental Method 2. X-ray powder diffraction (XRPD)

Instrument model: Bruker D8 Advance X-ray Diffractometer
Detailed XRPD parameters are as follows.
X-ray tube: Cu, k$\alpha$1 ($\lambda$=1.54056Å)
X-ray tube voltage: 40 kV, X-ray tube current: 40 mA
Scan range: 2 to 40 degrees or 3 to 40 degrees
Step width: 0.02 degrees
Step time: 0.3 seconds or 0.12 seconds
Sample stage rotation speed: 15 rpm

### Experimental Method 3. Differential scanning calorimetry (DSC)

Instrument model: TA Discovery 2500 Differential Scanning Calorimeter
A sample (about 1 to 2 mg) is placed in a DSC aluminum pan and tested. Under a nitrogen flow of 50 mL/min, the sample is heated from 0°C to 250°C at a heating rate of 10°C/min.

### Experimental Method 4. Thermalgravimetric analysis (TGA)

Instrument model: Discovery 5500 Thermalgravimetric Analyzer
A sample (about 2 to 10 mg) is placed in a TGA platinum pan and tested. Under a nitrogen flow of 25 mL/min, the sample is heated from room temperature to 300°C or until a 20% weight loss is reached, at a heating rate of 10°C/min.

### Experimental Method 5. Dynamic vapor sorption (DVS)

Instrument model: SMS DVS Advantage Dynamic Vapor Sorption System

Test method: A sample (10 to 15 mg) is taken and placed on a DVS sample pan, and measurement is performed.
Detailed DVS parameters are as follows.
Temperature: 25°C
Equilibrium dm/dt = 0.002%/min: (minimum: 60 min, maximum: 240 min)
Relative humidity (RH (%)) measurement gradient: 10%
Relative Humidity (RH (%)) measurement gradient cycle: 40-0-95-0-40%
The following description provides the criteria for determining hygroscopicity:

[Table 6]

| Classification of hygroscopicity | Weight increase due to moisture absorption* |
|---|---|
| Deliquescent | Absorbs sufficient moisture to form a liquid |
| Very hygroscopic | Weight increase due to moisture absorption is 15% or more |
| Hygroscopic | Weight increase due to moisture absorption is less than 15% and 2% or more |
| Slightly hygroscopic | Weight increase due to moisture absorption is less than 2% and 0.2% or more |
| Non-hygroscopic or almost non-hygroscopic | Weight increase due to moisture absorption is less than 0.2% |
| * indicates weight increase due to moisture absorption at 25°C/80% RH. | |

**Experimental Method 6. High performance liquid chromatography (HPLC)**

The HPLC analysis method is as shown in Table 7.

[Table 7]

| **Instrument** | **SHIMADZU LC-20AD** | | |
|---|---|---|---|
| Chromatographic column | C18, 150 x 4.6 mm, 5 μm (PDS-HPLC-010) | | |
| Detector | DAD | | |
| Mobile phase A | 0.0375% Trifluoroacetic acid aqueous solution | | |
| Mobile phase B | 0.01875% Trifluoroacetic acid acetonitrile solution | | |
| Flow rate | 1.5 mL/min | | |
| Sample injection volume | 10 μL | | |
| Detection wavelength | 220 nm | | |
| Column temperature | 50°C | | |
| Diluent | Ethanol | | |
| Gradient elution procedure | Time (min) | Mobile phase A (%) | Mobile phase B (%) |
| | 0.00 | 10 | 1.5 |
| | 4.20 | 80 | 1.5 |
| | 5.30 | 80 | 1.5 |
| | 5.31 | 10 | 1.5 |
| | 6.00 | 10 | 1.5 |

**Experimental Method 7. Parallel artificial membrane permeability assay (PAMPA)**

- PAMPA: PMBBB-096 Kit
- Assay format: 96-well plate
- Reference substance: Imipramine (H) (10 μM)
- Test substance concentration: 10 μM
- Number of replicates: 3
- Reaction time: 6 hours at 25°C on a shaker (75 - 80 rpm)
- Analytical method: LC-MS/MS
- Data processing: $P_e$ (cm/s)

**Example 1. Preparation of compound of Formula (1)**

By way of non-limiting examples, the compound of Formula (1) ((3S,6R)-1-(2-(2-chloro-4-fluorophenyl)acetyl)-6-methylpiperidine-3-carboxylic acid) may be prepared as shown in Scheme 1 below, as well as in the examples disclosed herein.

Formula (1)

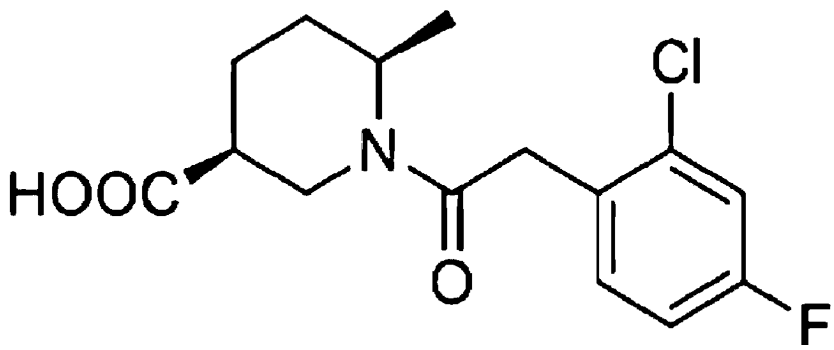

[Scheme 1]

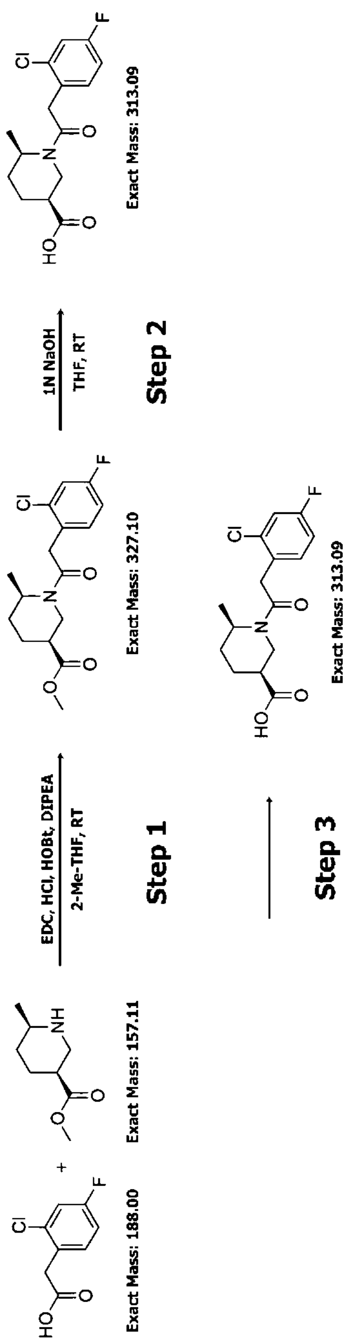


**Example 1.1. Preparation of methyl (3S,6R)-1-(2-(2-chloro-4-fluorophenyl)acetyl)-6-methylpiperidine-3-carboxylate (Step 1)**

2-(2-Chloro-4-fluorophenyl)acetic acid (1.7 kg), (3S,6R)-methyl 6-methylpiperidine-3-carboxylate (1.05 eq.), DIPEA (1.5 eq.), HOBt (0.3 eq.), and EDC HCl (1.2 eq.) were added to a solution of 2-MeTHF (10 V). The mixture was heated at 25°C to 35°C for 1.3 hours. The mixture was washed twice with $H_2O$ (5 V) to obtain methyl (3S,6R)-1-(2-(2-chloro-4-fluorophenyl)acetyl)-6-methylpiperidine-3-carboxylate with a purity of 99.6%.

**Example 1.2. Preparation of ((3S,6R)-1-(2-(2-chloro-4-fluorophenyl)acetyl)-6-methylpiperidine-3-carboxylic acid) (Step 2)**

Methyl (3S,6R)-1-(2-(2-chloro-4-fluorophenyl)acetyl)-6-methylpiperidine-3-carboxylate was scaled up to 2.953 kg. Methyl (3S,6R)-1-(2-(2-chloro-4-fluorophenyl)acetyl)-6-methylpiperidine-3-carboxylate was added to 2-MeTHF (10 V) with 1 N NaOH (1.3 eq.). The mixture was heated at 25°C for 3 hours. Crystallization from 2-MeTHF/n-heptane yielded

2.754 kg of (3S,6R)-1-(2-(2-chloro-4-fluorophenyl)acetyl)-6-methylpiperidine-3-carboxylic acid with impurities of 0.1% or less.

**Example 1.3. Purification of ((3S,6R)-1-(2-(2-chloro-4-fluorophenyl)acetyl)-6-methylpiperidine-3-carboxylic acid) (Step 3)**

(3S,6R)-1-(2-(2-chloro-4-fluorophenyl)acetyl)-6-methylpiperidine-3-carboxylic acid (2.57 kg) was added to EA (5 V) and n-heptane (15 V). The mixture was heated from 0°C to 45°C to yield 2.404 kg of a dried cake as a white solid.

**Example 2. Preparation of crystalline form A (anhydrous) of compound of Formula (1)**

Crystalline form A of the compound of Formula (1) was prepared by the following method.

(1) Equilibration in MEK, MTBE, IPAc, THF/heptane (v:v=1:1), 2-MeTHF/heptane (v:v=1:1), DMSO/water (v:v=24:76; a.w.=0.9) and DMF/water (v:v=31:69; a.w.=0.9) at 25°C for 2 weeks or at 50°C for 1 week,
(2) equilibration in acetone or DCM/heptane (v:v=1:1) at 25°C for 2 weeks,
(3) equilibration in toluene at 50°C for 1 week,
(4) equilibration in acetone, ACN, or 1,4-dioxane/heptane (v:v=1:1) under temperature cycling between 5°C and 50°C at a heating/cooling rate of 0.1°C/min for 10 cycles,
(5) slow evaporation (about 20 to 25°C, 40 to 70% RH) and fast evaporation (using dry nitrogen at about 20 to 25°C) in most solvent systems (methanol, ethanol, acetone, ACN, and the like),
(6) slow cooling in acetone (dissolving in a minimal amount of solvent at 50°C, followed by filtration through a 0.45 μm syringe membrane filter, cooling the resulting clear solution to 5°C at a rate of 0.1°C/min, and further cooling to -20°C),
(7) rapid cooling in acetone or ACN (dissolving in a minimal amount of solvent at 50°C, followed by filtration through a 0.45 μm syringe membrane filter, and stirring the resulting clear solution in an ice bath at 0°C),
(8) crystallization by addition of an antisolvent (water) in ethanol (dissolving in a minimum amount of solvent at ambient temperature, followed by filtration through a 0.45 μm syringe membrane filter, and adding 4 to 8 volumes of antisolvent to the resulting clear solution to form a precipitate), and
(9) crystallization by vapor diffusion using 1,4-dioxane, MEK, or acetone (dissolving in a minimum amount of solvent at ambient temperature of about 20 to 25°C, followed by filtration through a 0.45 μm syringe membrane filter, transferring the resulting clear solution to a capless 4 ml glass vial, transferring the 4 ml vial into a 40 ml glass vial, and adding an antisolvent (heptane, heptane, or MTBE, respectively); and then tightly capping the vial and allowing it to stand at ambient temperature for up to 14 days).

In the above method, equilibration was performed using a stirring bar on a magnetic stirring plate at 300 to 400 rpm. The finally obtained precipitate was centrifuged through a 0.45 μm syringe membrane filter at 14,000 rpm to obtain crystalline form A as a solid.

Meanwhile, crystalline forms B, C, D, and E of the compound of Formula (1) were prepared from crystalline form A, and a schematic diagram of the interconversion relationship among crystalline forms A, B, C, D, and E is shown in Fig. 6. Specific preparation methods for crystalline forms B, C, D, and E of the compound of Formula (1) are described in detail in the following examples.

The obtained crystalline form A was analyzed by solubility analysis and by X-ray powder diffraction (XRPD), nuclear magnetic resonance ($^1$H-NMR), differential scanning calorimetry (DSC), and thermogravimetric analysis (TGA) according to the methods described in Experimental Methods 1 to 4. The results are shown in Figs. 1a to 1d, respectively.

As a result of XRPD, it was confirmed to have peaks at diffraction angles 2θ(°) of 4.96, 9.88, 12.09, 13.4, 14.85, 16.2, 18.4, 19.65, 19.83, 20.06, 20.49, 21.47, 22.49, 22.51, 24.04, 24.45, 24.84, 25.23, 27.39, 28.68, 29.64, 30.01, 31.15, 31.5, 35.03, and 36.77. The XRPD results indicate high crystallinity of crystalline form A (Fig. 1a).

A single melting peak (128.3°C) with an enthalpy of about 61 J/g was confirmed through the DSC results (Fig. 1c). Through the TGA results, it was confirmed that crystalline form A had a weight loss of 0.7% upon heating from ambient temperature to 120°C (Fig. 1d). It was confirmed through the $^1$H-NMR results that there was no detectable residual solvent (Fig. 1b).

<u>Solubility assay</u>

For solubility assay, about 5 mg or about 10 mg of crystalline form A was weighed into a 2 mL glass vial. A 20 μL aliquot of each of the 17 solvents listed in Table 8 was added to the vial to dissolve the drug substance. For about 5 mg of crystalline form A, the drug substance was dissolved at 25°C; and for about 10 mg of crystalline form A, the drug substance was dissolved at 50°C. To aid dissolution, vortexing and sonication were applied to facilitate dissolution, and the maximum

volume of each solvent added was 1 mL. The solubility results are shown in Table 8.

[Table 8]

| Solubility of crystalline form A at 25°C and 50°C | | | |
|---|---|---|---|
| No. | Solvent | Solubility (mg/mL) | |
| | | 25°C | 50°C |
| 1 | Water | <5 | <10 |
| 2 | Methanol | >250 | >500 |
| 3 | Ethanol | 125-250 | >500 |
| 4 | Acetone | 125-250 | >500 |
| 5 | Acetonitrile | 41-50 | >500 |
| 6 | Methyl ethyl ketone | 83-125 | 250-500 |
| 7 | Tetrahydrofuran | >250 | >500 |
| 8 | Ethyl acetate | 41-50 | 250-500 |
| 9 | MTBE | 16-20 | 33-50 |
| 10 | Dichloromethane | >250 | >500 |
| 11 | 1,4-Dioxane | >250 | >500 |
| 12 | Heptane | <5 | <10 |
| 13 | 2-MeTHF | >250 | >500 |
| 14 | Isopropyl acetate | 63-83 | 125-167 |
| 15 | DMSO | >250 | >500 |
| 16 | DMF | >250 | >500 |
| 17 | Toluene | 17-25 | 50-100 |

**Example 3. Preparation of crystalline form B (anhydrous) of compound of Formula (1)**

Crystalline form B of the compound of Formula (1) was obtained from crystalline form A through slurry equilibration of salt screening in ACN and EA. The obtained solid was also analyzed by XRPD, DSC, and TGA, and the results are shown in Figs. 2a to 2c, respectively.

As a result of XRPD, it was confirmed to have peaks at diffraction angles 2θ(°) of 8.22, 8.62, 8.64, 9.09, 9.12, 11.88, 14.21, 14.7, 15.09, 15.78, 16.48, 18.01, 18.28, 19.26, 19.99, 20.34, 20.67, 22.14, 22.61, 22.9, 23.88, 24.49, 24.78, 25.86, 26.4, 26.7, 26.95, 27.58, 27.77, 28.11, 28.89, 29.05, 32.59, and 36.46 (Fig. 2a). The XRPD results indicate high crystallinity of crystalline form B. A single melting peak (111.4°C) with an enthalpy of about 64 J/g and a single endothermic peak (126.8°C) with an enthalpy of about 2 J/g were confirmed through the DSC results (Fig. 2b). Through the TGA results, it was confirmed that crystalline form B had a weight loss of 0.4% upon heating from ambient temperature to 110°C (Fig. 2c). Crystalline form B exhibited low reproducibility.

**Example 4. Preparation of crystalline form C (anhydrous) of compound of Formula (1)**

Crystalline form C was prepared by the following method: (1) Equilibration in ACN or EA at 25°C for 2 weeks, (2) equilibration in EA under temperature cycling between 5°C and 50°C at a heating/cooling rate of 0.1°C/min for 10 cycles, (2) rapid cooling in MEK, EA, MTBE, IPAc, THF/heptane (v:v=1:1), 2-MeTHF/heptane (v:v=1:1), and acetone/water (v:v=35:65, a.w.=0.9), (3) slow cooling in EA, (4) addition of an antisolvent (water or heptane, respectively) in acetone or EA, (4) competitive equilibration of forms A and C in acetone, ACN, EA, IPAc, and acetone/water (v:v=35:65, a.w.=0.9) at 50°C or lower.

In the above method, equilibration, rapid cooling, slow cooling, addition of the antisolvent, and recovery of the final precipitate were performed in the same manner as described for the preparation of crystalline form A.

The specific method for competitive equilibration is as follows. 500 mg of crystalline form A was weighed into an 8 mL glass vial. 2 mL of EA was added to the vial at ambient temperature (about 20 to 25°C) (suspension). About 10 mg of

crystalline form C seeds (crystalline form A) were added to the suspension. The suspension was continuously stirred at 25°C for about 2 days (suspension). The suspension was cooled to 5°C and continuously stirred at 5°C for about 2 days (suspension). For the competitive equilibrium experiment, about 156 mg of crystalline form C was isolated by centrifugation. 300 mg of crystalline form A was added to the suspension (suspension). The suspension was heated to 40°C and continuously stirred at 40°C for about 3 days (suspension). The solid was collected by filtration. About 397 mg of crystalline form C was obtained as an off-white solid in a yield of 49.6%.

The obtained solid was also analyzed by XRPD, $^1$H-NMR, DSC, and TGA, and the results are shown in Figs. 3a to 3d, respectively.

As a result of XRPD, it was confirmed to have peaks at diffraction angles 2θ(°) of 8.55, 8.57, 10.03, 15.06, 15.96, 17.18, 17.96, 17.98, 20.17, 21.05, 23.38, 25.51, 26.06, 26.43, and 34.24 (Fig. 3a). The XRPD results indicate high crystallinity of crystalline form C.

A single melting peak (116.1°C) with an enthalpy of about 76 J/g was confirmed through the DSC results (Fig. 3c). Through the TGA results, it was confirmed that crystalline form C had a weight loss of 0.3% upon heating from ambient temperature to 110°C (Fig. 3d).

**Example 5. Preparation of crystalline form D (toluene solvate) of compound of Formula (1)**

Crystalline form D of the compound of Formula (1) was obtained from crystalline form A through equilibration at 25°C and temperature cycling in toluene. The temperature cycling was performed under temperature cycling between 5°C and 50°C at a heating/cooling rate of 0.1°C/min in 0.1 to 0.2 mL of solvent for 10 cycles. Equilibration was performed using a stirring bar on a magnetic stirring plate at a speed of 300 to 400 rpm. The obtained solid was also analyzed by XRPD, $^1$H-NMR, DSC, and TGA, and the results are shown in Figs. 4a to 4d, respectively.

As a result of XRPD, it was confirmed to have peaks at diffraction angles 2θ(°) of 8.76, 9.52, 10.72, 12.5, 15.36, 15.65, 16.1, 16.69, 17.14, 17.57, 18.32, 19.1, 21, 21.53, 22.16, 22.51, 22.88, 23.41, 25.07, 25.25, 26.99, 27.37, 27.78, 28.64, 29.81, 30.87, 32.09, 32.56, and 34.07 (Fig. 4a). The XRPD results indicate high crystallinity of crystalline form D.

A desolvation peak (about 62°C), a single endothermic peak (114.4°C) with an enthalpy of about 39 J/g, and a single endothermic peak (128.1°C) with an enthalpy of about 22 J/g were confirmed through the DSC results (Fig. 4c). Through the TGA results, it was confirmed that crystalline form D had a weight loss of about 4.8% upon heating from ambient temperature to about 80°C, and a weight loss of about 2.5% upon heating from about 80°C to 170°C (Fig. 4d). The $^1$H-NMR results indicate the presence of 5.5% by weight of toluene residue (0.2 equivalents of toluene on a molar basis) (Fig. 4b).

**Example 6. Preparation of crystalline form E (ethanol solvate) of compound of Formula (1)**

Crystalline form E of the compound of Formula (1) was obtained by cooling crystalline form A in ethanol to -20°C. The obtained solid was also analyzed by XRPD, $^1$H-NMR, DSC, and TGA, and the results are shown in Figs. 5a to 5d, respectively.

As a result of XRPD, it was confirmed to have peaks at diffraction angles 2θ(°) of 4.93, 7.16, 9.86, 10.17, 11.29, 13.38, 14.81, 16.1, 16.36, 17.88, 18.33, 18.59, 19.62, 19.82, 20.03, 20.45, 20.82, 21.03, 21.46, 22.46, 22.81, 23.54, 24.01, 24.42, 24.81, 25.2, 25.42, 26.1, 27.35, 28.63, 28.86, 29.53, 29.84, 31.23, 32.52, 32.81, and 39.47 (Fig. 5a). The XRPD results indicate high crystallinity of crystalline form E. A desolvation peak (about 40°C) was confirmed through the DSC results (Fig. 5c). Through the TGA results, it was confirmed that crystalline form E had a weight loss of 10.2% upon heating from ambient temperature to 130°C. The $^1$H-NMR results indicate the presence of 9.3% by weight of ethanol residue (0.7 equivalents of ethanol on a molar basis) (Fig. 5b).

**Example 7. Competitive equilibrium experiments for crystalline forms A and C**

Competitive equilibrium experiments were performed in various solvent systems to determine the relative stability of crystalline forms A and C.

About 15 mg of crystalline form A and 15 mg of crystalline form C were added to 0.2 mL of a saturated solution of each of selected solvents (acetone, acetonitrile, ethyl acetate, isopropyl acetate, and acetone/water (v:v=35:65)) (Table 9). The obtained suspensions were stirred at 5°C, 25°C, and 50°C, respectively, for 1 week. The solid portion (wet cake) was isolated by centrifugal filtration and analyzed by XRPD and DSC, and the results are shown in Figs. 7a to 7f.

[Table 9]

Competitive equilibrium experimental conditions for crystalline forms A and C

Competitive equilibrium experimental conditions for crystalline forms A and C

| Exp. ID | Solvent | XRPD | | | | | |
|---|---|---|---|---|---|---|---|
| | | 5°C | | 25°C | | 50°C | |
| | Initial form | Crystaline form A+C (1:1) | | | | | |
| CE1 | Acetone | Crystaline form A+C (4 days) Fig. 6a | Crystaline form C (1 week) Fig. 6b | Crystaline form C (4 days) Fig.6c | // | Crystaline form C (4 days) Fig.6e | // |
| CE2 | Acetonitrile | Crystaline form A+C (4 days) Fig. 6a | Crystaline form C (1 week) Fig. 6b | Crystaline form C (4 days) Fig.6c | Crystaline form C (1 week) Fig.6d | Crystaline form A+C (6 days) Fig.6e | Crystaline form C (1 week) Fig.6f |
| CE3 | Ethyl acetate | Crystaline form C (4 days) Fig. 6a | // | Crystaline form C (4 days) Fig.6c | // | Crystaline form C (4 days) Fig.6e | // |
| CE4 | Isopropyl acetate | Crystaline form A+C (4 days) Fig. 6a | Crystaline form C (10 days) Fig. 6b | Crystaline form A+C (4 days) Fig.6c | Crystaline form C (1 week) Fig.6d | Crystaline form A+C (4 days) Fig.6e | Crystaline form C (1 week) Fig.6f |
| CE5 | Acetone/water (v:v=35:65) a.w.=0.9 | Crystaline form A+C (4 days) Fig. 6a | Crystaline form C (1 week) Fig. 6b | Crystaline form A+C (4 days) Fig.6c | Crystaline form C (1 week) Fig.6d | Crystaline form C (4 days) Fig.6e | // |
| "//": Not performed | | | | | | | |

Through the XRPD results, it was confirmed that crystalline form C was obtained in all solvents listed in Table 9, suggesting that crystalline form C is the most thermodynamically stable crystalline form below 50°C.

**Example 8. Bulk stability of crystalline form C**

Crystalline form C was placed in an open container at 25°C/92.5% RH, in an open container at 40°C/75% RH, and in a closed container at 60°C for 1 week, respectively. After exposure to the stress conditions, the samples were characterized by XRPD and HPLC, and examined for color changes.

Reference is made to Experimental Method 6 for the HPLC conditions. The HPLC analysis results are shown in Table 10, and the XRPD analysis results are shown in Fig. 8.

[Table 10]

| Bulk stability results of crystalline form C | | | |
|---|---|---|---|
| **Exp. ID** | **Experimental conditions** | **Purity (HPLC) Initial purity:99.9%** | **Color** |
| BS1 | Solid state, 25°C/92.5% RH, open container, 1 week | 99.9% | No color change |
| BS2 | Solid state, 40°C/75% RH, open container, 1 week | 99.9% | No color change |
| BS3 | Solid state, 60°C, closed container, 1 week | 99.9% | No color change |

From the HPLC and XRPD results, it can be seen that crystalline form C exhibits high bulk stability both physically and chemically.

**Example 9. Solubility of crystalline form C**

About 4 mg of crystalline form C was weighed into a 4 mL or 8 mL glass vial, and 4 to 9 mL of dissolution medium was added to dissolve the drug substance at 25°C. The approximate solubility was determined by visual observation.

The solvents and solubility results are shown in Table 11 (solubility of crystalline form C at 37°C).

[Table 11]

| Exp. ID | Solvent | Solubility (mg/mL) |
|---|---|---|
| ES1 | pH 1.2 HCl buffer (0.2N) | 0.5-0.6 |
| ES2 | pH 4.5 acetate buffer (50mM) | 0.9-1.0 |
| ES3 | pH 6.8 phosphate buffer (50mM) | 1.8-2.0 |
| ES4 | SGF, pH 2.0 | 0.4-0.5 |
| ES5 | FaSSIF-v1, pH 6.5 | 1.8-2.0 |
| ES6 | FeSSIF-v1, pH 5.0 | 1.8-2.0 |
| ES7 | Water | 0.6-0.7 |

**Example 10. Hygroscopicity of crystalline form C**

The moisture adsorption and desorption behavior of crystalline form C was investigated by the DVS test of Experimental Method 5. XRPD was performed to confirm any change in form after the DVS test.

The DVS results are shown in Table 12 (moisture adsorption and desorption experiments of crystalline form C (DVS)), Fig. 9a, and Fig. 9b, and the XRPD results after the DVS tests are shown in Fig. 9c.

[Table 12]

| **Method** | **40-0-95-0-40%RH, dm/dt 0.002, minimum equilibration time of 60 min, maximum equilibration time of 240 min, 25°C** | | | |
|---|---|---|---|---|
| Relative humidity at 25°C | Weight change (%) in 1st desorption | Weight change (%) in 1st adsorption | Weight change (%) in 2nd desorption | Weight change (%) in 2nd adsorption |
| 0% | 0 | 0 | 0 | 0 |
| 10% | 0 | 0 | 0 | 0 |

(continued)

| Method | 40-0-95-0-40%RH, dm/dt 0.002, minimum equilibration time of 60 min, maximum equilibration time of 240 min, 25°C | | | |
|---|---|---|---|---|
| Relative humidity at 25°C | Weight change (%) in 1st desorption | Weight change (%) in 1st adsorption | Weight change (%) in 2nd desorption | Weight change (%) in 2nd adsorption |
| 20% | 0 | 0 | 0 | 0 |
| 30% | 0 | 0 | 0 | 0 |
| 40% | 0 | 0 | 0.1 | 0 |
| 50% | Not applicable | 0.1 | 0.1 | Not applicable |
| 60% | Not applicable | 0.1 | 0.1 | Not applicable |
| 70% | Not applicable | 0.1 | 0.1 | Not applicable |
| 80% | Not applicable | 0.1 | 0.1 | Not applicable |
| 90% | Not applicable | 0.1 | 0.1 | Not applicable |
| 95% | Not applicable | 0.1 | 0.1 | Not applicable |

From the DVS results, it can be seen that crystalline form C is non-hygroscopic, with a moisture uptake of less than 0.2% up to 95% RH. In addition, form the XRPD results, it can be seen that the sample obtained after the DVS test still exhibits crystalline pattern C.

**Example 11. Mechanical strength of crystalline form C**

To test the mechanical strength of crystalline form C, compression simulation experiments, dry grinding simulation experiments, and wet granulation simulation experiments were conducted.

Compression simulation experiment

About 5 mg of crystalline form C was compressed for 5 minutes at 2 MPa, 5 MPa, and 10 MPa using a hydraulic press. Potential changes in form and crystallinity were evaluated by XRPD.
The XRPD results of the compression simulation experiments are shown in Fig. 10, where CSE1, CSE2, and CSE3 represent experiment IDs for pressures of 2 MPa, 5 MPa, and 10 MPa, respectively.

Dry grinding simulation experiment

About 5 mg of crystalline form C was manually ground for 1, 3, and 5 minutes using a mortar and pestle. Potential changes in form and crystallinity were evaluated by XRPD.
The XRPD results of the dry grinding simulation experiments are shown in Fig. 11, where GSE1, GSE2, and GSE3 represent experiment IDs for grinding for 1 minute, 3 minutes, and 5 minutes, respectively.

Wet granulation simulation experiment

Water or ethanol was added dropwise to about 10 mg of crystalline form C until it was sufficiently wetted. The wet sample was gently ground using a mortar and pestle. After the granulation, the sample was dried under ambient conditions for 10 minutes. Potential changes in form and crystallinity were evaluated by XRPD.
The XRPD results of the wet granulation simulation experiments are shown in Fig. 12, where GNSE1 and GNSE2 represent experiment IDs for water and ethanol, respectively.
The experimental results show that crystalline form C exhibited excellent resistance to compression and granulation with water without any change in form or apparent decrease in crystallinity. On the other hand, during grinding or granulation with ethanol, crystalline form C showed no change in form, but its crystallinity decreased significantly.

**Example 12. BBB permeability of crystalline forms A and C**

To confirm the BBB permeability of crystalline forms A and C of the present disclosure, a parallel artificial

membrane permeability assay (PAMPA) was performed. Reference is made to Experimental Method 7 for the specific experimental conditions.

PAMPA assay

The test compounds (crystalline forms A and C) were dissolved in DMSO at a concentration of 10 mM (1 mg/mL) to prepare standard solutions. Next, dried brain lipids were resuspended in 600 μL dodecane to prepare a BBB lipid solution in dodecane. Pipetting up and down was repeated (~100 times) until all dried brain lipids were dissolved (vortexing or sonication may be helpful). A 10 μM reaction solution in PBS containing 5% DMSO was prepared.

The experiments were conducted in a 96-well plate using the prepared reaction solution of each test compound and the BBB lipid solution according to the following assay procedure.

1. 500 μL of a 500 μM test compound solution (prepared by mixing 25 μL of a 10 mM test compound + 475 μL of PBS (pH 7.2) in DMSO) was prepared in a separate centrifuge tube. When a permeability control was used, it was diluted to 500 μM using PBS (prepared by mixing 25 μL of permeability control + 475 μL of PBS (pH 7.2)).
2. For each test compound and control, a 200 μM equilibrium standard was prepared in a separate tube (by mixing 80 μL of 500 μM test compound or control with 120 μL of PBS (pH 7.2)). If the compound can permeate the membrane and reach equilibrium completely, 200 μM would be the final concentration of the solution in both the donor and acceptor wells. Next, a blank control was prepared in a separate tube by mixing 5 μL of DMSO + 245 μL of PBS (pH 7.2). The equilibrium standards and the blank control were set aside for analysis on the following day.
3. 300 μL of PBS (pH 7.2) was added to the wells of the acceptor plate.
4. With the donor plate left in the tray, 5 μL of the BBB lipid solution in dodecane was added directly to the well membrane of the donor plate. During addition, care should be taken not to puncture the membrane with the pipette tip.
5. To replicate the wells of the donor plate, 200 μL each of the 500 μM test compound and the 500 μM permeability control were added.
6. The donor plate was carefully placed onto the wells of the acceptor plate and incubated at RT or 37 °C for 18 hours or a desired incubation time (for example, 16 to 24 hours).
7. The donor plate was carefully removed, a solvent was added, and then the liquid (acceptor solution) was collected in the wells of the acceptor plate for analysis.
8. For each test compound and permeability control, 100 μL of the acceptor solution and 100 μL of the equilibrium standard were added, and 100 μL of the blank control was added to the wells of a UV plate (Cat # P96UV).
9. The absorbance spectrum from 200 nm to 500 nm was measured at 10 nm intervals to determine the peak absorbance of the test compounds. The blank control is used to confirm whether the peak is attributable to the test compound rather than DMSO in the solution. The peak absorbances of the high-permeability and low-permeability controls are 250 nm and 270 nm, respectively.

Data analysis

For the compounds and permeability controls, the permeability rate ($P_e$) was determined using the following equation:

$$P_e = C \times -\ln\left(1 - \frac{OD_A}{OD_E}\right) cm/s$$

Here, $OD_A$ is the absorbance of the acceptor solution minus the blank, and $OD_E$ is the absorbance of the equilibrium standard minus the blank. For each absorbance value, the peak absorbance determined for each test was used. In the permeability rate equation, $C = 7.72 \times 10^{-6}$ when an 18-hour incubation is used.

The PAMPA assay results obtained by the above method are shown in Table 13.

[Table 13]

| Compound | PAMPA $P_e$ ($10^{-9}$ cm/sec) |
|---|---|
| Crystalline form A | 9.254 |

(continued)

| Compound | PAMPA $P_e$ ($10^{-9}$ cm/sec) |
|---|---|
| Crystalline form C | 11.79 |

As shown in Table 13, it was confirmed that crystalline forms A and C of the present disclosure exhibit BBB permeability, and crystalline form C exhibit superior BBB permeability compared to crystalline form A.

**Example 13. Analysis of efficacy in enhancing ex vivo synaptic plasticity in animal model of Alzheimer's disease**

Using APP/PS1 mice (15 months old, Taconic Biosciences), an animal model of Alzheimer's disease, the efficacy of crystalline form C and aducanumab (Creative Biolabs) was evaluated when administered alone or in combination, respectively. A control group and four experimental groups were prepared as follows (see Table 14).

[Table 14]

| Group | Mouse | Administered substance |
|---|---|---|
| Control group | Normal mice | Control antibody + Vehicle |
| Experimental group 1 | APP/PS1 mice (AD mice) | Control antibody + Vehicle |
| Experimental group 2 | APP/PS1 mice (AD mice) | Control antibody + BnH-015B |
| Experimental group 3 | APP/PS1 mice (AD mice) | Aducanumab + Vehicle |
| Experimental group 4 | APP/PS1 mice (AD mice) | Aducanumab + BnH-015B |

For the control antibody, an isotype antibody was administered intravenously, and for the vehicle, 200 μL of a mixed solution of DMSO (10):PEG400 (44):saline (55) was administered orally. The administration schedule is as shown in Fig. 13a. Five mice were used for each treatment group.

For intravenous administration, as shown in Fig. 13a, aducanumab or the control antibody was prepared as a 1 mg/mL stock solution and used in the experiment. Aducanumab was administered at a dose of 5 mg/kg, and the control antibody, which is an inactivated form of aducanumab, was administered at a dose of 5 mg/kg. For the intravenous administration, a total of four injections were administered into the tail vein on the starting day of the experiment and at one-week intervals thereafter.

For oral administration, 1 mg/kg of the compound of the present disclosure (crystalline form C, BnH-015B) or a vehicle was administered orally once daily for about 11 to 13 days, starting about 2 weeks after the initiation of the experiment.

4 weeks after the initiation of the experiment, the mice were sacrificed, and changes in the slope of the field excitatory postsynaptic potential (fEPSP) were observed in the CA1 region of the Schaffer collateral circuit in the hippocampus. As a result, as shown in FIG. 13b, it was confirmed that crystalline form C alone, aducanumab alone, and the combination of crystalline form C and aducanumab all increased synaptic efficacy. In particular, for administration of crystalline form C alone or combination of crystalline form C and aducanumab, synaptic efficacy was significantly increased. In addition, as a result of measuring long-term potentiation (LTP) of the fEPSP in the CA1 region of the Schaffer collateral circuit in the hippocampus, it was also confirmed that synaptic LTP of the excitatory SC pathway was significantly increased. Since increases in the fEPSP slope and LTP indicate enhanced synaptic plasticity, these results demonstrate that administration of crystalline form C alone and in combination with aducanumab increases synaptic potency in the SC pathway of the hippocampus, which indicates that administration of crystalline form C alone and in combination with aducanumab is effective in treating Alzheimer's disease.

Meanwhile, the efficacy of the compound against Alzheimer's disease was evaluated by measuring the potency and the GABA/AMPA ratio in the sensory cerebral cortex. In particular, the effects of crystalline form C and aducanumab were compared when administered alone and in combination. When crystalline form C was administered alone or in combination with aducanumab to APP/PS1 mice, it increased excitatory TC synaptic potency (TC potency) (FIG. 13c, left panel) while maintaining the balance of excitation/inhibition (FIG. 13c, right panel), as confirmed by the measurement of the GABA/AMPA ratio. Since increased TC potency indicates enhanced synaptic plasticity, these results demonstrate that administration of crystalline form C alone or in combination with aducanumab increases TC synaptic potency in the sensory cortex while maintaining the balance of excitation/inhibition, which indicates that administration of crystalline form C alone or in combination with aducanumab is effective in treating Alzheimer's disease. In this case, significantly superior

effects were observed when crystalline form C and aducanumab were administered in combination, compared to when crystalline form C or aducanumab was administered alone.

**Example 14. Analysis of efficacy in enhancing ex vivo synaptic plasticity**

Following oral administration of crystalline form C, the efficacy of the compound was evaluated by measuring the potency and the GABA/AMPA ratio in the barrel cortex.

Crystalline form C was orally administered at a dose of 1 mg/kg to 8-week-old C57BL/6J mice. For the control group, 200 μL of a mixed solution of DMSO (10):PEG400 (44):saline (55) was orally administered as a vehicle. Experiments were performed on five mice each for the control (vehicle) group and the compound-treated group. As a result, it was confirmed that crystalline form C can increase TC synaptic potency in the sensory cortex while maintaining the balance of excitation/inhibition (Figs. 14a and 14b)

**Example 15. Analysis of efficacy in inhibiting amyloid-beta protein accumulation**

To evaluate the efficacy of crystalline form C of the present disclosure in inhibiting amyloid-beta protein accumulation, mouse proteomic profiling array analysis and Bielschowsky silver staining were performed. 15-week-old APP/PS1 mice were used in the experiment, and crystalline form C of the present disclosure (1 mg/kg of BnH-015B) or a vehicle was administered orally.

Osteopontin (OPN) has attracted attention as a nextgeneration therapeutic candidate for Alzheimer's Disease (AD), as it can contribute to IL-33-mediated improvement of AD-related phenotypes. OPN is a cytokine expressed by activated peripheral dendritic cells and a subset of macrophages, and it is known to regulate innate and adaptive immune responses through interactions with integrin receptors (see Patarca et al., 1989; Ashkar et al., 2000; Inoue & Shinohara, 2015; Danzaki et al., 2016; Aggarwal et al., 2021). Furthermore, it is known that the level of CD11c+ microglia producing OPN strongly correlates with the degree of cognitive deficiency and AD neuropathology (see Qiu et al., 2023); and it has been reported that OPN gene knockout or administration of a monoclonal anti-OPN antibody improves cognitive function in 5XFAD mice by reducing proinflammatory microglia, plaque formation, and the number of atrophic neurites (see Qiu et al., 2023).

Amyloid-beta (Aβ) is one of the danger-related molecular patterns for microglia. It is known that microglia can be activated by Aβ and promote inflammatory responses by secreting various cytokines such as IL-1β, IL-6, and TNF-α (Clark & Vissel, 2015).

Accordingly, it was experimentally confirmed that crystalline form C regulates neuroinflammation by modulating OPN-expressing microglia in AD mice.

<u>Mouse proteome profiling array analysis</u>

Relative levels of mouse cytokines, chemokines, and growth factors were determined using a membrane-based antibody array kit (Proteome Profiler Mouse XL Cytokine Array Kit, ARY028 R&D Systems, Inc., Minneapolis, USA) which features 111 capture antibodies coated in duplicate on a nitrocellulose membrane. Homogenates of the somatosensory cortex of APP/PS1 mice were prepared and tested according to the manufacturer's instructions. The array was detected using an X-ray film (Super RX, Fujifilm, Tokyo, Japan), and the film was read using an Epson scanner (L655 series, Epson, Suwa, Japan), followed by image analysis using ImageJ with the Protein Array Analyzer Macro (National Institutes of Health, Bethesda, MD, USA).

As a result of confirming whether crystalline form C can alter the expression of IL-33 and OPN in the cerebral cortex of 15-month-old APP/PS1 mice, 14 distinct cytokine spots that were positively stained were clearly identified. After normalization of the staining results, the relative expression levels of the 14 cytokines in the group administered crystalline form C for 14 days in 15-month-old APP/PS1 mice were compared with those in the vehicle-treated control group.

The expression level of IL-33 was significantly increased in mice administered crystalline form C compared to the vehicle-treated control group, whereas OPN expression was suppressed in mice administered crystalline form C compared to the vehicle-treated control group (Fig. 15).

<u>Bielschowski silver staining</u>

Bielschowski silver staining (Bielschowski Silver Staining Kit, ab245877, abcam, Cambridge, UK) was performed according to the manufacturer's protocol. 40 μm-thick brain sections of APP/PS1 mice were incubated in a warm 20% silver nitrate solution at 40°C for 15 minutes, followed by four washes with distilled water and three rinses with water for 3 minutes each. The slides were incubated in an ammoniacal silver solution at 40°C for 10 minutes, and then placed in a developer solution (containing 8 drops of 20% formalin, 8 drops of citric acid, and 4 drops of nitric acid in 50 mL $H_2O$) for a

few seconds until the tissue sections turned yellow/brown. Subsequently, the slides were placed in aqueous ammonia for 30 seconds. The sections were washed three times with water for 2 minutes each and incubated in a 5% thiosulfate solution for 2 minutes. The slides were then dehydrated and coverslipped using Permount mounting medium (Thermo Fisher Scientific), and images were obtained using an Olympus BX 53 light microscope with cellSens software in bright-field mode at 10x magnification.

Aβ plaques in the brain were visualized using the Bielschowsky silver staining method for Aβ monomers (Fig. 16a). Crystalline form C reduced Aβ plaques by enhancing phagocytic activity of microglia in the cerebral cortex of 15-month-old APP/PS1 mice (Fig. 16b).

Data statistical analysis

Statistical analysis was performed using GraphPad Prism 9.5.0 software (San Diego, CA, USA). A one-tailed unpaired t-test with Welch's correction was used to determine statistical significance between two experimental groups. Mean differences between groups were considered significant at $p<0.05$ (*) and $p<0.01$ (**). All data are expressed as mean ± SEM.

## Claims

1. A crystalline form of a compound represented by Formula (1):

Formula (1)

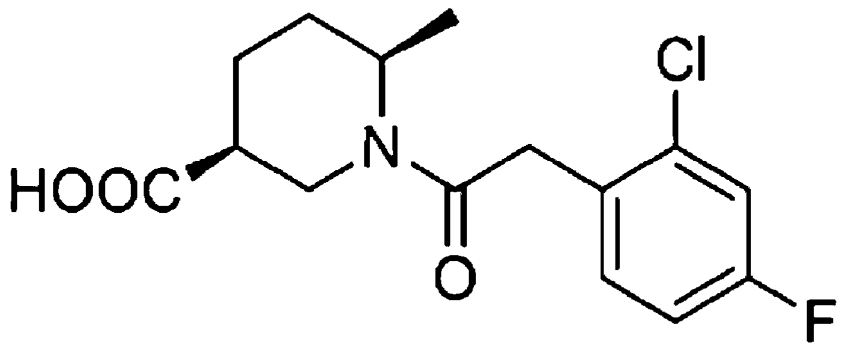

.

2. The crystalline form A of the compound of claim 1, **characterized by** an X-ray powder diffraction pattern having diffraction peaks at diffraction angles 2θ(°) of 9.9±0.10, 14.9±0.10, 18.4±0.10, 24.5±0.10, 24.8±0.10, 25.2±0.10, and 28.7±0.10.

3. The crystalline form A of the compound of claim 2, wherein the X-ray powder diffraction pattern of the crystalline form further comprises diffraction peaks at diffraction angles 2θ(°) of 5.0±0.10, 13.4±0.10, 19.7±0.10, 19.8±0.10, 20.5 ±0.10, 22.5±0.10, 24.0±0.10, and 35.0±0.10.

4. The crystalline form A of the compound of claim 3, wherein the X-ray powder diffraction pattern of the crystalline form further comprises diffraction peaks at diffraction angles 2θ(°) of 12.1±0.10, 16.2±0.10, 20.1±0.10, 21.5±0.10, 27.4 ±0.10, 29.6±0.10, 30.0±0.10, 31.2±0.10, 31.5±0.10, and 36.8±0.10.

5. The crystalline form B of the compound of claim 1, **characterized by** an X-ray powder diffraction pattern having diffraction peaks at diffraction angles 2θ(°) of 8.6±0.10, 9.1±0.10 and/or 9.2±0.10, 15.8±0.10, 20.0±0.10, 20.3 ±0.10, 20.7±0.10, 23.9±0.10, and 32.6±0.10.

6. The crystalline form B of the compound of claim 5, wherein the X-ray powder diffraction pattern of the crystalline form further comprises diffraction peaks at diffraction angles 2θ(°) of 8.2±0.10, 14.2±0.10, 14.7±0.10, 18.0±0.10, 18.3 ±0.10, 22.9±0.10, 26.4±0.10, 26.7±0.10, 27.6±0.10, 28.1±0.10, and 29.1±0.10.

7. The crystalline form B of the compound of claim 6, wherein the X-ray powder diffraction pattern of the crystalline form

further comprises diffraction peaks at diffraction angles 2θ(°) of 11.9±0.10, 15.1±0.10, 16.5±0.10, 19.3±0.10, 22.1 ±0.10, 22.6±0.10, 24.5±0.10, 24.8±0.10, 25.9±0.10, 27.0±0.10, 27.8±0.10, 28.9±0.10, and 36.5±0.10.

8. The crystalline form C of the compound of claim 1, **characterized by** an X-ray powder diffraction pattern having diffraction peaks at diffraction angles 2θ(°) of 8.6±0.10, 10.0±0.10, 18.0±0.10, and 26.4±0.10.

9. The crystalline form C of the compound of claim 8, wherein the X-ray powder diffraction pattern of the crystalline form further comprises diffraction peaks at diffraction angles 2θ(°) of 17.2±0.10, 20.2±0.10, and 25.5±0.10.

10. The crystalline form C of the compound of claim 9, wherein the X-ray powder diffraction pattern of the crystalline form further comprises diffraction peaks at diffraction angles 2θ(°) of 15.1±0.10, 16.0±0.10, 21.1±0.10, 23.4±0.10, 26.1 ±0.10, and 34.2±0.10.

11. The crystalline form D of the compound of claim 1, **characterized by** an X-ray powder diffraction pattern having diffraction peaks at diffraction angles 2θ(°) of 8.8±0.10, 10.7±0.10, 17.1±0.10, 17.6±0.10, 19.1±0.10, 21.5±0.10, 22.2±0.10, and 23.4±0.10.

12. The crystalline form D of the compound of claim 11, wherein the X-ray powder diffraction pattern of the crystalline form further comprises diffraction peaks at diffraction angles 2θ(°) of 12.5±0.10, 15.4±0.10, 16.1±0.10, 16.7±0.10, 18.3 ±0.10, 22.5±0.10, 22.9±0.10, 27.4±0.10, 27.8±0.10, and 29.8±0.10.

13. The crystalline form D of the compound of claim 12, wherein the X-ray powder diffraction pattern of the crystalline form further comprises diffraction peaks at diffraction angles 2θ(°) of 9.5±0.10, 15.7±0.10, 21.0±0.10, 25.1±0.10, 25.3 ±0.10, 27.0±0.10, 28.6±0.10, 30.9±0.10, 32.1±0.10, 32.6±0.10, and 34.1±0.10.

14. The crystalline form E of the compound of claim 1, **characterized by** an X-ray powder diffraction pattern having diffraction peaks at diffraction angles 2θ(°) of 10.2±0.10, 11.3±0.10, 18.3±0.10, 20.5±0.10, 22.8±0.10, 23.5±0.10, 25.2±0.10, 26.1±0.10, 28.6±0.10, 28.9±0.10, and 31.2±0.10.

15. The crystalline form E of the compound of claim 14, wherein the X-ray powder diffraction pattern of the crystalline form further comprises diffraction peaks at diffraction angles 2θ(°) of 9.9±0.10, 13.4±0.10, 17.9±0.10, 18.6±0.10, 19.6 ±0.10, 20.8±0.10, 21.0±0.10, 24.4±0.10, 24.8±0.10, 29.5±0.10, 29.8±0.10, and 32.5±0.10.

16. The crystalline form E of the compound of claim 15, wherein the X-ray powder diffraction pattern of the crystalline form further comprises diffraction peaks at diffraction angles 2θ(°) of 4.9±0.10, 7.2±0.10, 14.8±0.10, 16.1±0.10, 16.4 ±0.10, 19.8±0.10, 20.0±0.10, 21.5±0.10, 22.5±0.10, 24.0±0.10, 25.4±0.10, 27.4±0.10, 32.8±0.10, and 39.5 ±0.10.

17. The crystalline form of the compound of any one of claims 1 to 16, wherein the crystalline form exhibits improved BBB permeability as compared with an amorphous form of the compound.

18. A pharmaceutical composition comprising, as an active ingredient, the crystalline form of the compound of any one of claims 1 to 16.

19. The pharmaceutical composition of claim 18, wherein the composition is for the prevention or treatment of a neurological disorder or a neurodegenerative disorder.

20. The pharmaceutical composition of claim 19, wherein the disorder is Alzheimer's disease.

21. The pharmaceutical composition of claim 19, wherein the disorder is PTSD.

22. The pharmaceutical composition of claim 18, further comprising a second therapeutic agent.

23. The pharmaceutical composition of claim 22, wherein the second therapeutic agent is selected from the group consisting of donepezil, rivastigmine, galantamine, memantine, verubecestat, solanezumab, bapineuzumab, aducanumab, lecanemab, tideglusib, epothilone D, and ABBV-8E12.

24. The pharmaceutical composition of claim 22, wherein the second therapeutic agent is an antibody that specifically

binds to amyloid-beta protein and inhibits or degrades accumulation thereof.

**25.** The pharmaceutical composition of claim 22, wherein the second therapeutic agent is aducanumab.

**26.** A method for preventing or treating a neurological disorder or a neurodegenerative disorder, comprising administering to a subject in need thereof the crystalline form of the compound of any one of claims 1 to 16.

FIG. 1a

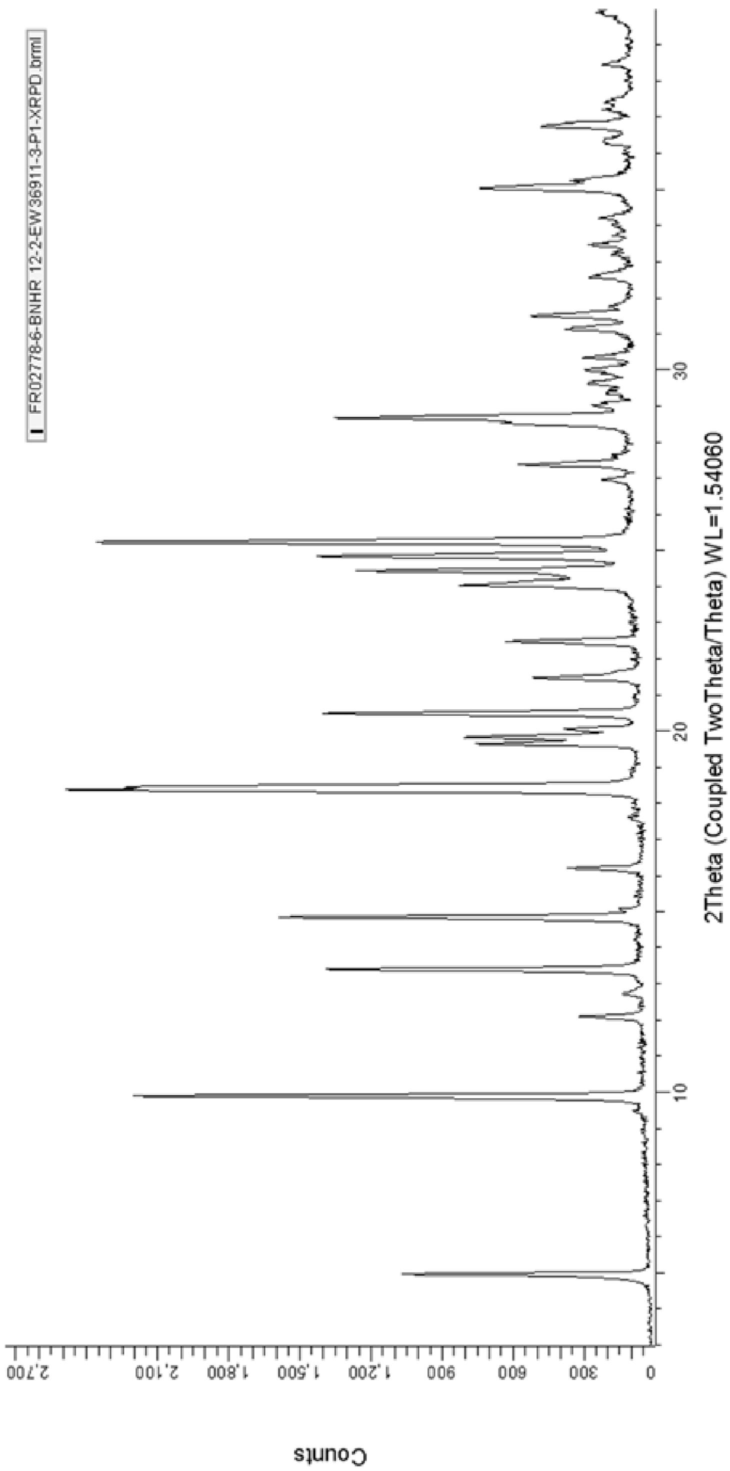
FR02778-6-BNHR 12-2-EW36911-3-P1-XRPD.brml
2Theta (Coupled TwoTheta/Theta) WL=1.54060
Counts
0
300
600
900
1.200
1.500
1.800
2.100
2.700
10
20
30

FIG. 1b

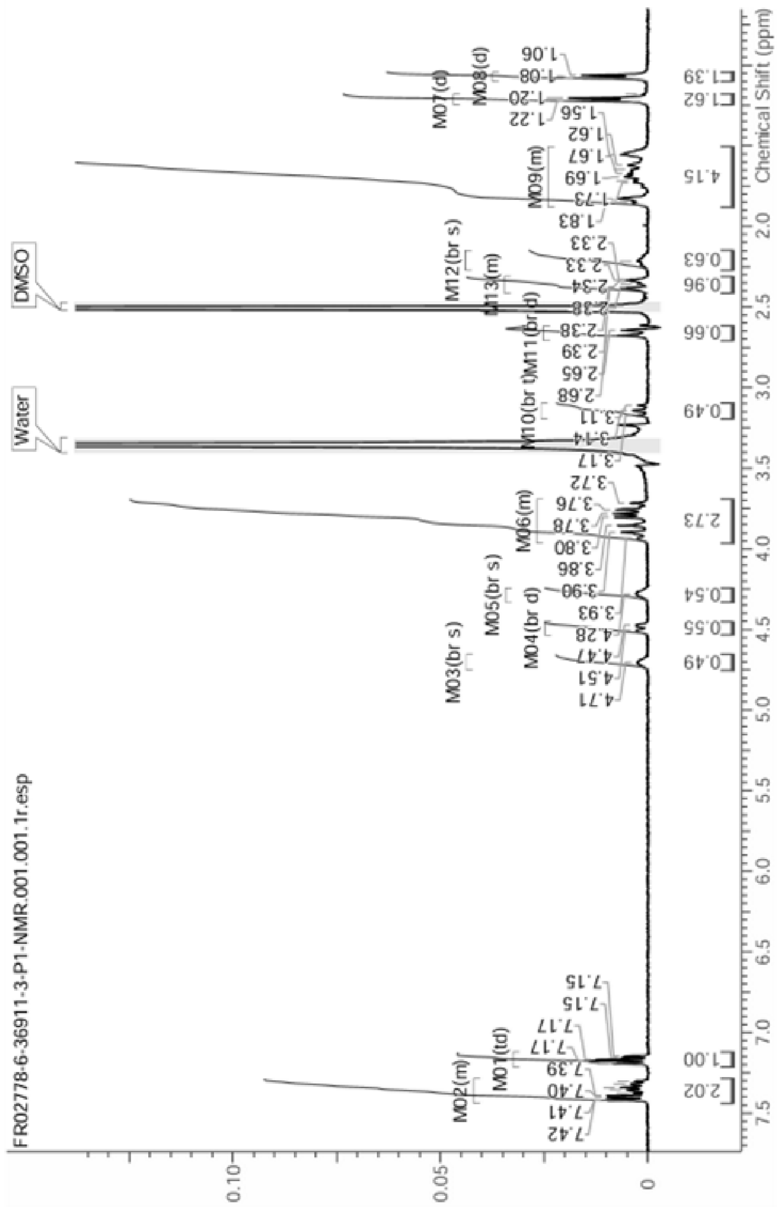
FR02778-6-36911-3-P1-NMR.001.001.1r.esp
DMSO
Water
Chemical Shift (ppm)

FIG. 1c

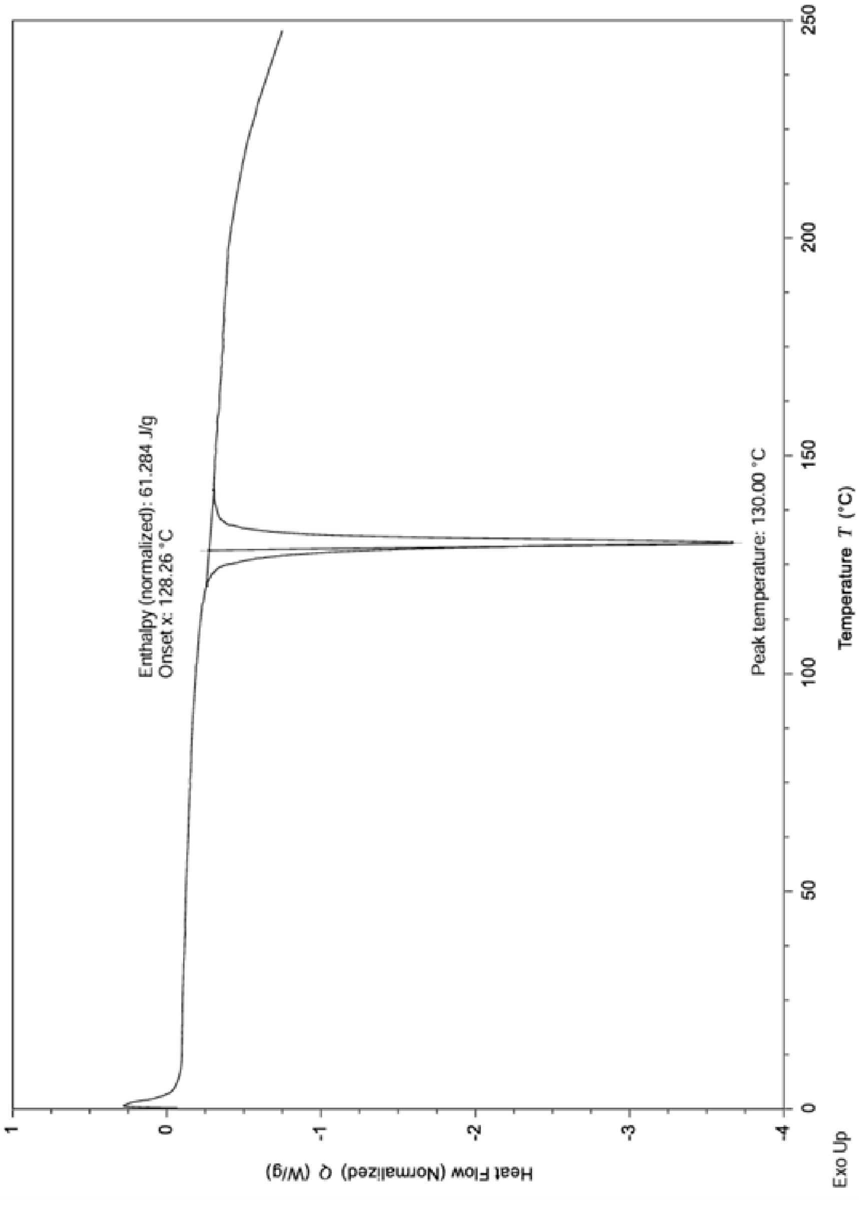
Enthalpy (normalized): 61.284 J/g
Onset x: 128.26 °C
Peak temperature: 130.00 °C
Temperature T (°C)
Heat Flow (Normalized) Q (W/g)
Exo Up
0
50
100
150
200
250
1
0
-1
-2
-3
-4

FIG. 1d

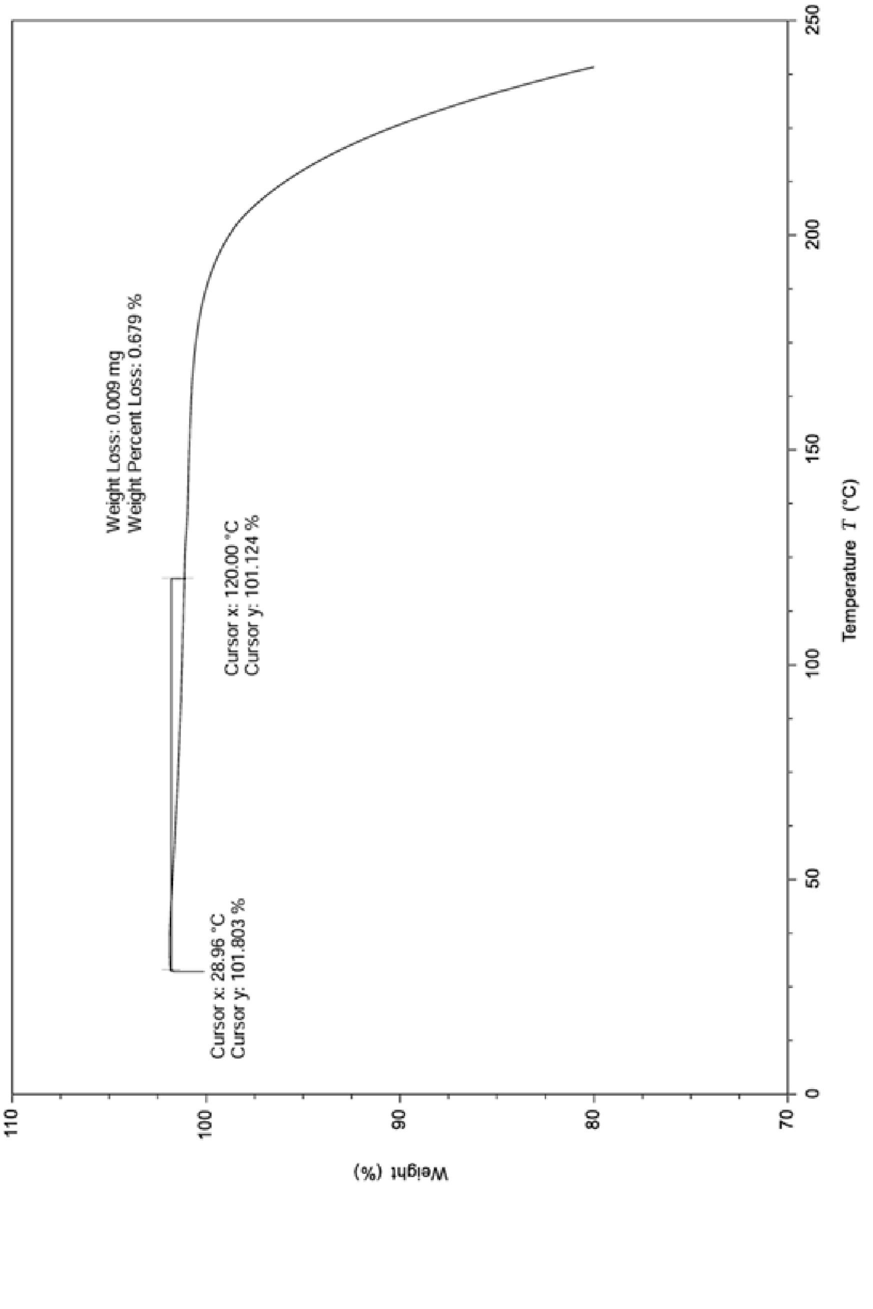
Weight Loss: 0.009 mg
Weight Percent Loss: 0.679 %
Cursor x: 120.00 °C
Cursor y: 101.124 %
Cursor x: 28.96 °C
Cursor y: 101.803 %
0
50
100
150
200
250
Temperature T (°C)
110
100
90
80
70
Weight (%)

FIG. 2a

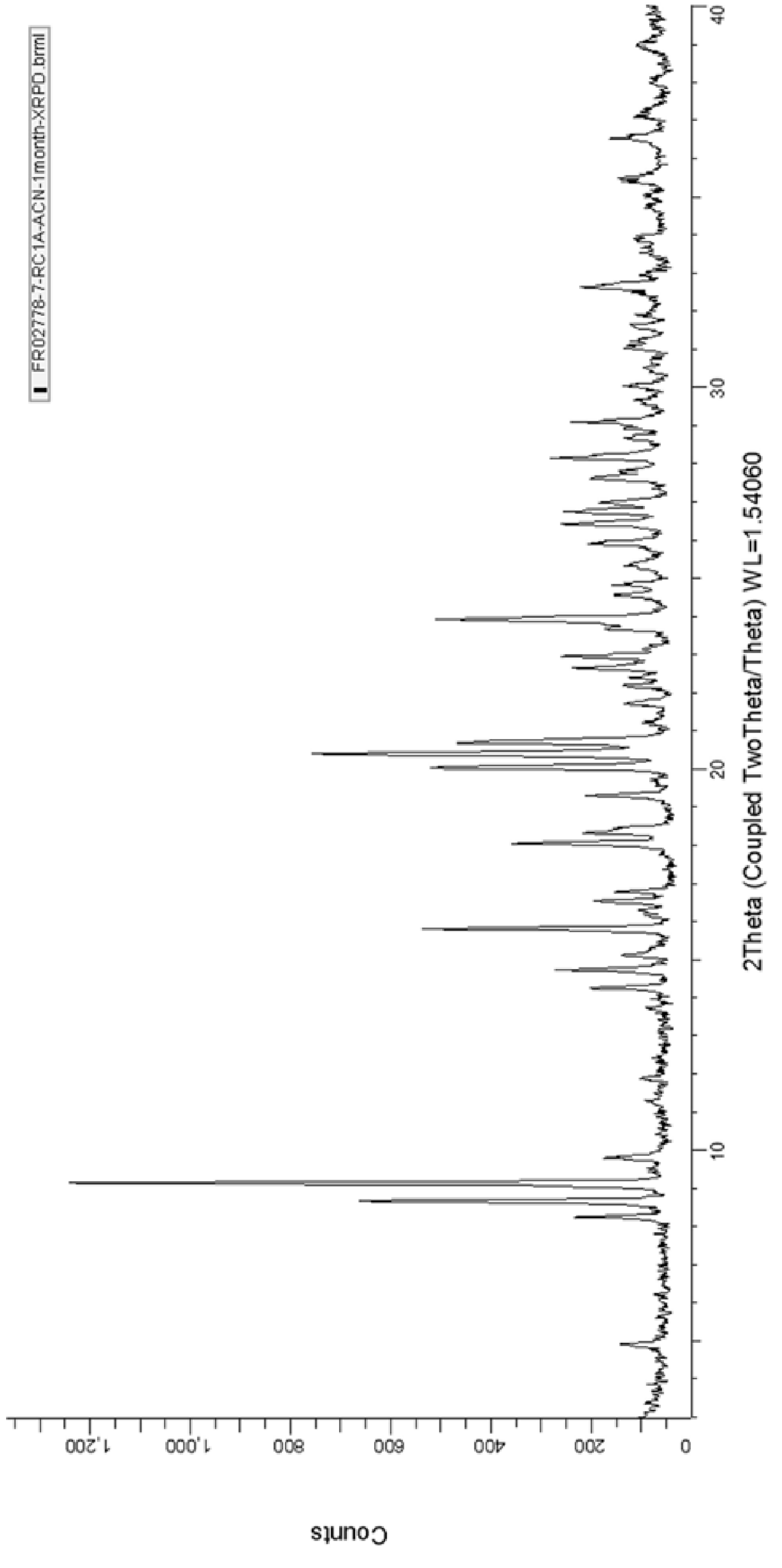
FR02778-7-RC1A-ACN-1month-XRPD.brml
Counts
0
200
400
600
800
1,000
1,200
10
20
30
40
2Theta (Coupled TwoTheta/Theta) WL=1.54060

FIG. 2b

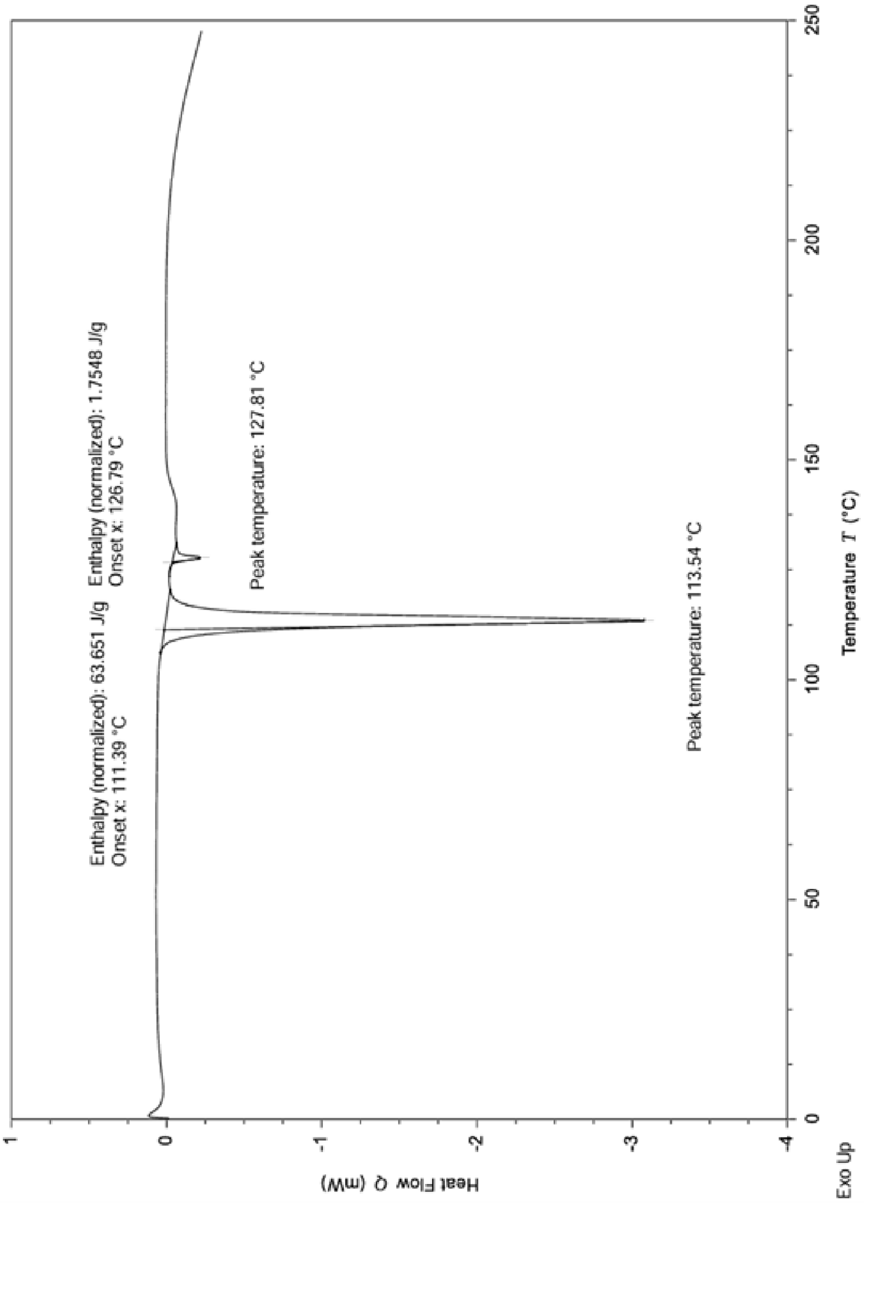
Enthalpy (normalized): 63.651 J/g
Onset x: 111.39 °C
Enthalpy (normalized): 1.7548 J/g
Onset x: 126.79 °C
Peak temperature: 127.81 °C
Peak temperature: 113.54 °C
Heat Flow Q (mW)
Temperature T (°C)
Exo Up
1
0
-1
-2
-3
-4
0
50
100
150
200
250

FIG. 2c

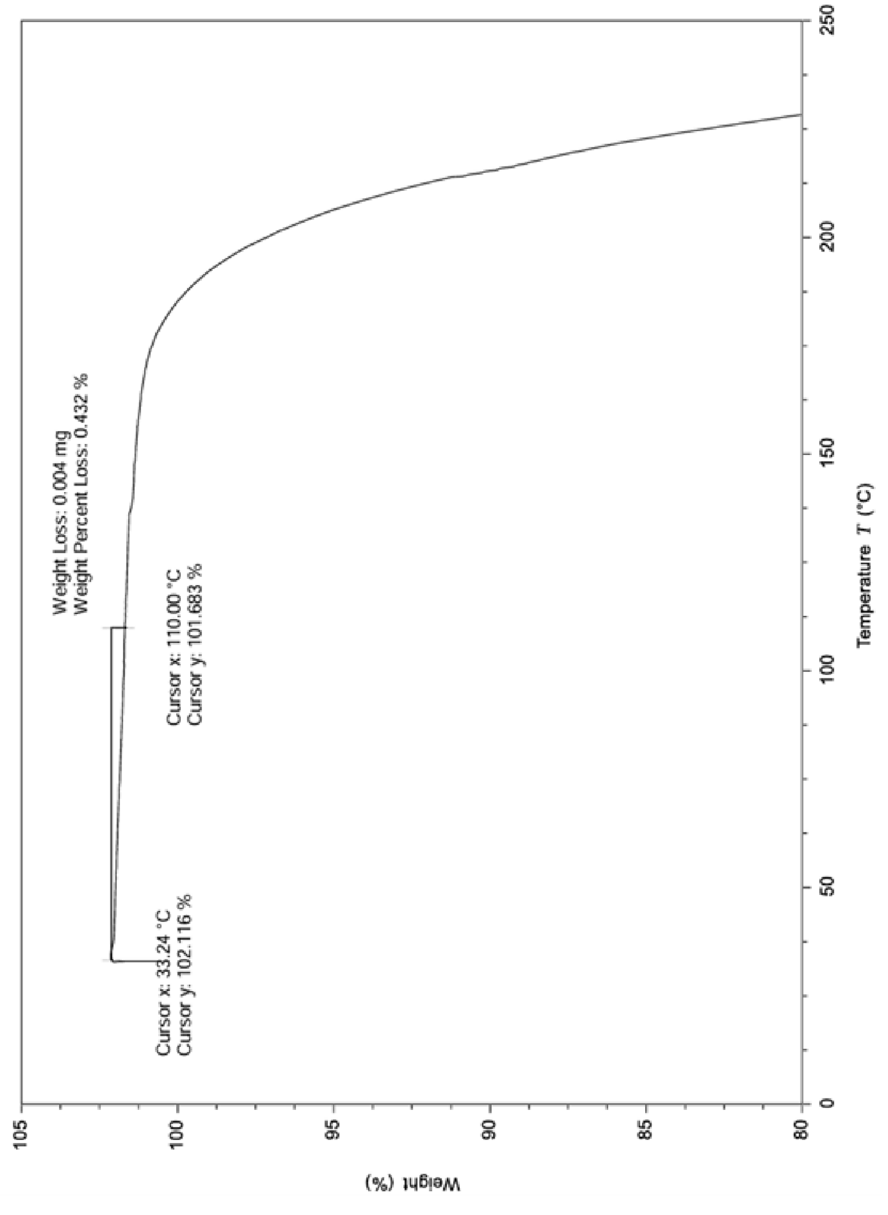
Weight Loss: 0.004 mg
Weight Percent Loss: 0.432 %
Cursor x: 110.00 °C
Cursor y: 101.683 %
Cursor x: 33.24 °C
Cursor y: 102.116 %
Temperature T (°C)
Weight (%)
0
50
100
150
200
250
105
100
95
90
85
80

FIG. 3a

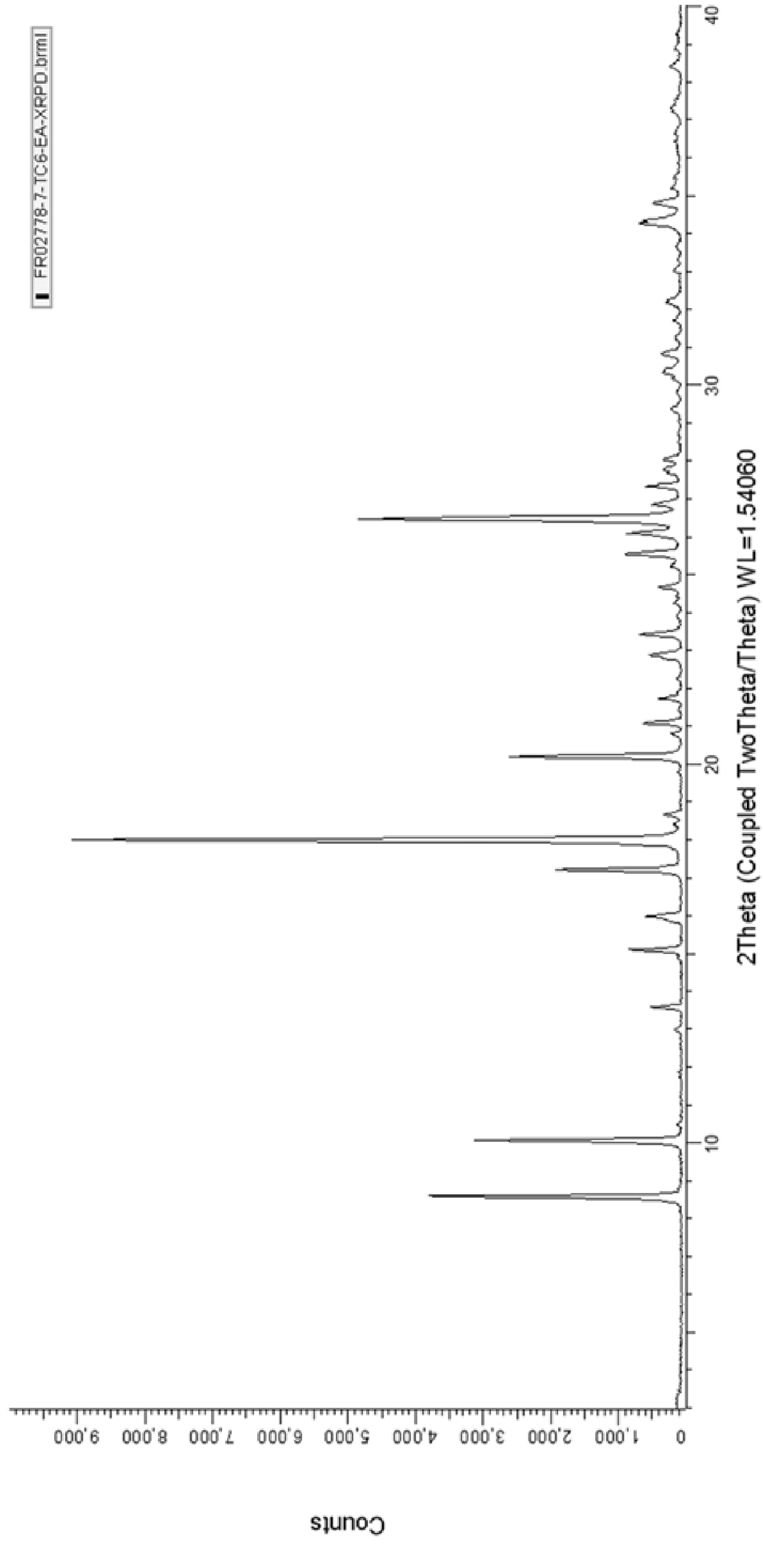
FR02778-7-TC6-EA-XRPD.brml
Counts
0
1,000
2,000
3,000
4,000
5,000
6,000
7,000
8,000
9,000
10
20
30
40
2Theta (Coupled TwoTheta/Theta) WL=1.54060

FIG. 3b

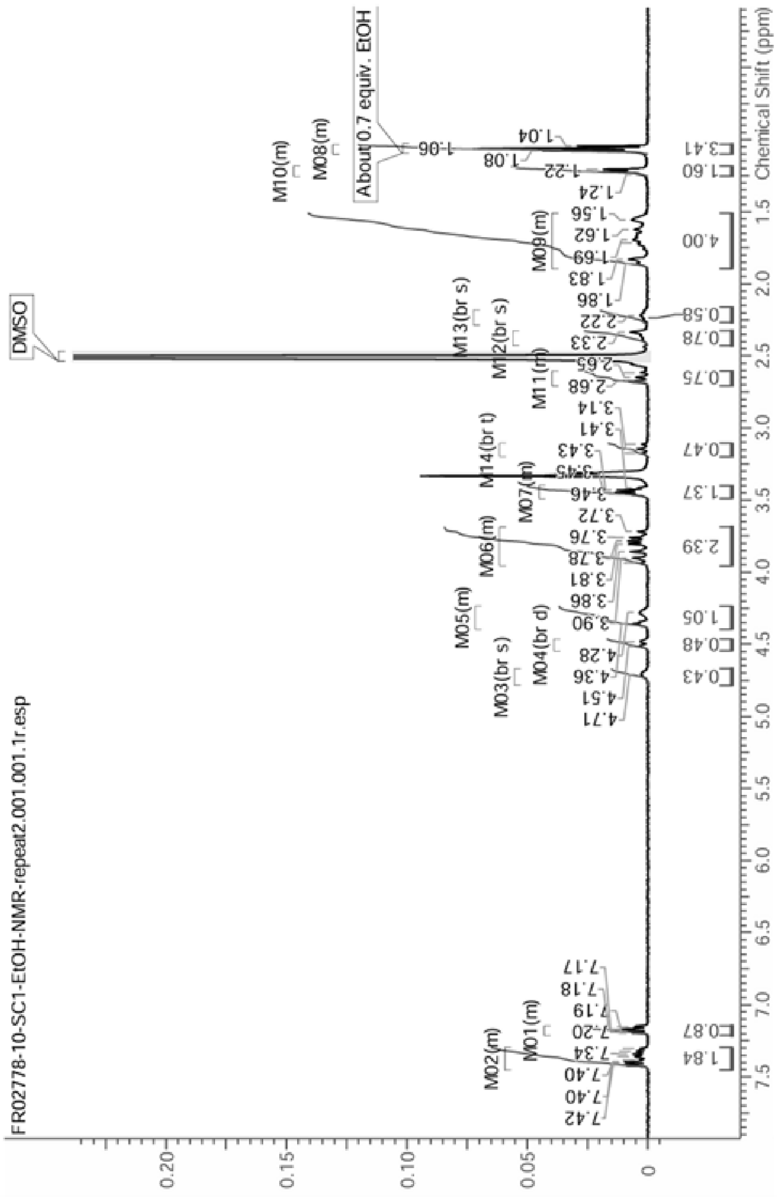
FR02778-10-SC1-EtOH-NMR-repeat2.001.001.1r.esp
DMSO
About 0.7 equiv. EtOH
Chemical Shift (ppm)

FIG. 3c

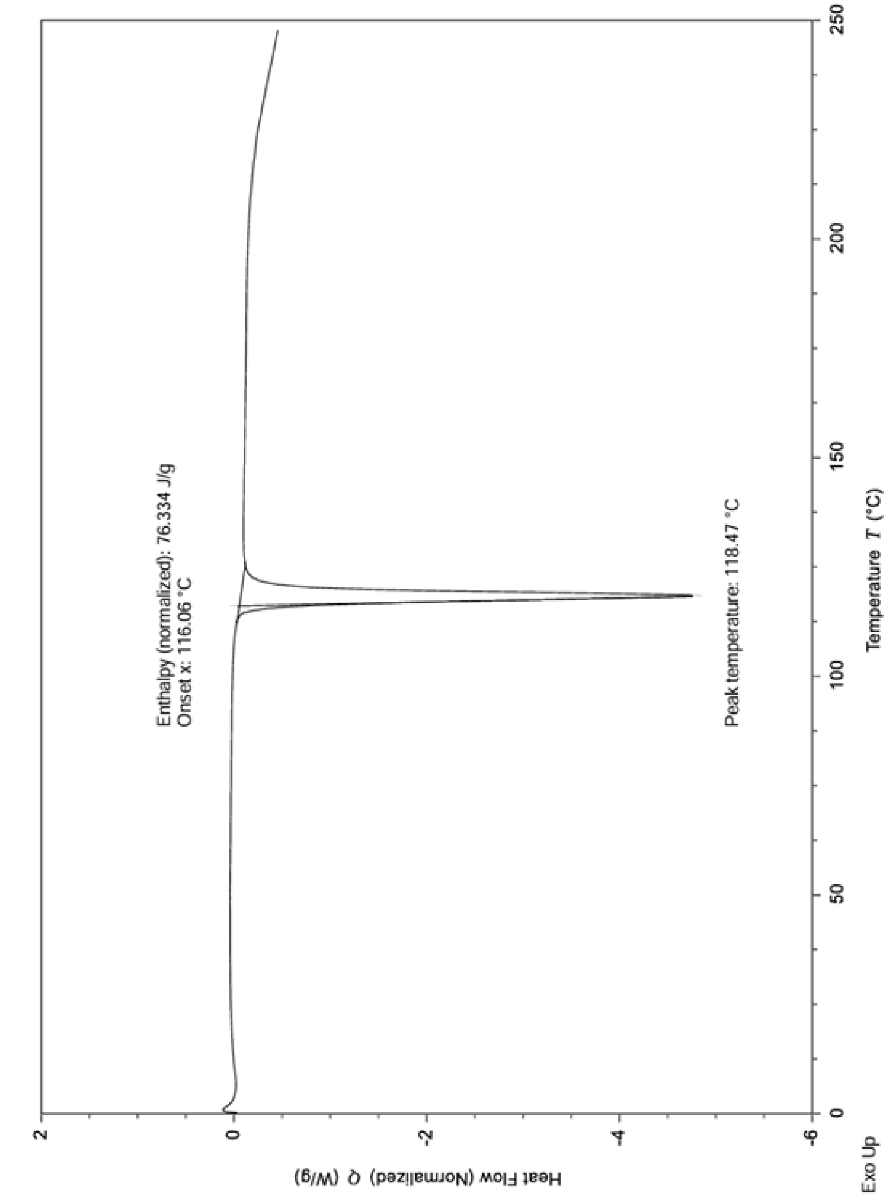
Enthalpy (normalized): 76.334 J/g
Onset x: 116.06 °C
Peak temperature: 118.47 °C
Temperature T (°C)
Heat Flow (Normalized) Q (W/g)
Exo Up
0
50
100
150
200
250
2
0
-2
-4
-6

FIG. 3d

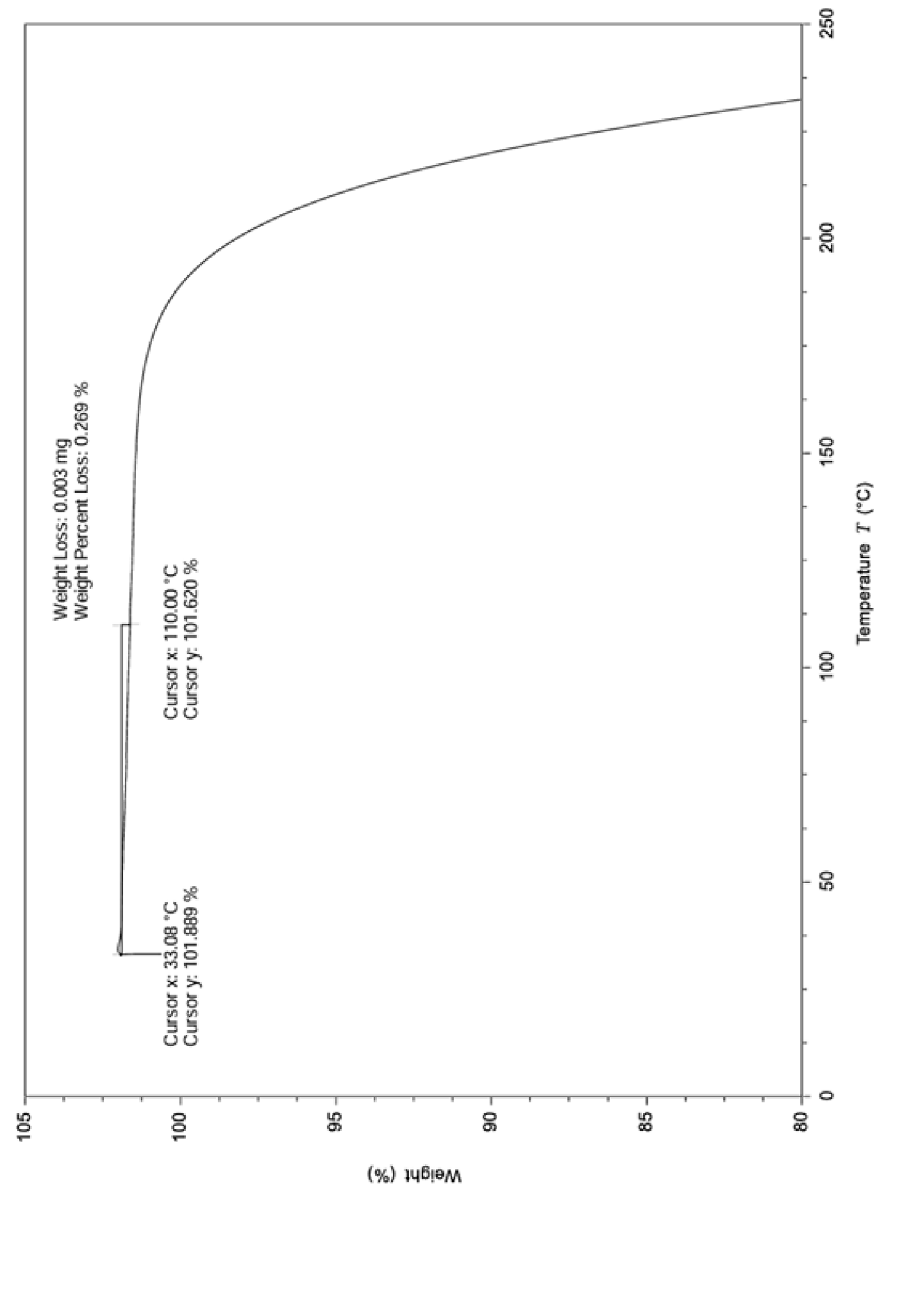
Weight Loss: 0.003 mg
Weight Percent Loss: 0.269 %
Cursor x: 110.00 °C
Cursor y: 101.620 %
Cursor x: 33.08 °C
Cursor y: 101.889 %
Temperature T (°C)
Weight (%)
0
50
100
150
200
250
105
100
95
90
85
80

FIG. 4a

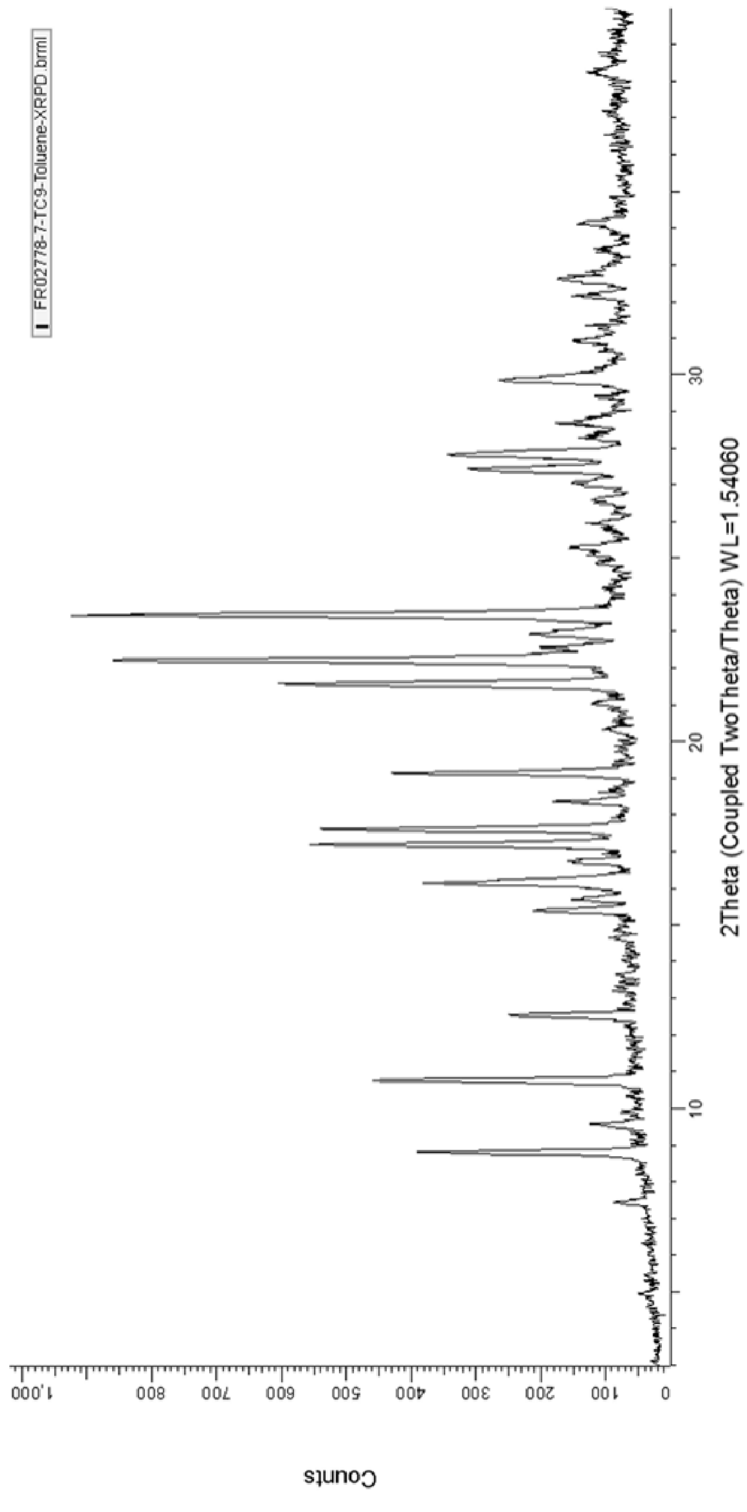
FR02778-7-TC9-Toluene-XRPD.brml
2Theta (Coupled TwoTheta/Theta) WL=1.54060
Counts
10
20
30
0
100
200
300
400
500
600
700
800
1,000

FIG. 4b

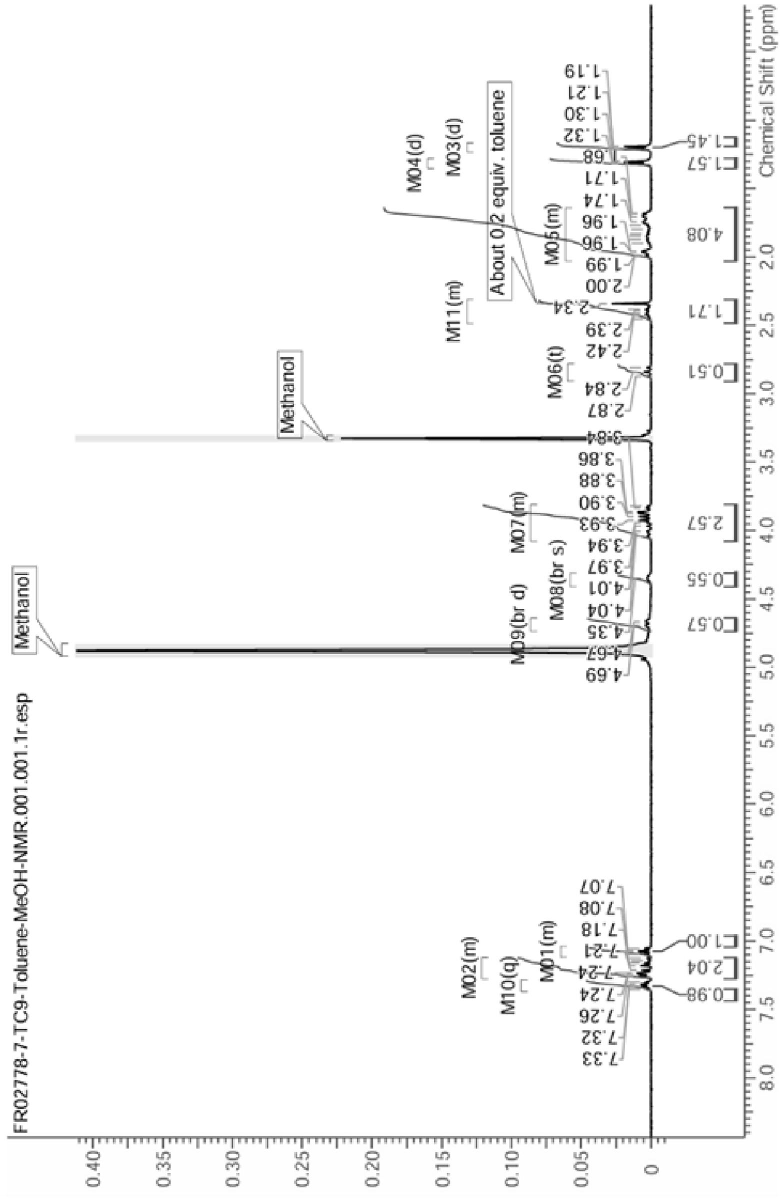
FR02778-7-TC9-Toluene-MeOH-NMR.001.001.1r.esp
Methanol
Methanol
About 0.2 equiv. toluene
M01(m)
M02(m)
M03(d)
M04(d)
M05(m)
M06(t)
M07(m)
M08(br s)
M09(br d)
M10(q)
M11(m)
Chemical Shift (ppm)

FIG. 4c

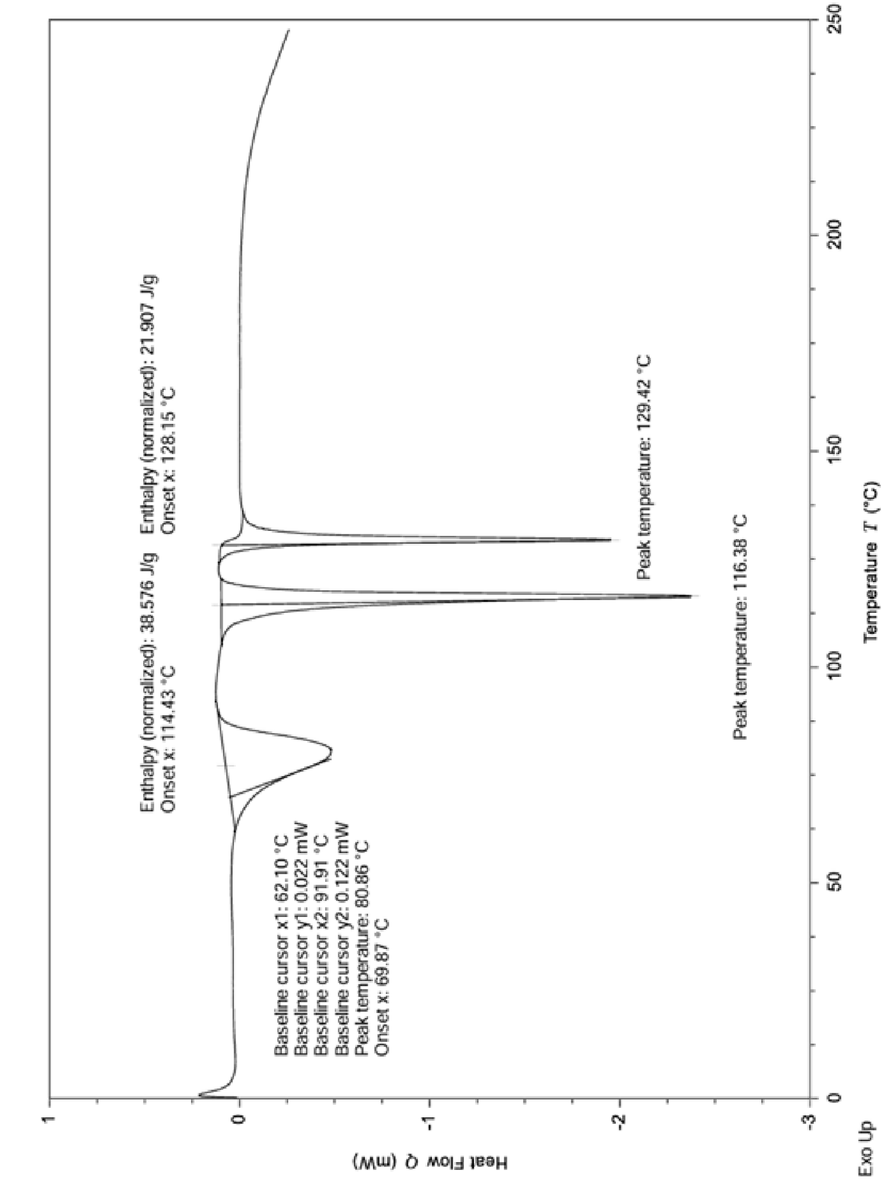
Enthalpy (normalized): 21.907 J/g
Onset x: 128.15 °C
Enthalpy (normalized): 38.576 J/g
Onset x: 114.43 °C
Peak temperature: 129.42 °C
Peak temperature: 116.38 °C
Baseline cursor x1: 62.10 °C
Baseline cursor y1: 0.022 mW
Baseline cursor x2: 91.91 °C
Baseline cursor y2: 0.122 mW
Peak temperature: 80.86 °C
Onset x: 69.87 °C
0
50
100
150
200
250
Temperature T (°C)
1
0
-1
-2
-3
Heat Flow Q (mW)
Exo Up

FIG. 4d

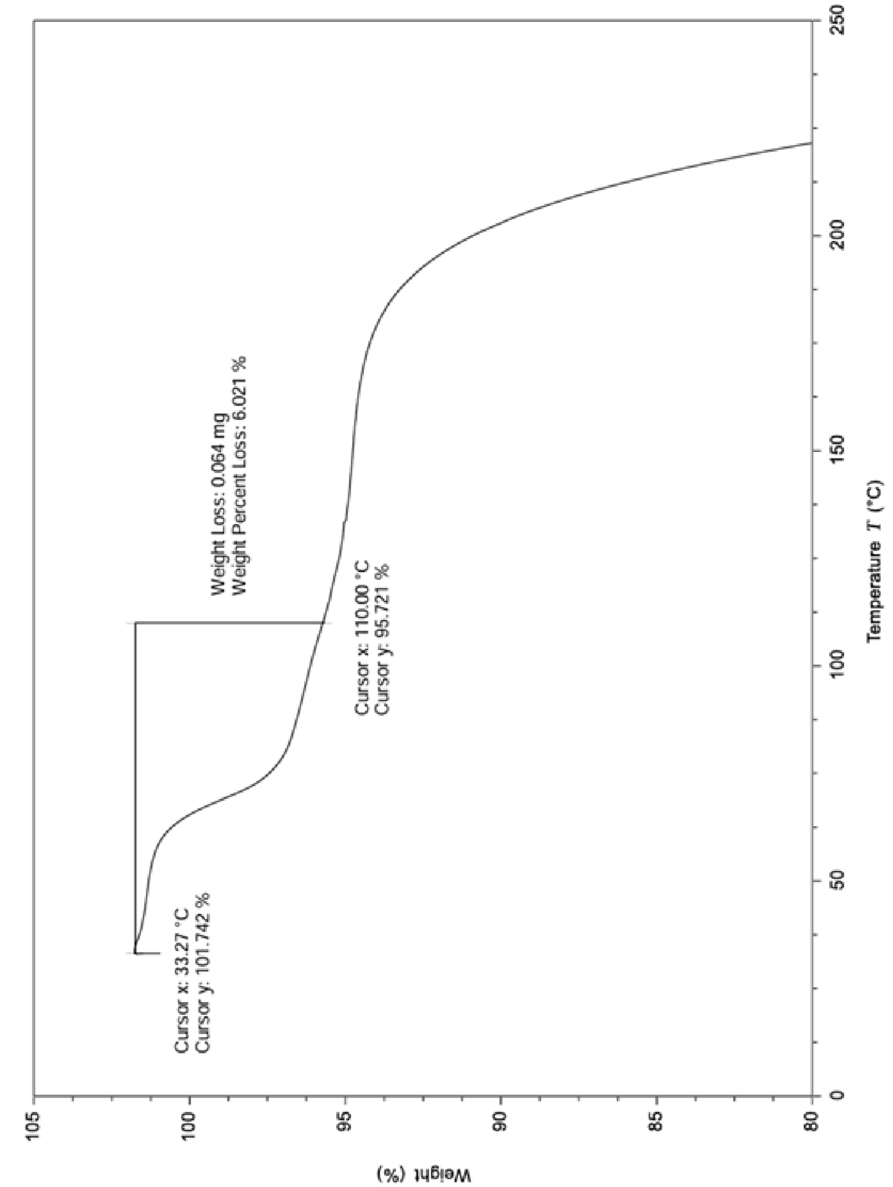
Weight Loss: 0.064 mg
Weight Percent Loss: 6.021 %
Cursor x: 110.00 °C
Cursor y: 95.721 %
Cursor x: 33.27 °C
Cursor y: 101.742 %
Temperature T (°C)
Weight (%)
0
50
100
150
200
250
105
100
95
90
85
80

FIG. 5a

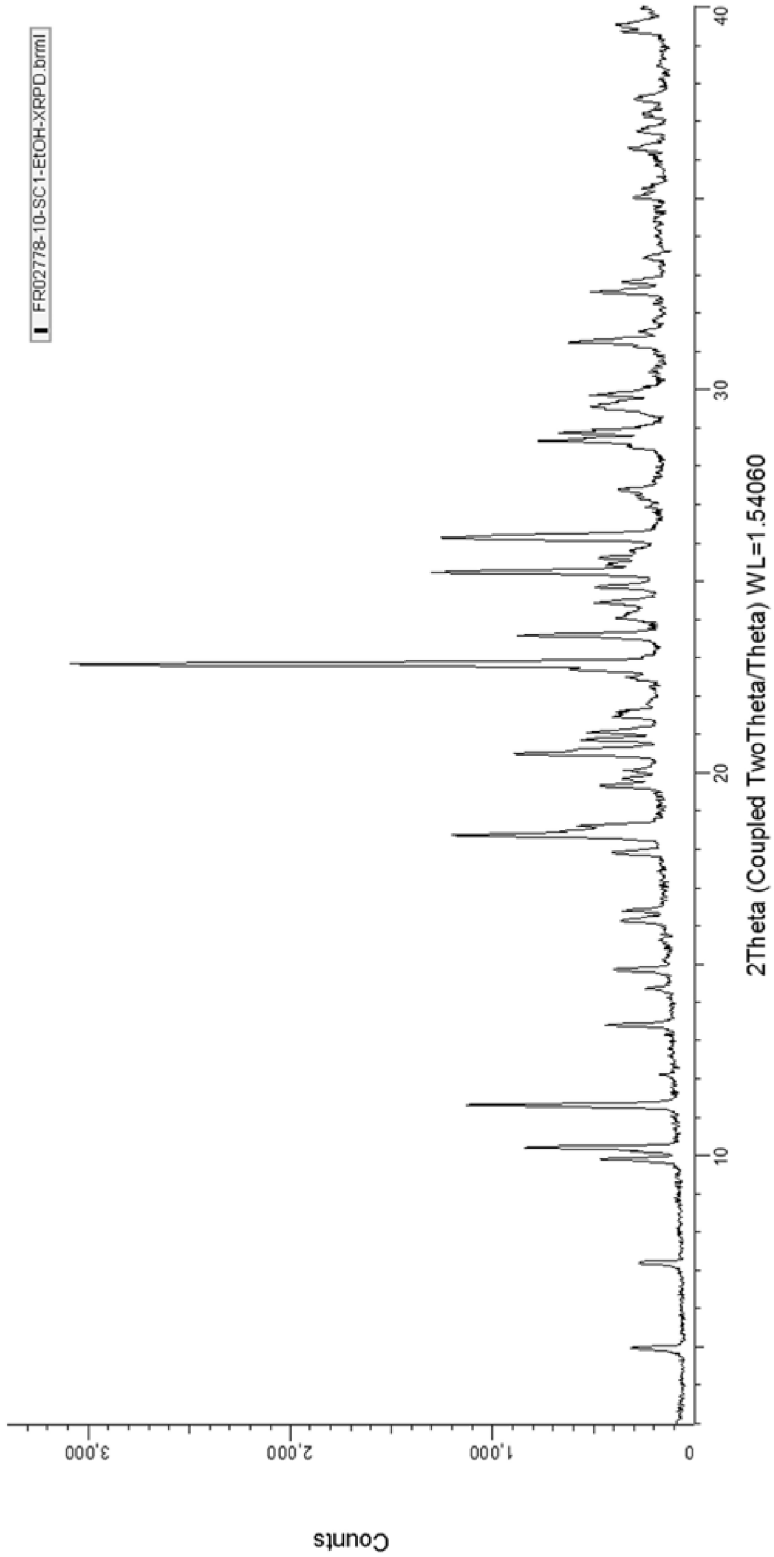
FR02778-10-SC1-EtOH-XRPD.brml
2Theta (Coupled TwoTheta/Theta) WL=1.54060
Counts
0
1,000
2,000
3,000
10
20
30
40

FIG. 5b

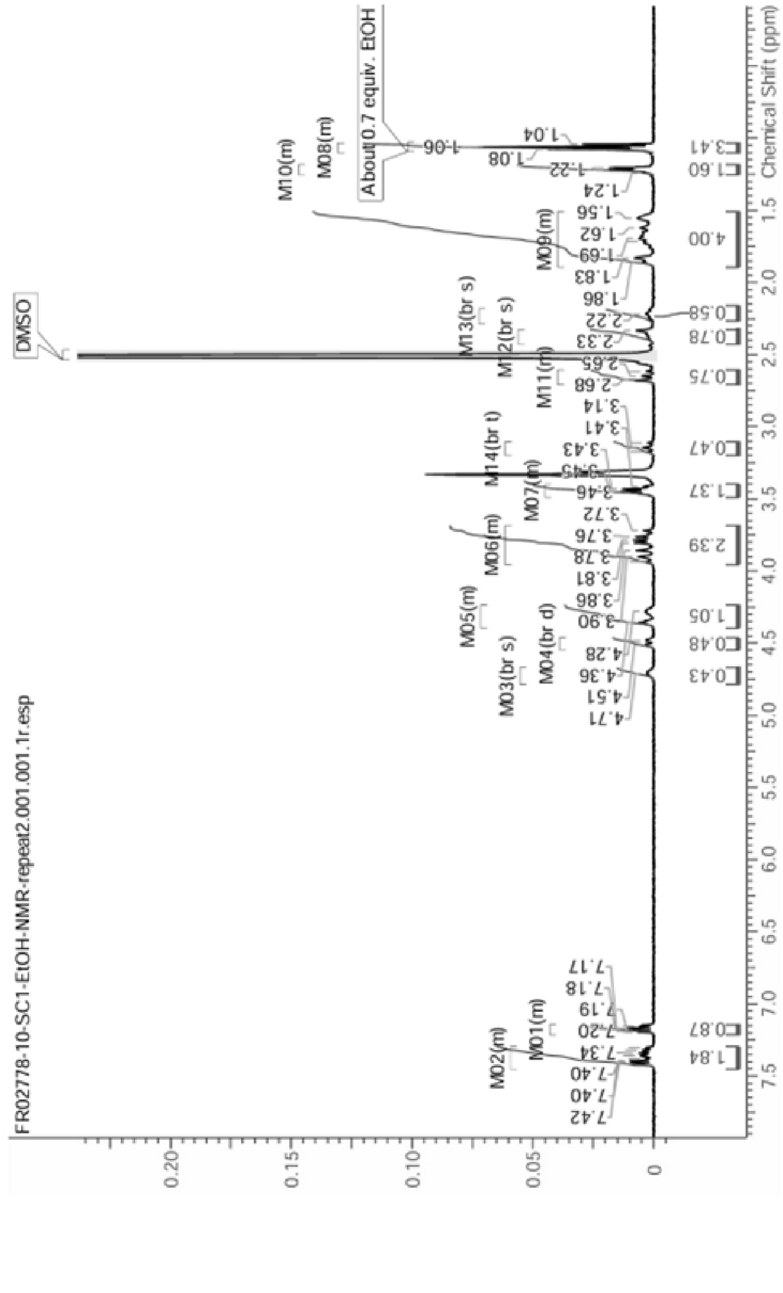
FR02778-10-SC1-EtOH-NMR-repeat2.001.001.1r.esp
DMSO
About 0.7 equiv. EtOH
Chemical Shift (ppm)

FIG. 5c

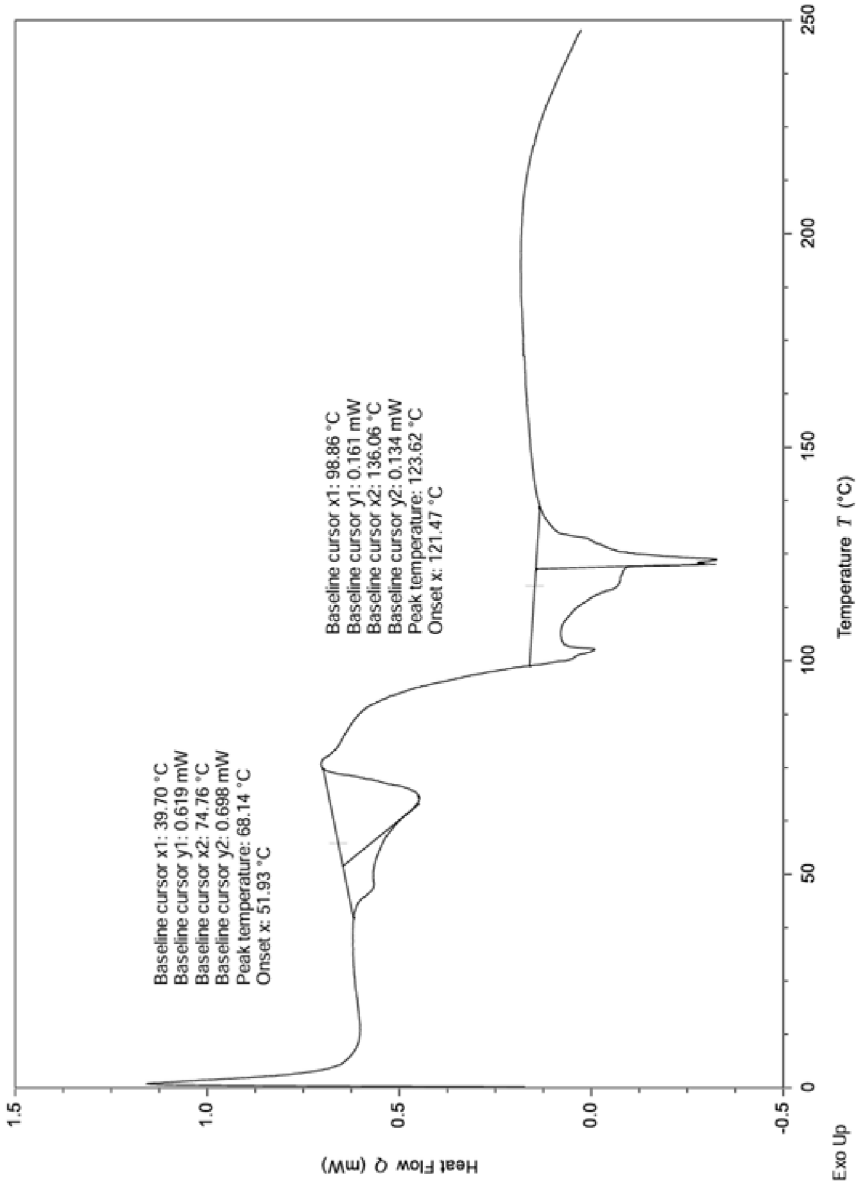
Baseline cursor x1: 39.70 °C
Baseline cursor y1: 0.619 mW
Baseline cursor x2: 74.76 °C
Baseline cursor y2: 0.698 mW
Peak temperature: 68.14 °C
Onset x: 51.93 °C
Baseline cursor x1: 98.86 °C
Baseline cursor y1: 0.161 mW
Baseline cursor x2: 136.06 °C
Baseline cursor y2: 0.134 mW
Peak temperature: 123.62 °C
Onset x: 121.47 °C
Heat Flow Q (mW)
Temperature T (°C)
Exo Up
1.5
1.0
0.5
0.0
-0.5
0
50
100
150
200
250

FIG. 5d

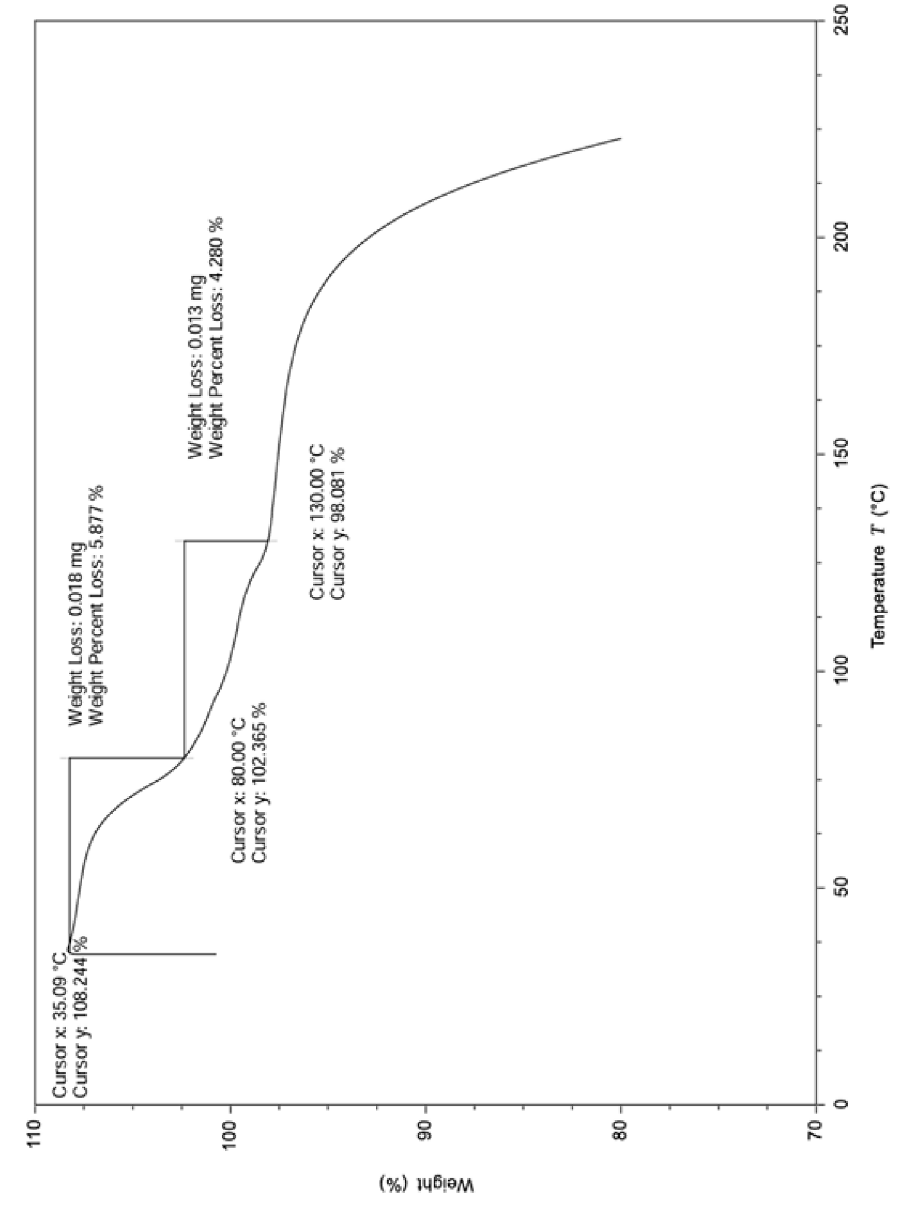
Cursor x: 35.09 °C
Cursor y: 108.244 %
Weight Loss: 0.018 mg
Weight Percent Loss: 5.877 %
Cursor x: 80.00 °C
Cursor y: 102.365 %
Weight Loss: 0.013 mg
Weight Percent Loss: 4.280 %
Cursor x: 130.00 °C
Cursor y: 98.081 %
0
50
100
150
200
250
Temperature T (°C)
110
100
90
80
70
Weight (%)

FIG. 6

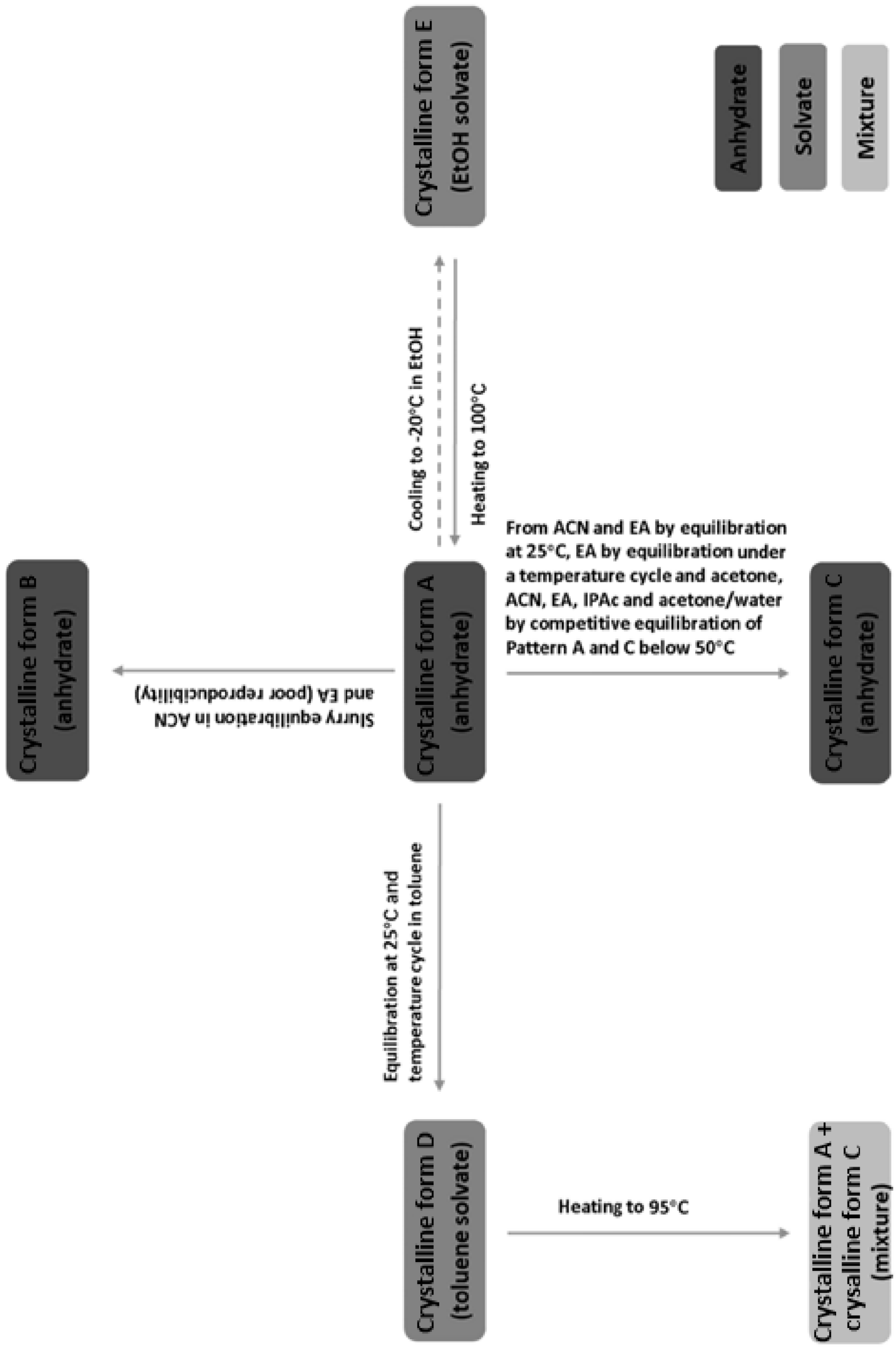

Crystalline form E (EtOH solvate)
Cooling to -20°C in EtOH
Heating to 100°C
Anhydrate
Solvate
Mixture
Crystalline form B (anhydrate)
Slurry equilibration in ACN and EA (poor reproducibility)
Crystalline form A (anhydrate)
From ACN and EA by equilibration at 25°C, EA by equilibration under a temperature cycle and acetone, ACN, EA, IPAc and acetone/water by competitive equilibration of Pattern A and C below 50°C
Crystalline form C (anhydrate)
Equilibration at 25°C and temperature cycle in toluene
Crystalline form D (toluene solvate)
Heating to 95°C
Crystalline form A + crysalline form C (mixture)

FIG. 7a

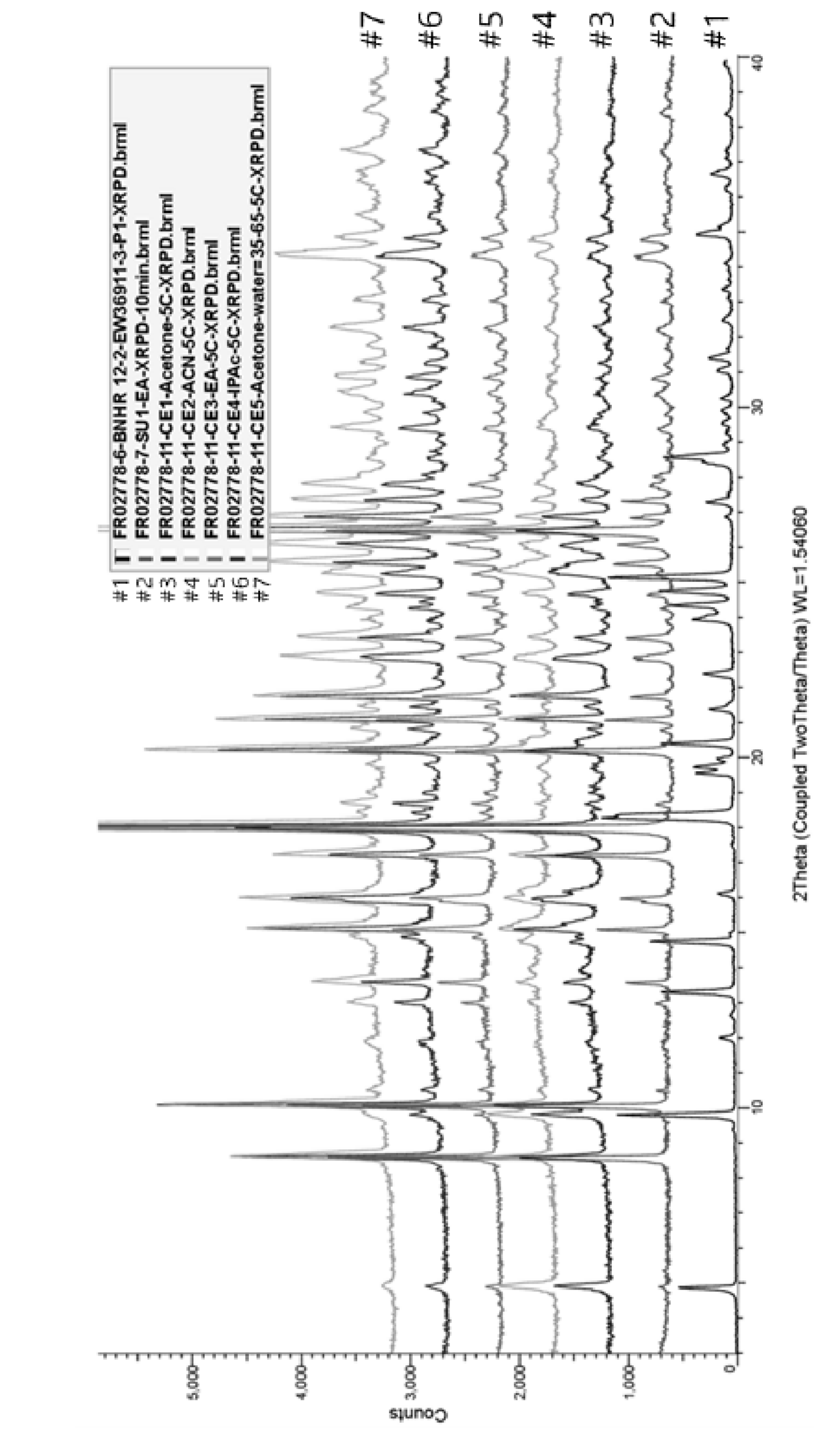
#1 FR02778-6-BNHR 12-2-EW36911-3-P1-XRPD.brml
#2 FR02778-7-SU1-EA-XRPD-10min.brml
#3 FR02778-11-CE1-Acetone-5C-XRPD.brml
#4 FR02778-11-CE2-ACN-5C-XRPD.brml
#5 FR02778-11-CE3-EA-5C-XRPD.brml
#6 FR02778-11-CE4-IPAc-5C-XRPD.brml
#7 FR02778-11-CE5-Acetone-water=35-65-5C-XRPD.brml
#7
#6
#5
#4
#3
#2
#1
Counts
5,000
4,000
3,000
2,000
1,000
0
10
20
30
40
2Theta (Coupled TwoTheta/Theta) WL=1.54060

FIG. 7b

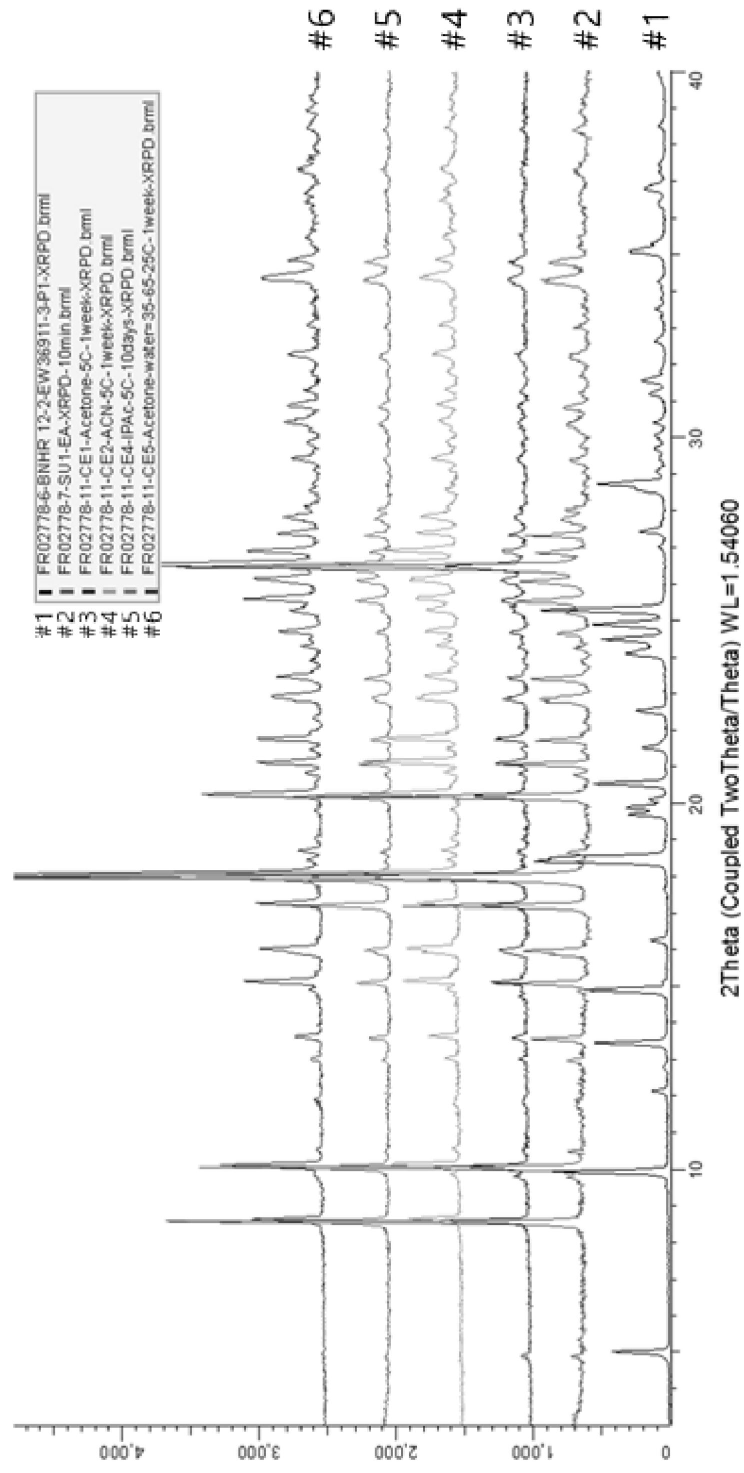
#1 FR02778-6-BNHR 12-2-EW36911-3-P1-XRPD.brml
#2 FR02778-7-SU1-EA-XRPD-10min.brml
#3 FR02778-11-CE1-Acetone-5C-1week-XRPD.brml
#4 FR02778-11-CE2-ACN-5C-1week-XRPD.brml
#5 FR02778-11-CE4-IPAc-5C-10days-XRPD.brml
#6 FR02778-11-CE5-Acetone-water=35-65-25C-1week-XRPD.brml
#6
#5
#4
#3
#2
#1
Counts
0
1,000
2,000
3,000
4,000
10
20
30
40
2Theta (Coupled TwoTheta/Theta) WL=1.54060

FIG. 7c

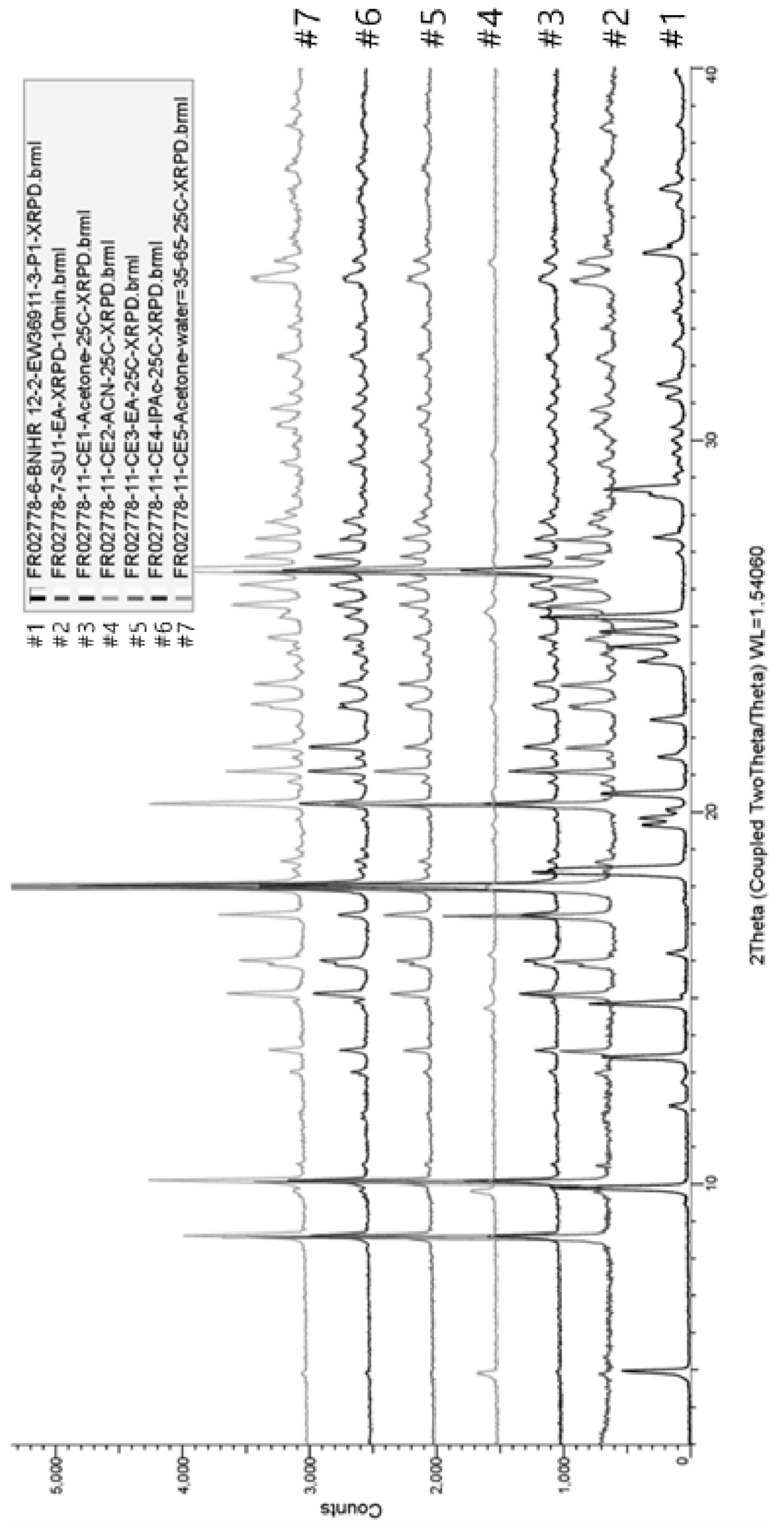
#7
#6
#5
#4
#3
#2
#1
#1 FR02778-6-BNHR 12-2-EW36911-3-P1-XRPD.brml
#2 FR02778-7-SU1-EA-XRPD-10min.brml
#3 FR02778-11-CE1-Acetone-25C-XRPD.brml
#4 FR02778-11-CE2-ACN-25C-XRPD.brml
#5 FR02778-11-CE3-EA-25C-XRPD.brml
#6 FR02778-11-CE4-IPAc-25C-XRPD.brml
#7 FR02778-11-CE5-Acetone-water=35-65-25C-XRPD.brml
Counts
5,000
4,000
3,000
2,000
1,000
0
10
20
30
40
2Theta (Coupled TwoTheta/Theta) WL=1.54060

FIG. 7d

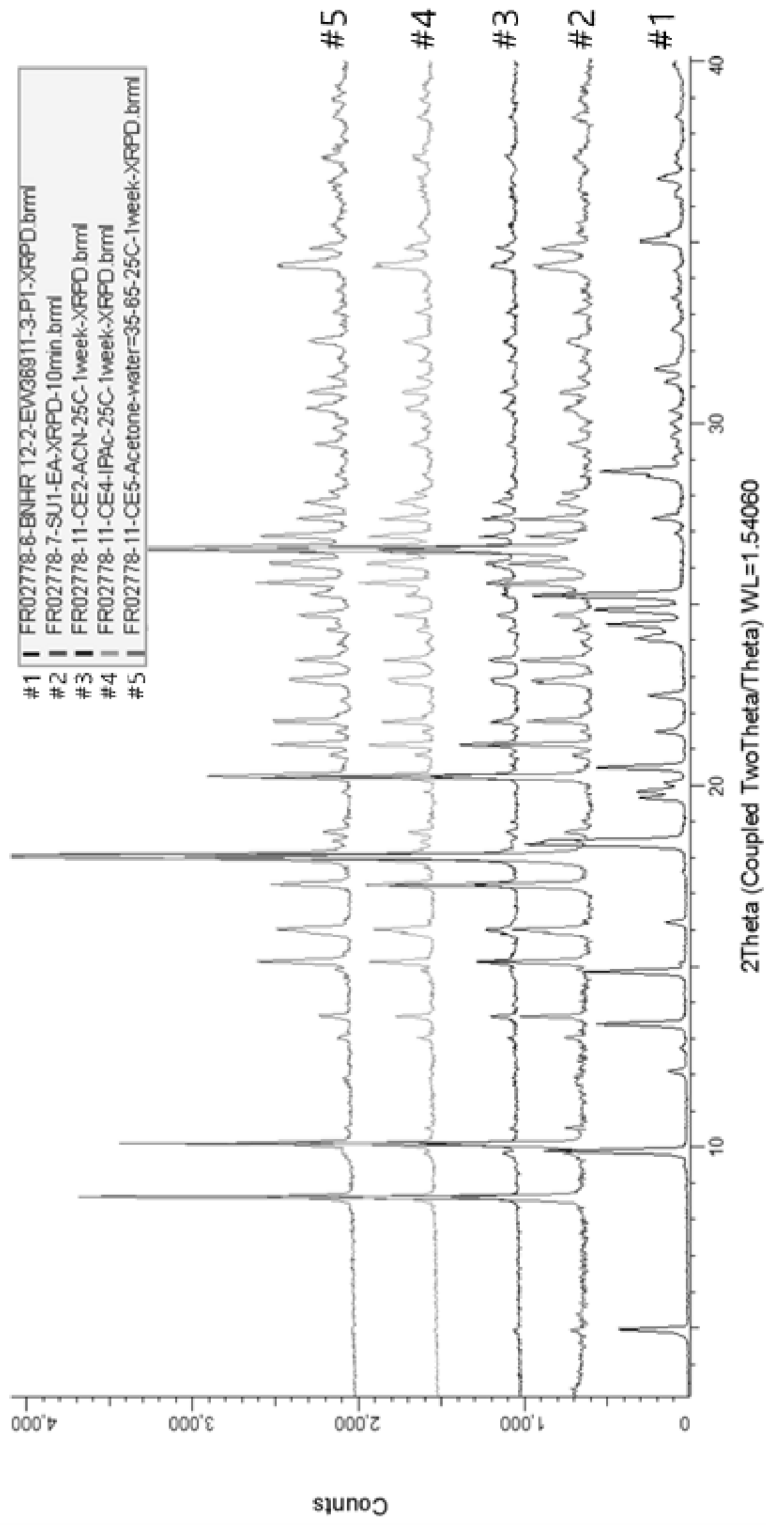
#1 FR02778-8-BNHR 12-2-EW38911-3-P1-XRPD.brml
#2 FR02778-7-SU1-EA-XRPD-10min.brml
#3 FR02778-11-CE2-ACN-25C-1week-XRPD.brml
#4 FR02778-11-CE4-IPAc-25C-1week-XRPD.brml
#5 FR02778-11-CE5-Acetone-water=35-65-25C-1week-XRPD.brml
#5
#4
#3
#2
#1
Counts
0
1,000
2,000
3,000
4,000
10
20
30
40
2Theta (Coupled TwoTheta/Theta) WL=1.54060

FIG. 7e

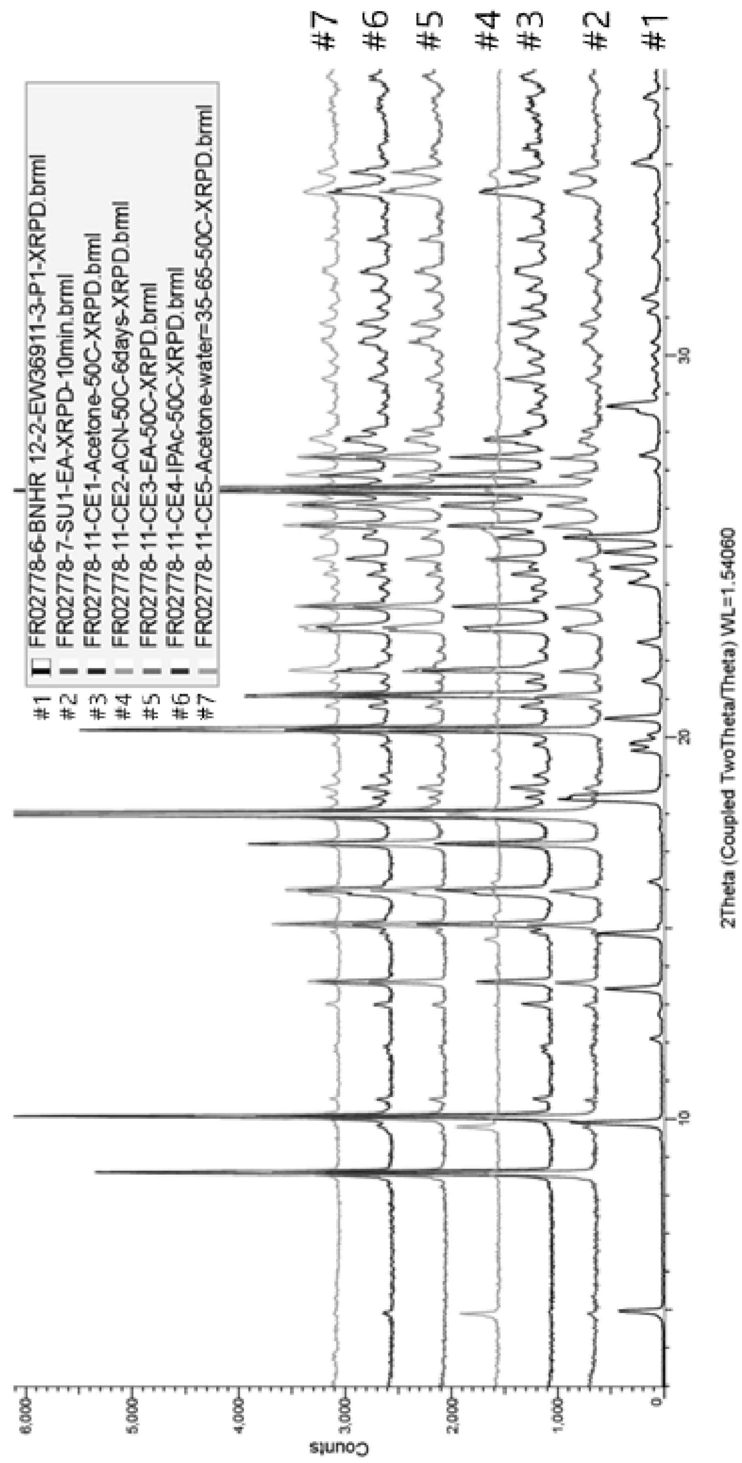
#7
#6
#5
#4
#3
#2
#1
#1 FR02778-6-BNHR 12-2-EW36911-3-P1-XRPD.brml
#2 FR02778-7-SU1-EA-XRPD-10min.brml
#3 FR02778-11-CE1-Acetone-50C-XRPD.brml
#4 FR02778-11-CE2-ACN-50C-6days-XRPD.brml
#5 FR02778-11-CE3-EA-50C-XRPD.brml
#6 FR02778-11-CE4-IPAc-50C-XRPD.brml
#7 FR02778-11-CE5-Acetone-water=35-65-50C-XRPD.brml
Counts
6,000
5,000
4,000
3,000
2,000
1,000
0
10
20
30
2Theta (Coupled TwoTheta/Theta) WL=1.54060

FIG. 7f

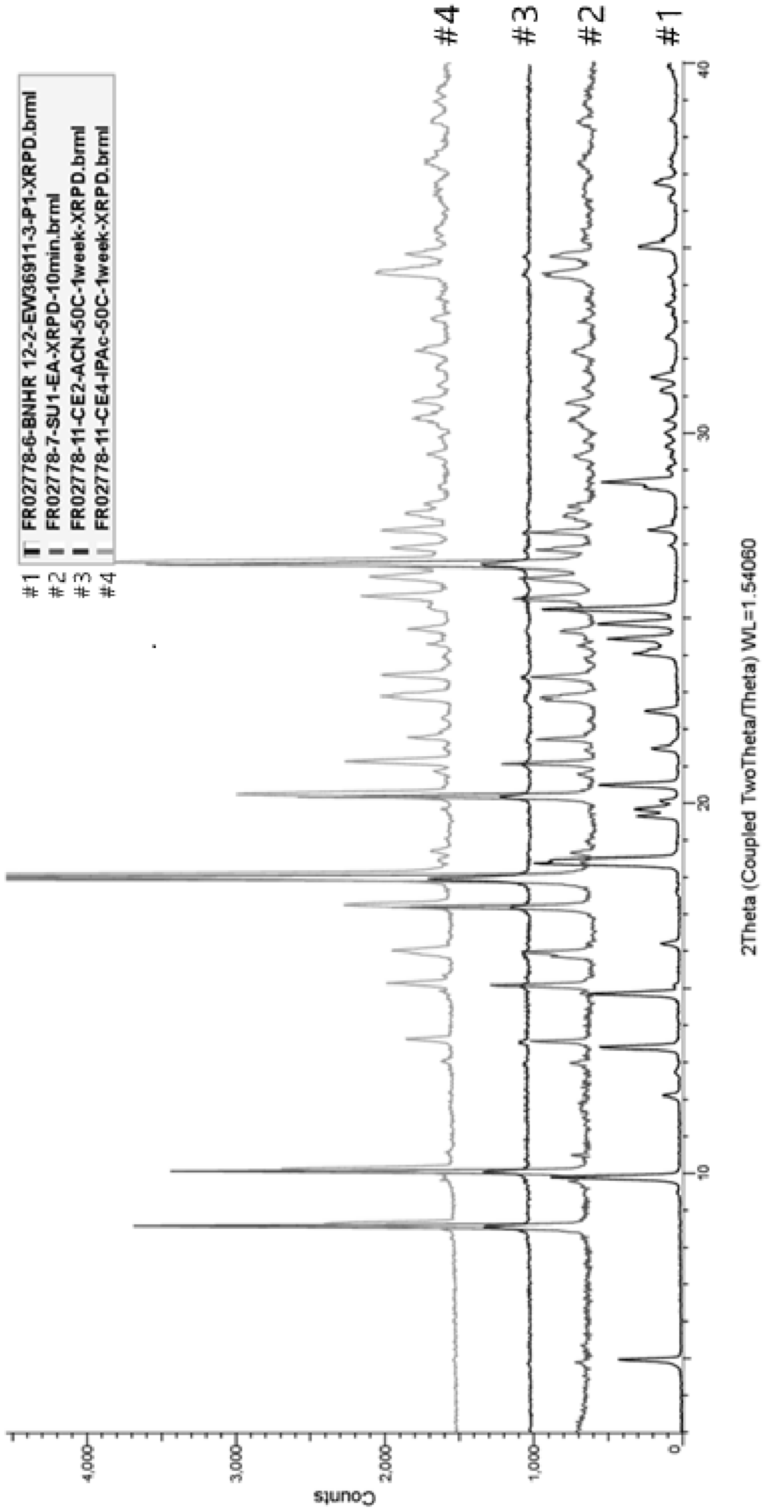
#1 FR02778-6-BNHR 12-2-EW36911-3-P1-XRPD.brml
#2 FR02778-7-SU1-EA-XRPD-10min.brml
#3 FR02778-11-CE2-ACN-50C-1week-XRPD.brml
#4 FR02778-11-CE4-IPAc-50C-1week-XRPD.brml
#4
#3
#2
#1
Counts
0
1,000
2,000
3,000
4,000
10
20
30
40
2Theta (Coupled TwoTheta/Theta) WL=1.54060

FIG. 8

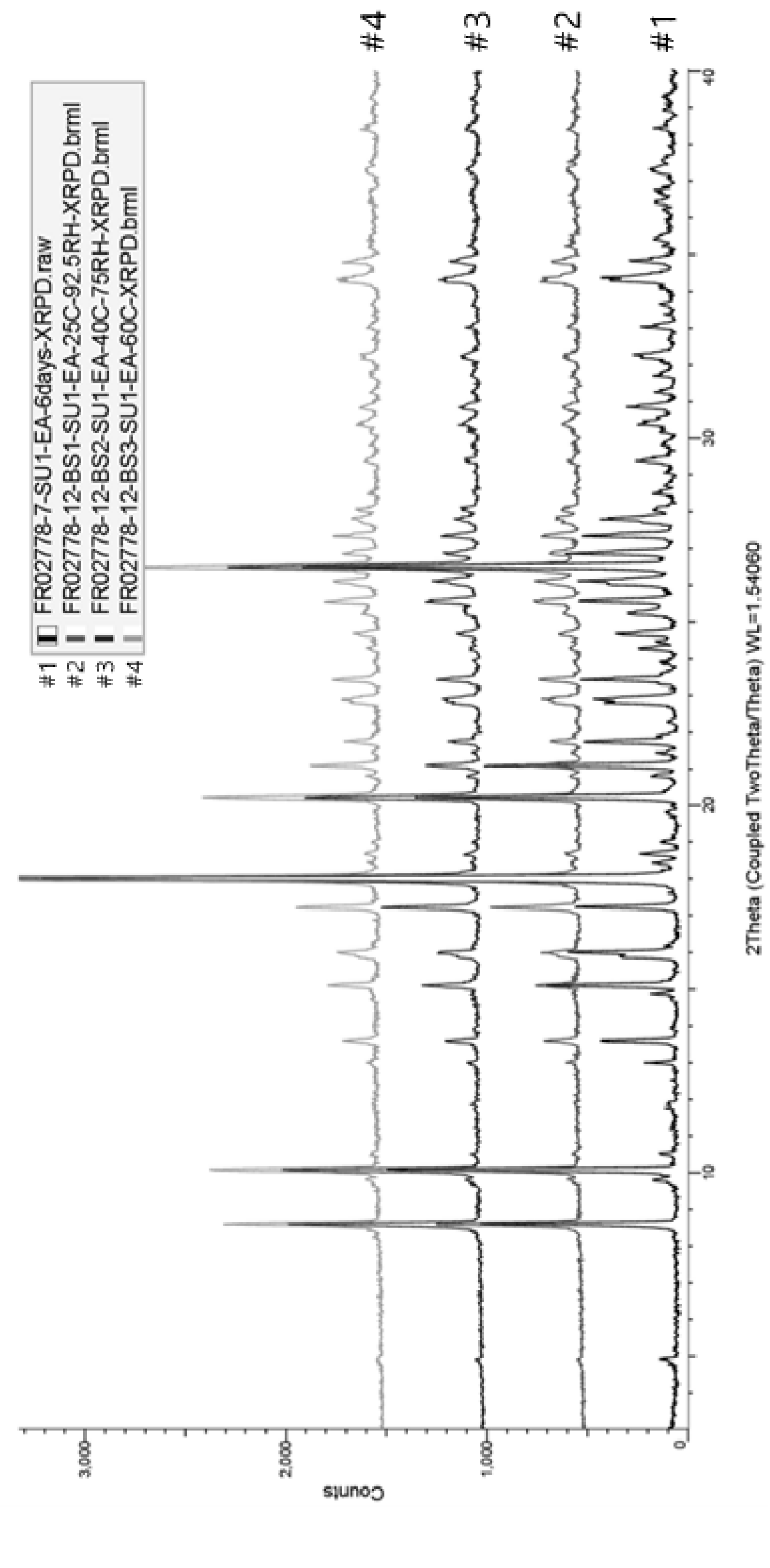

#1 FR02778-7-SU1-EA-6days-XRPD.raw
#2 FR02778-12-BS1-SU1-EA-25C-92.5RH-XRPD.brml
#3 FR02778-12-BS2-SU1-EA-40C-75RH-XRPD.brml
#4 FR02778-12-BS3-SU1-EA-60C-XRPD.brml
#4
#3
#2
#1
Counts
0
1,000
2,000
3,000
10
20
30
40
2Theta (Coupled TwoTheta/Theta) WL=1.54060

FIG. 9a

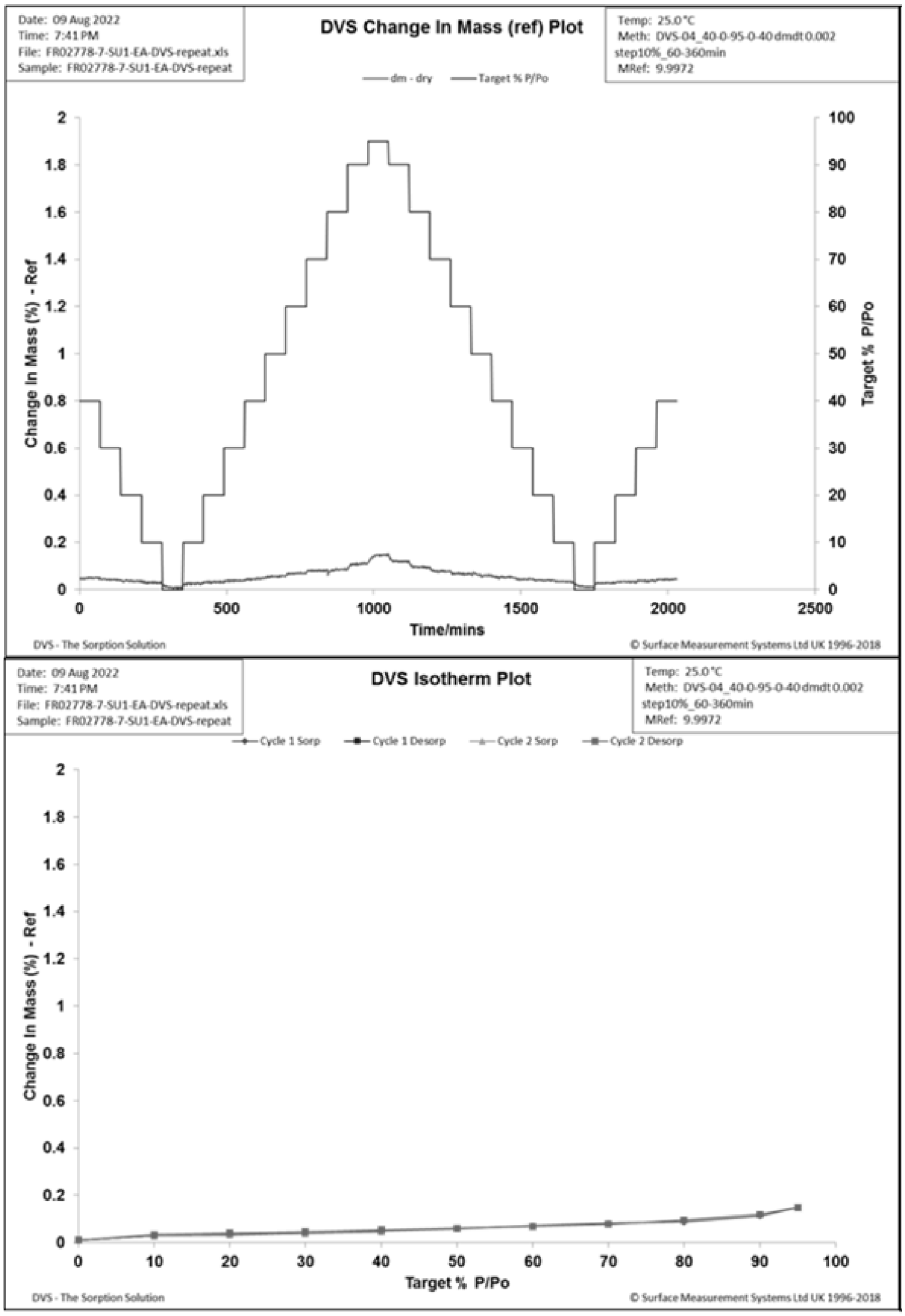
Date: 09 Aug 2022
Time: 7:41 PM
File: FR02778-7-SU1-EA-DVS-repeat.xls
Sample: FR02778-7-SU1-EA-DVS-repeat
DVS Change In Mass (ref) Plot
Temp: 25.0°C
Meth: DVS-04_40-0-95-0-40dmdt0.002 step10%_60-360min
MRef: 9.9972
dm - dry
Target % P/Po
Change In Mass (%) - Ref
Target % P/Po
Time/mins
DVS - The Sorption Solution
© Surface Measurement Systems Ltd UK 1996-2018
Date: 09 Aug 2022
Time: 7:41 PM
File: FR02778-7-SU1-EA-DVS-repeat.xls
Sample: FR02778-7-SU1-EA-DVS-repeat
DVS Isotherm Plot
Temp: 25.0°C
Meth: DVS-04_40-0-95-0-40dmdt0.002 step10%_60-360min
MRef: 9.9972
Cycle 1 Sorp
Cycle 1 Desorp
Cycle 2 Sorp
Cycle 2 Desorp
Change In Mass (%) - Ref
Target % P/Po
DVS - The Sorption Solution
© Surface Measurement Systems Ltd UK 1996-2018

FIG. 9b

# DVS Isotherm Analysis Report

Date: 09 Aug 2022
Time: 7:41 PM
File: C:\PDS-NDL\2022\preformulation\BNHR-20211012\DVS\FR02778-7-SU1-EA-DVS-repeat.xls
Meth: DVS-04_40-0-95-0-40 dmdt 0.002 step10%_60-360min
Sample: FR02778-7-SU1-EA-DVS-repeat
Temp: 25.0 °C
MRef: 9.9972 from Custom Mass

| | Target | Change In Mass (%) - ref | | |
|---|---|---|---|---|
| | % P/Po | Sorption | Desorption | Hysteresis |
| Cycle 1 | 0.0 | 0.0087 | 0.0087 | |
| | 10.0 | 0.0280 | 0.0291 | 0.0011 |
| | 20.0 | 0.0327 | 0.0371 | 0.0044 |
| | 30.0 | 0.0390 | 0.0411 | 0.0021 |
| | 40.0 | 0.0495 | 0.0498 | 0.0003 |
| | 50.0 | 0.0585 | | |
| | 60.0 | 0.0705 | | |
| | 70.0 | 0.0817 | | |
| | 80.0 | 0.0865 | | |
| | 90.0 | 0.1118 | | |
| | 95.0 | 0.1484 | | |
| Cycle 2 | 0.0 | 0.0109 | 0.0109 | |
| | 10.0 | 0.0287 | 0.0331 | 0.0044 |
| | 20.0 | 0.0336 | 0.0404 | 0.0068 |
| | 30.0 | 0.0384 | 0.0460 | 0.0076 |
| | 40.0 | 0.0457 | 0.0538 | 0.0081 |
| | 50.0 | | 0.0604 | |
| | 60.0 | | 0.0679 | |
| | 70.0 | | 0.0777 | |
| | 80.0 | | 0.0943 | |
| | 90.0 | | 0.1189 | |
| | 95.0 | | 0.1484 | |

FIG. 9c

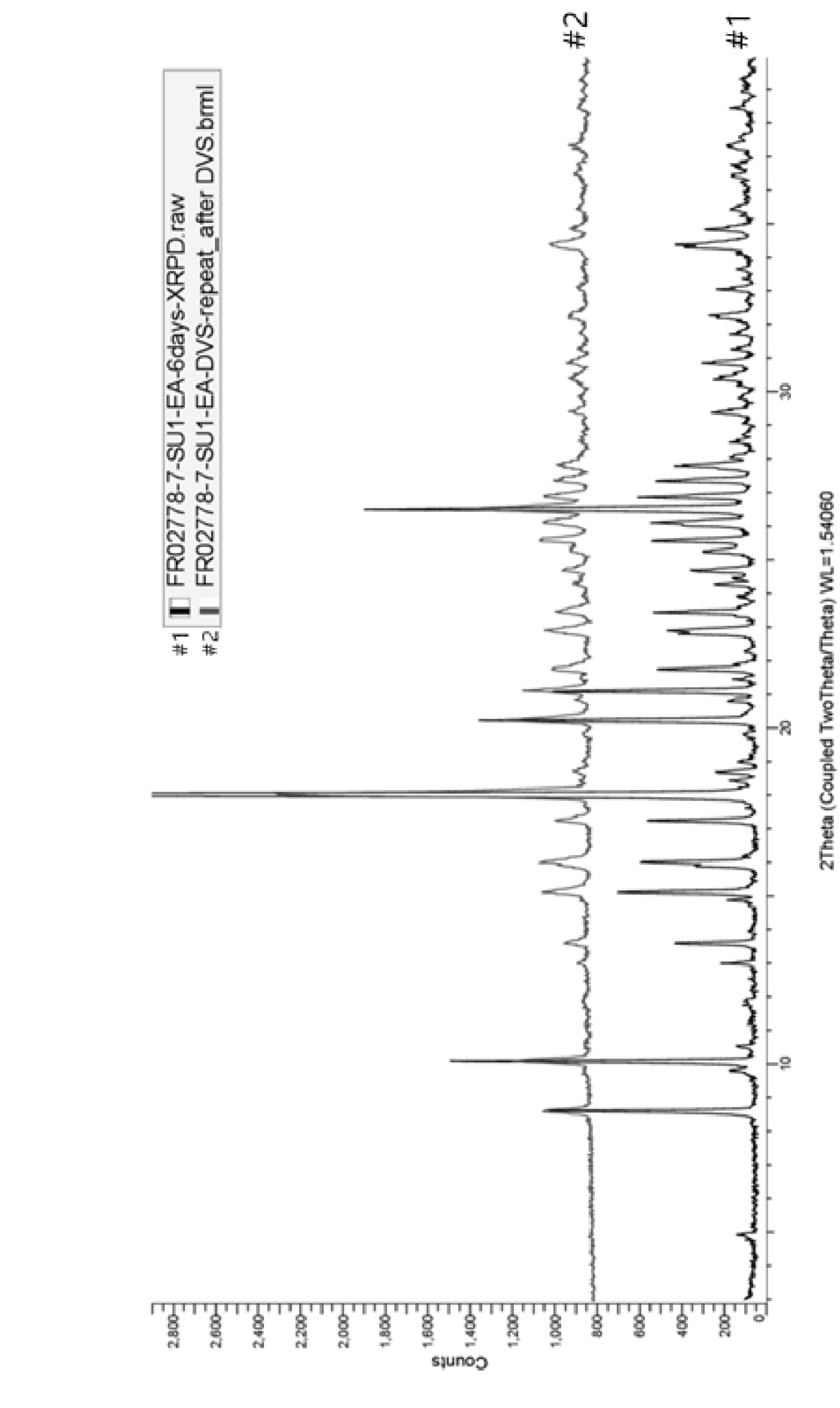
#1 FR02778-7-SU1-EA-6days-XRPD.raw
#2 FR02778-7-SU1-EA-DVS-repeat_after DVS.brml
#2
#1
Counts
2Theta (Coupled TwoTheta/Theta) WL=1.54060

FIG. 10

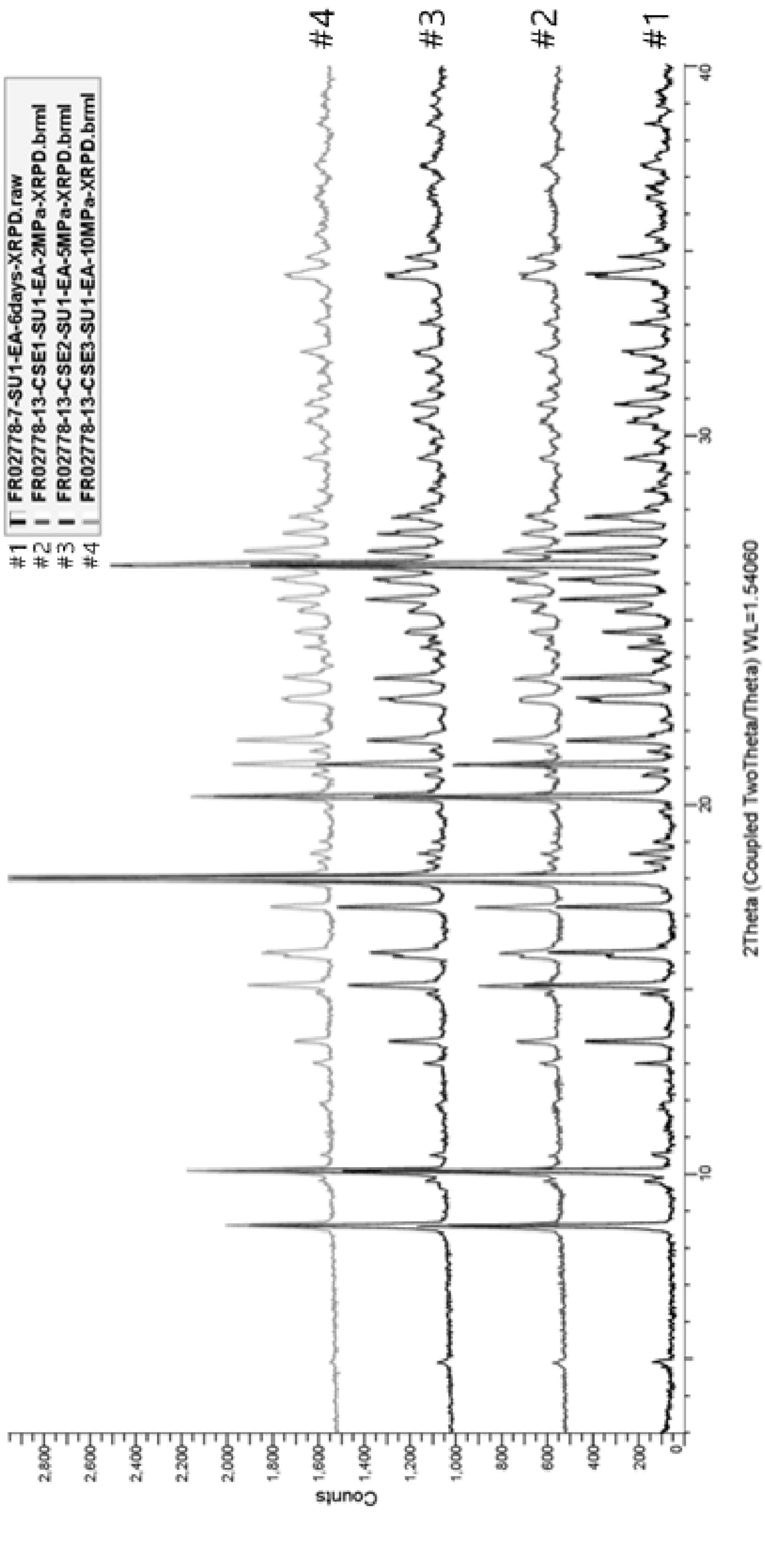
#1 FR02778-7-SU1-EA-6days-XRPD.raw
#2 FR02778-13-CSE1-SU1-EA-2MPa-XRPD.brml
#3 FR02778-13-CSE2-SU1-EA-5MPa-XRPD.brml
#4 FR02778-13-CSE3-SU1-EA-10MPa-XRPD.brml
#4
#3
#2
#1
Counts
2Theta (Coupled TwoTheta/Theta) WL=1.54060

FIG. 11

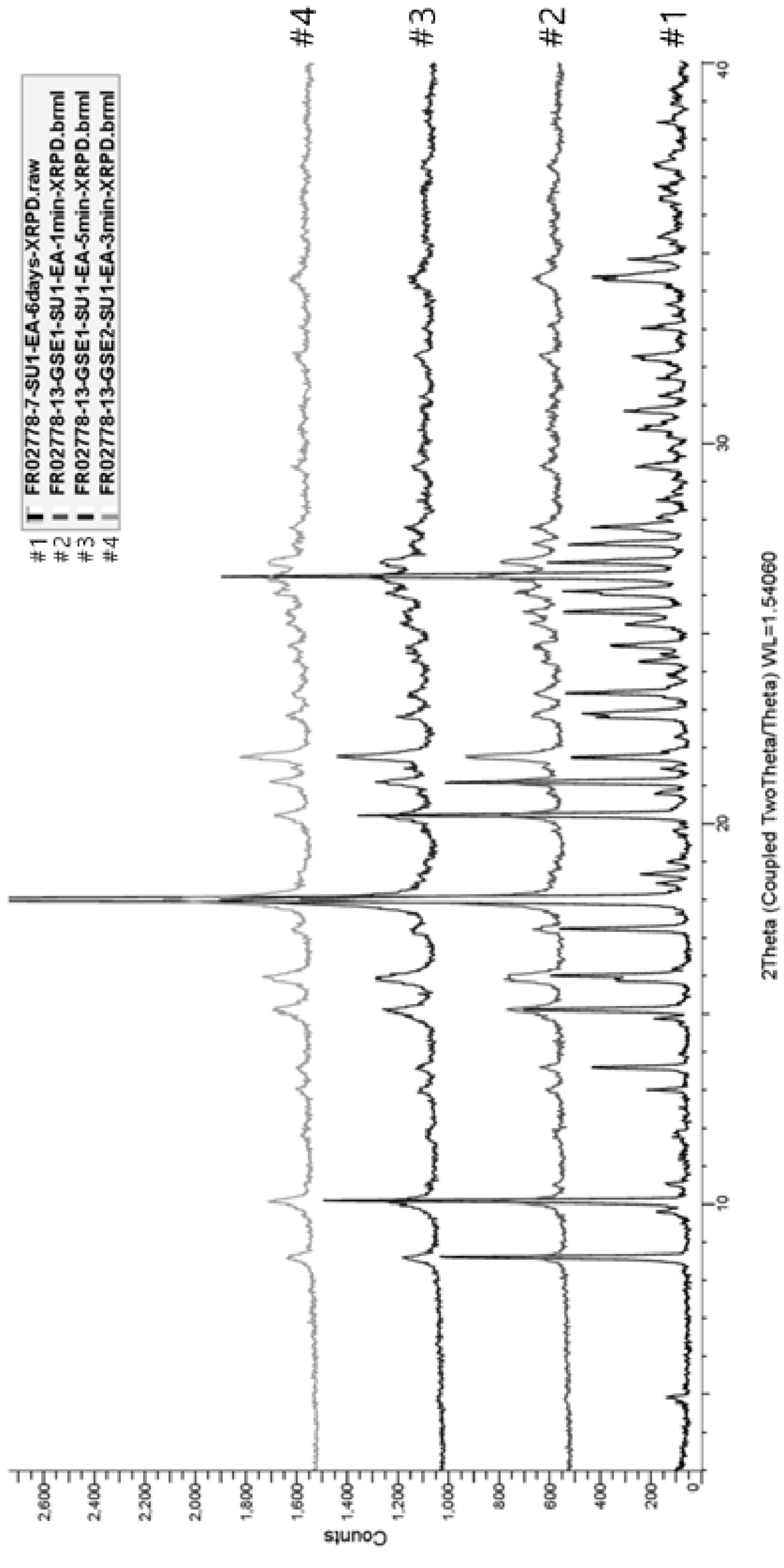

#1 FR02778-7-SU1-EA-6days-XRPD.raw
#2 FR02778-13-GSE1-SU1-EA-1min-XRPD.brml
#3 FR02778-13-GSE1-SU1-EA-5min-XRPD.brml
#4 FR02778-13-GSE2-SU1-EA-3min-XRPD.brml
#4
#3
#2
#1
Counts
2Theta (Coupled TwoTheta/Theta) WL=1.54060

FIG. 12

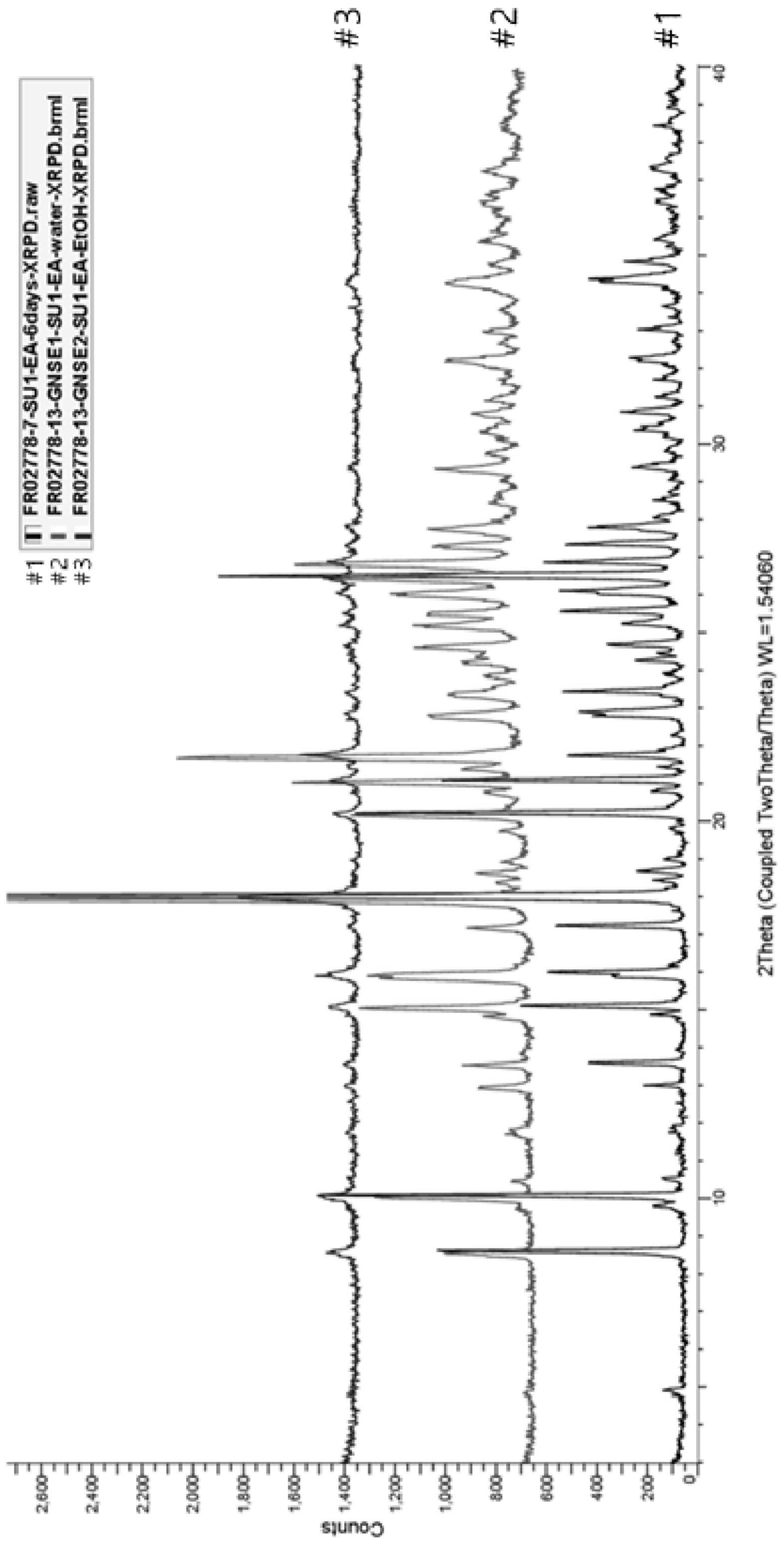
#1 FR02778-7-SU1-EA-6days-XRPD.raw
#2 FR02778-13-GNSE1-SU1-EA-water-XRPD.brml
#3 FR02778-13-GNSE2-SU1-EA-EtOH-XRPD.brml
#3
#2
#1
Counts
2Theta (Coupled TwoTheta/Theta) WL=1.54060

FIG. 13a

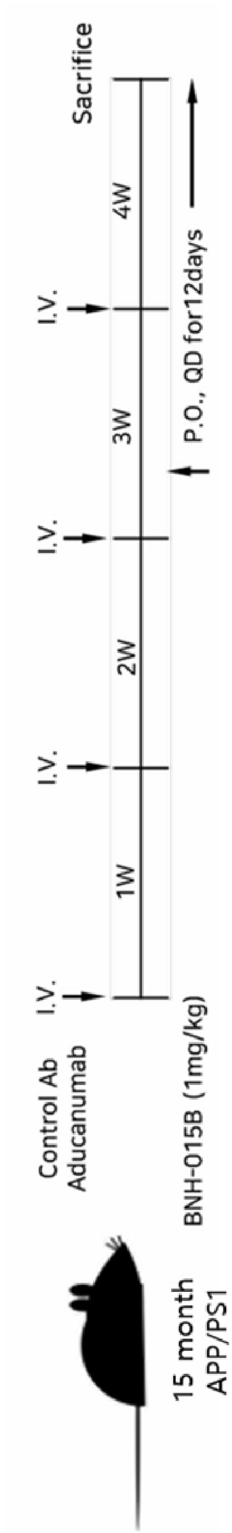
15 month
APP/PS1
Control Ab
Aducanumab
BNH-015B (1mg/kg)
I.V.
1W
I.V.
2W
I.V.
3W
I.V.
4W
Sacrifice
P.O., QD for12days

FIG. 13b

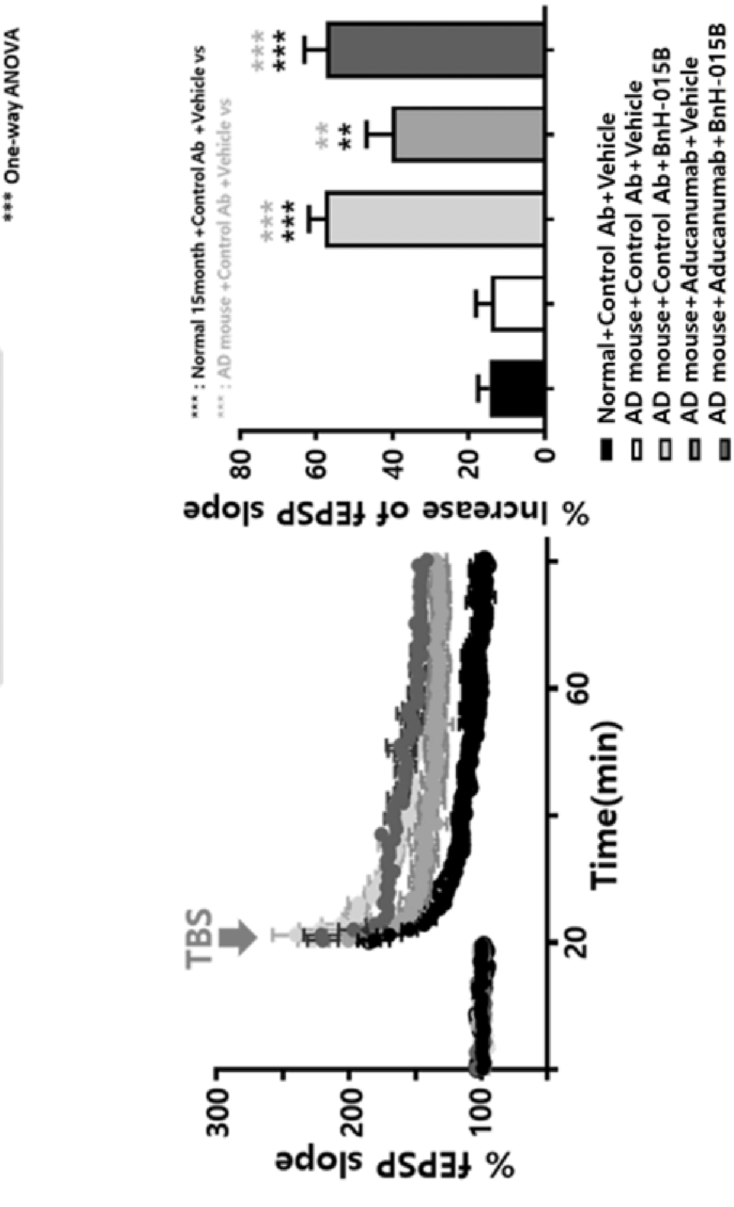
Hippocampus
*** One-way ANOVA
TBS
% fEPSP slope
300
200
100
20
60
Time(min)
% Increase of fEPSP slope
80
60
40
20
0
*** : Normal 15month +Control Ab +Vehicle vs
*** : AD mouse +Control Ab +Vehicle vs
***
**
Normal+Control Ab+Vehicle
AD mouse+Control Ab+Vehicle
AD mouse+Control Ab+BnH-015B
AD mouse+Aducanumab+Vehicle
AD mouse+Aducanumab+BnH-015B

FIG. 13c

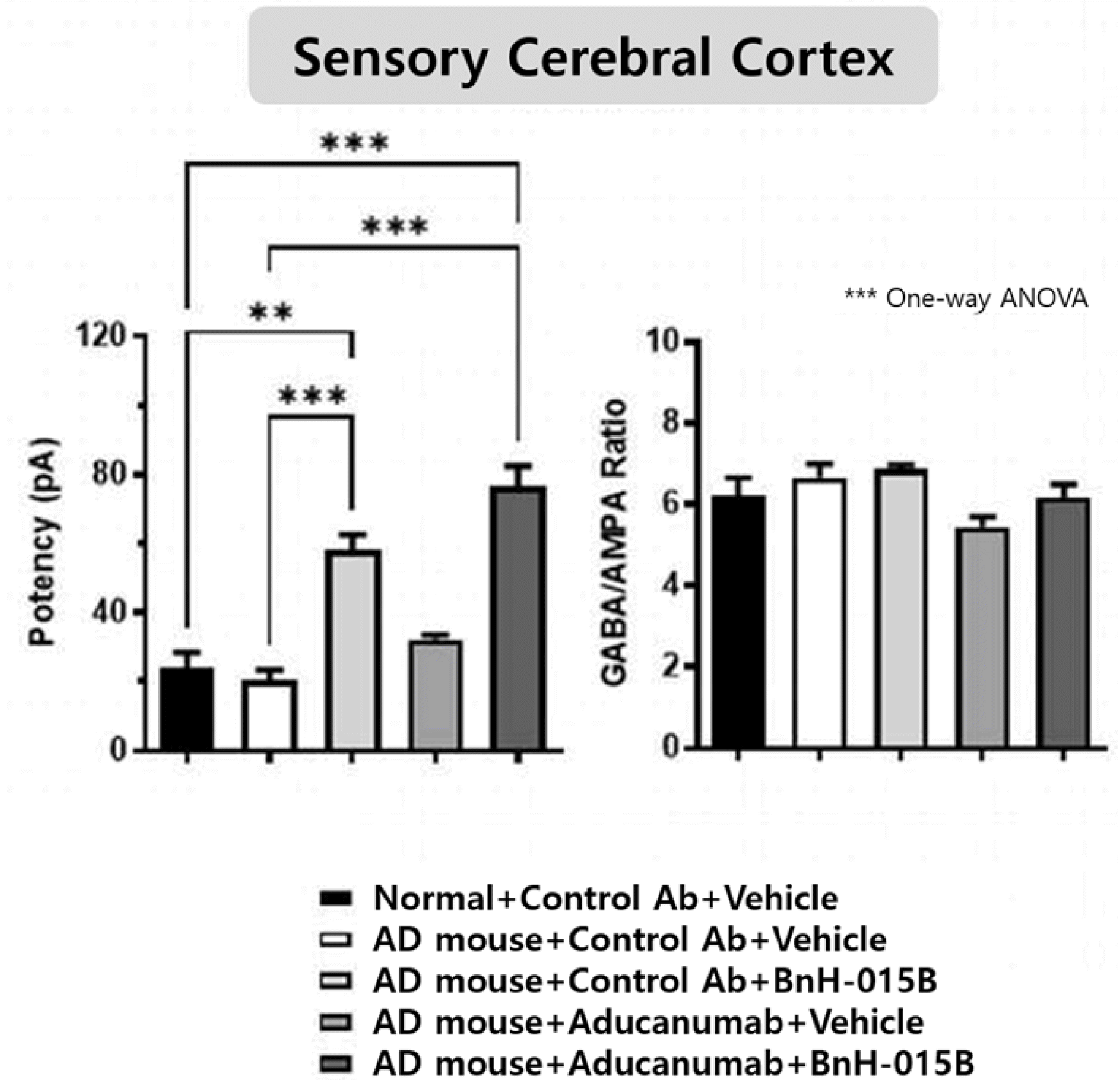
Sensory Cerebral Cortex
***
***
**
***
*** One-way ANOVA
Potency (pA)
120
80
40
0
GABA/AMPA Ratio
10
8
6
4
2
0
Normal+Control Ab+Vehicle
AD mouse+Control Ab+Vehicle
AD mouse+Control Ab+BnH-015B
AD mouse+Aducanumab+Vehicle
AD mouse+Aducanumab+BnH-015B

FIG. 14a

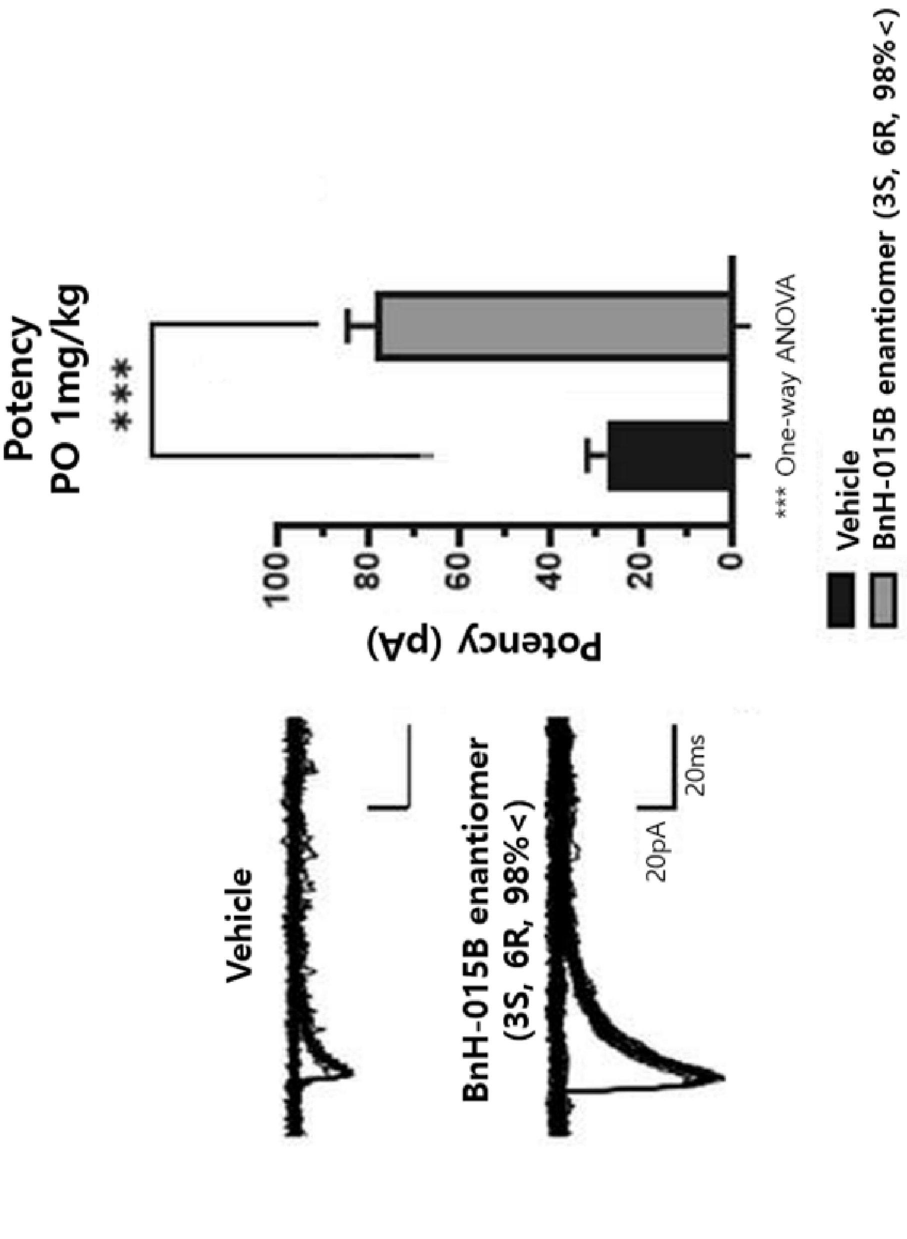

Potency
PO 1mg/kg
***
Potency (pA)
100
80
60
40
20
0
*** One-way ANOVA
Vehicle
BnH-015B enantiomer (3S, 6R, 98%<)
Vehicle
BnH-015B enantiomer
(3S, 6R, 98%<)
20pA
20ms

FIG. 14b

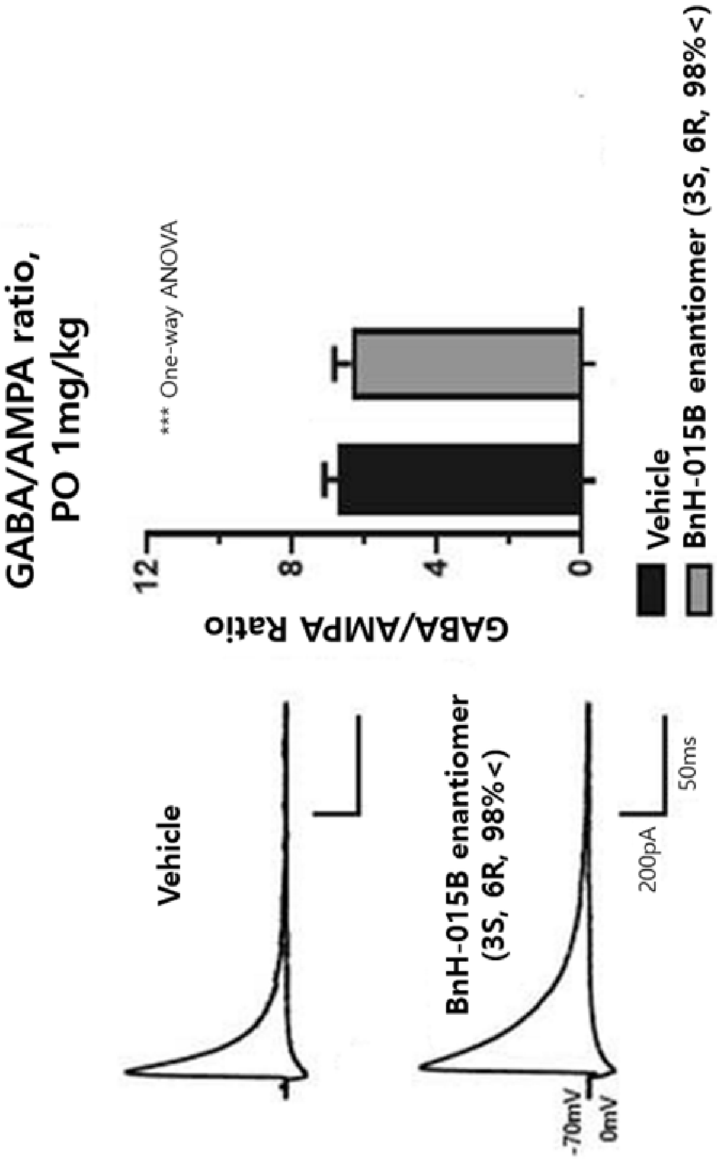
Vehicle
BnH-015B enantiomer
(3S, 6R, 98%<)
-70mV
0mV
200pA
50ms
GABA/AMPA ratio,
PO 1mg/kg
*** One-way ANOVA
GABA/AMPA Ratio
12
8
4
0
Vehicle
BnH-015B enantiomer (3S, 6R, 98%<)

FIG. 15

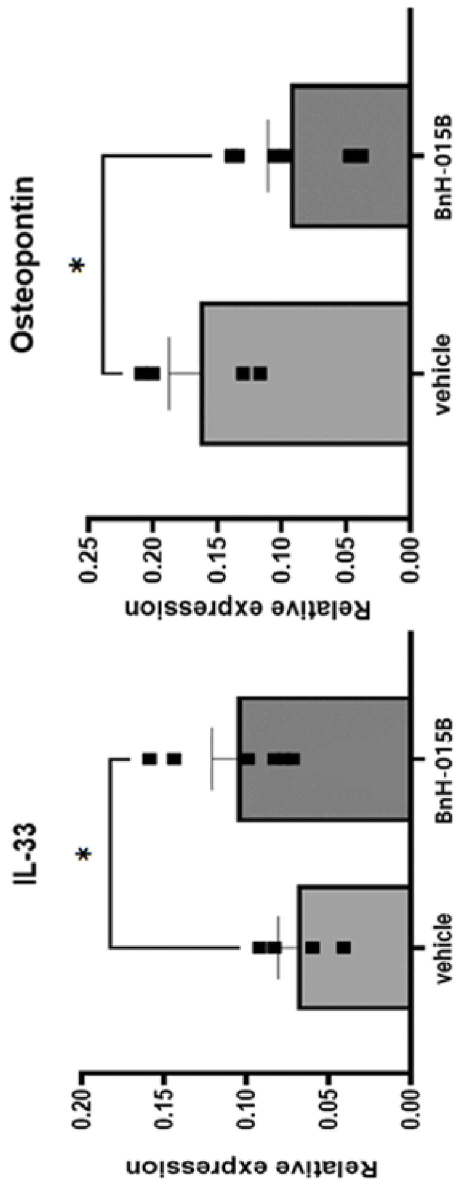
IL-33
Osteopontin
Relative expression
0.00
0.05
0.10
0.15
0.20
0.25
vehicle
BnH-015B
*

FIG. 16a

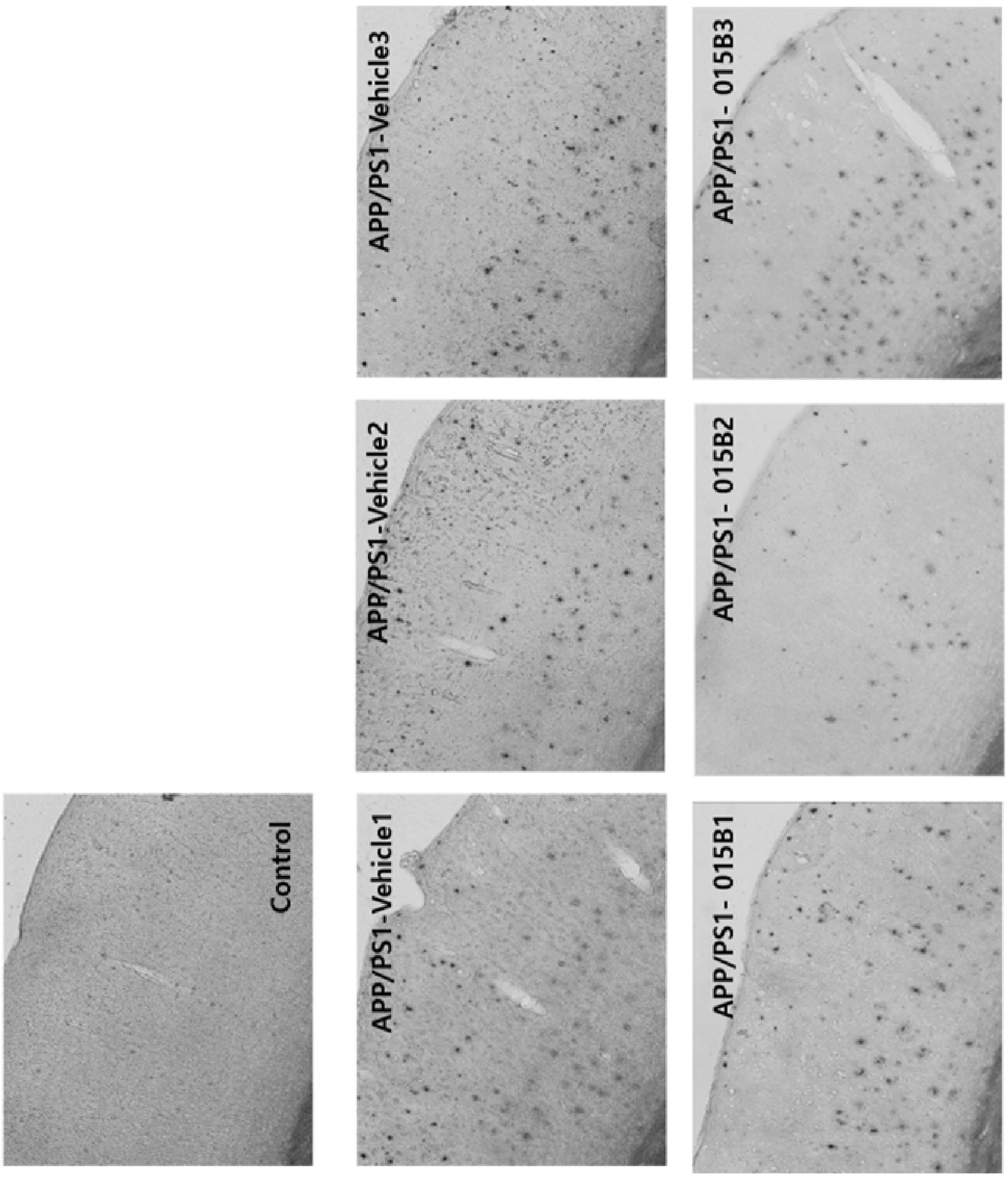
APP/PS1-Vehicle3
APP/PS1- 015B3
APP/PS1-Vehicle2
APP/PS1- 015B2
Control
APP/PS1-Vehicle1
APP/PS1- 015B1

FIG. 16b

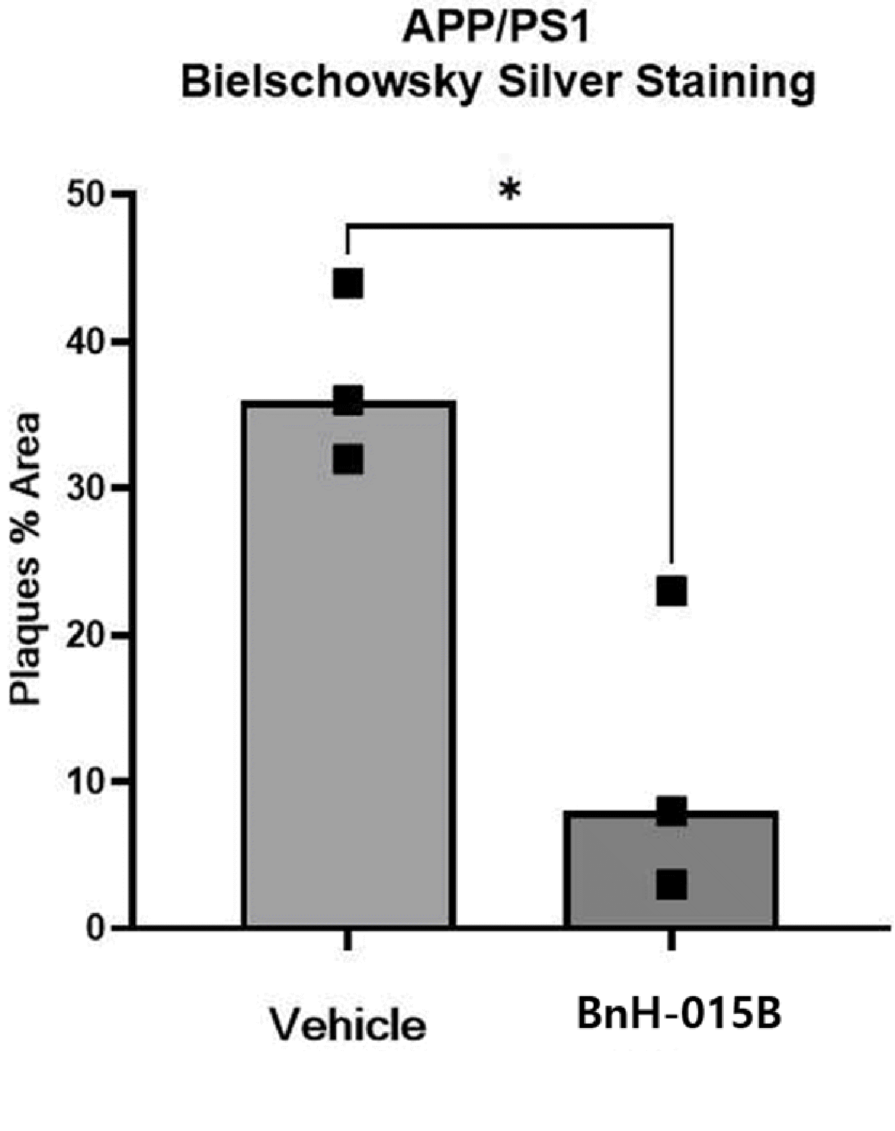
APP/PS1
Bielschowsky Silver Staining
Plaques % Area
50
40
30
20
10
0
*
Vehicle
BnH-015B

## INTERNATIONAL SEARCH REPORT

International application No.
**PCT/KR2024/014808**

### A. CLASSIFICATION OF SUBJECT MATTER

**C07D 211/60**(2006.01)i; **A61K 31/445**(2006.01)i; **A61K 45/06**(2006.01)i; **A61P 25/00**(2006.01)i; **A61P 25/28**(2006.01)i; **A61K 39/00**(2006.01)i

According to International Patent Classification (IPC) or to both national classification and IPC

### B. FIELDS SEARCHED

Minimum documentation searched (classification system followed by classification symbols)

C07D 211/60(2006.01); C07D 211/34(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 결정질(crystalline), 피리딘(pyridine), 페닐(phenyl), 알츠하이머병(alzheimer's disease), PTSD

### C. DOCUMENTS CONSIDERED TO BE RELEVANT

| Category* | Citation of document, with indication, where appropriate, of the relevant passages | Relevant to claim No. |
|---|---|---|
| A | BARRAZA, S. J. et al. Discovery of anthranilamides as a novel class of inhibitors of neurotropic alphavirus replication. Bioorganic & medicinal chemistry. 2015, vol. 23, no. 7, pp. 1569-1587. See formula 2. | 1-25 |
| A | MUSTAZZA, C. et al. Synthesis and cholinesterase activity of phenylcarbamates related to Rivastigmine, a therapeutic agent for Alzheimer`s disease. European journal of medicinal chemistry. 2002, vol. 37, pp. 91-109. See abstract; and figures 1 and 2. | 1-25 |
| A | WO 2006-043064 A1 (MERCK SHARP & DOHME LIMITED et al.) 27 April 2006 (2006-04-27) See claims 1-13. | 1-25 |
| A | WO 02-22572 A2 (SEPRACOR, INC.) 21 March 2002 (2002-03-21) See abstract; and claim 1. | 1-25 |
| A | WO 2008-012555 A2 (ISIS INNOVATION LIMITED et al.) 31 January 2008 (2008-01-31) See claims 1-37. | 1-25 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 January 2025** | **13 January 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/014808**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **26**
because they relate to subject matter not required to be searched by this Authority, namely:
Claim 26 pertains to a method for treatment of the human body by surgery or therapy, as well as a diagnostic method (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/014808**

| Patent document cited in search report | Publication date (day/month/year) | Patent family member(s) | Publication date (day/month/year) |
|---|---|---|---|
| WO 2006-043064 A1 | 27 April 2006 | AT E455763 T1 | 15 February 2010 |
| | | AU 2005-297063 A1 | 27 April 2006 |
| | | AU 2005-297063 B2 | 02 June 2011 |
| | | CA 2584504 A1 | 27 April 2006 |
| | | CN 101044115 A | 26 September 2007 |
| | | EP 1805141 A1 | 11 July 2007 |
| | | EP 1805141 B1 | 20 January 2010 |
| | | JP 2008-517047 A | 22 May 2008 |
| | | US 2008-0021043 A1 | 24 January 2008 |
| | | US 7638629 B2 | 29 December 2009 |
| WO 02-22572 A2 | 21 March 2002 | AU 2001-290873 B2 | 27 July 2006 |
| | | AU 2001-90873 A1 | 26 March 2002 |
| | | CA 2422055 A1 | 21 March 2002 |
| | | EP 1318988 A2 | 18 June 2003 |
| | | JP 2004-509103 A | 25 March 2004 |
| | | US 2003-0050309 A1 | 13 March 2003 |
| | | US 2004-0077706 A1 | 22 April 2004 |
| | | US 2005-0080078 A1 | 14 April 2005 |
| | | US 2009-0258901 A1 | 15 October 2009 |
| | | US 7132551 B2 | 07 November 2006 |
| | | US 7294637 B2 | 13 November 2007 |
| | | US 7517892 B2 | 14 April 2009 |
| | | US 7816375 B2 | 19 October 2010 |
| | | WO 02-22572 A3 | 01 August 2002 |
| | | WO 2005-077463 A2 | 25 August 2005 |
| | | WO 2005-077463 A3 | 26 January 2006 |
| WO 2008-012555 A2 | 31 January 2008 | EP 2051708 A2 | 29 April 2009 |
| | | US 2010-0022620 A1 | 28 January 2010 |
| | | WO 2008-012555 A3 | 25 September 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230131187
- KR 1020230040835
- KR 2023004138 W


### Non-patent literature cited in the description


- **YU et al.** *Neuron*, 2012, vol. 74, 731-42
- **PETRUS et al.** *Neuron*, 2014, vol. 81, 664-73
- **GAO et al.** *Cell*, 2014, vol. 159 (4), 775-788
- **GAO et al.** *J Neurosci.*, 2014, vol. 34 (32), 10770-10779
- **YU** ; **XIN et al.** Thalamocortical inputs show post-critical-period plasticity. *Neuron*, 2012, vol. 74 (4), 731-742
- **PETRUS** ; **EMILY et al.** Crossmodal induction of thalamocortical potentiation leads to enhanced information processing in the auditory cortex. *Neuron*, 2014, vol. 81 (3), 664-673
- **GAO** ; **PENG et al.** Deterministic progenitor behavior and unitary production of neurons in the neocortex. *Cell*, 2014, vol. 159 (4), 775-788
- **GAO** ; **MING et al.** Rebound potentiation of inhibition in juvenile visual cortex requires vision-induced BDNF expression. *Journal of Neuroscience*, 2014, vol. 34 (32), 10770-10779
- Diagnostic and Statistical Manual of Mental Disorders. American Psychiatric Association, 1994
- **FRAY et al.** CANTAB battery: proposed utility in neurotoxicology. *Neurotoxicol Teratol*, 1996, vol. 18 (4), 499-504
- **DEHAENE et al.** Reward-dependent learning in neuronal networks for planning and decision making. *Brain Res*, 2000, vol. 126, 21729
- **IVERSON et al.** Interpreting change on the WAIS-III/WMS-I11 in clinical samples. *Arch Clin Neuropsychol*, 2001, vol. 16 (2), 183-91
- **WEAVER et al.** Mild memory impairment in healthy older adults is distinct from normal aging. *Cogn*, 2006, vol. 60 (2), 146-55
- Cognitive-Behavioral Therapies. Guilford Publications, Inc, 2002
- **BY JUDITH S.S. BECK**. The New Handbook of Cognitive Therapy: Basics and Beyond. Guilford Publications, Inc, 1995